(19) Europäisches Patentamt
European Patent Office
Office européen des brevets

(11) **EP 2 279 165 B1**

(12) **EUROPÄISCHE PATENTSCHRIFT**

(45) Veröffentlichungstag und Bekanntmachung des Hinweises auf die Patenterteilung:
**04.06.2014 Patentblatt 2014/23**

(21) Anmeldenummer: **09753803.7**

(22) Anmeldetag: **18.05.2009**

(51) Int Cl.:
*C07C 209/78* (2006.01)   *C07C 211/50* (2006.01)
*C07C 265/14* (2006.01)

(86) Internationale Anmeldenummer:
**PCT/EP2009/055986**

(87) Internationale Veröffentlichungsnummer:
**WO 2009/144148 (03.12.2009 Gazette 2009/49)**

(54) **CHLOR ARMES MEHRSTUFIGES VERFAHREN ZUR HERSTELLUNG VON CYCLOALIPHATISCHEN DIISOCYANATEN**

LOW CHLORINE, MULTI-STAGED METHOD FOR PRODUCING CYCLOALIPHATIC DIISOCYANATES

PROCÉDÉ PAUVRE EN CHLORE EN PLUSIEURS ÉTAPES, POUR LA PRÉPARATION DE DIISOCYANATES CYCLOALIPHATIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **27.05.2008 DE 102008002002**

(43) Veröffentlichungstag der Anmeldung:
**02.02.2011 Patentblatt 2011/05**

(73) Patentinhaber: **Evonik Degussa GmbH 45128 Essen (DE)**

(72) Erfinder:
• **GRUND, Gerda**
**48653 Coesfeld (DE)**
• **KRECZINSKI, Manfred**
**44652 Herne (DE)**
• **KOHLSTRUK, Stephan**
**48249 Dülmen (DE)**
• **LETTMANN, Christian**
**48653 Coesfeld (DE)**

(56) Entgegenhaltungen:
EP-A- 0 000 778    EP-A- 0 043 933
EP-A- 1 512 681    DE-B- 1 210 872
US-A- 4 554 378

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Chlor armes mehrstufiges Verfahren zur kontinuierlichen Chlor freien Herstellung von cycloaliphatischen Diisocyanaten, das die Synthese von Diaminodiphenylalkanen, ihrer Hydrierung zu den entsprechenden cycloaliphatischen Diaminen und die anschließende Umwandlung von cycloaliphatischen Diaminen in die entsprechenden Cycloalkylenbiscarbamate und die thermische Spaltung letzterer in die cycloaliphatischen Diisocyanate und Alkohol umfasst.

Es ist bekannt, Diaminodiphenylalkane (darunter subsummiert werden auch substituierte Diphenyle als auch Systeme mit kondensierten aromatischen Ringen (2-oder 3-Ringe) verstanden) durch Kondensation von einem aromatischen Amin, wie z. B. Anilin und Aldehyd an sauren Katalysatoren herzustellen. Diaminodiphenylalkane werden im Rahmen dieser Erfindung auch als aromatische Diamine bezeichnet.

Die Reaktion erfolgt so, dass zunächst aus einem aromatischen Amin (Anilin) und Aldehyd N-Alkyl-Verbindungen gebildet werden. Diese Vorkondensate reagieren dann in Gegenwart von sauren Katalysatoren weiter zu Aminalen. Diese Aminale lagern sich anschließend unter Einwirkung eines sauren Katalysators zu Diaminodiphenylalkanen um.

Im Stand der Technik wurde häufig die Herstellung von Diaminodiphenylalkanen aus der Kondensation von aromatischen Aminen und Aldehyd, insbesondere Anilin und Formaldehyd, durchgeführt. Dabei wurde je nach Reaktionsvariante entweder zunächst das Kondensationsprodukt aus Anilin und Formaldehyd hergestellt und dieses dann in Gegenwart von Säuren wie beispielsweise Salzsäure umgelagert oder aber die Kondensation wurde bereits in Gegenwart von Säuren unter Umlagerungsbedingungen durchgeführt.

Ein Nachteil dieses Vorgehens ist es, dass bei der homogenen Katalyse mit Mineralsäuren, insbesondere mit Salzsäure, salzhaltige Abwässer anfallen, die bei der Neutralisation der Säuren entstehen. Dabei sind die Chlorsalze als besonders kritisch anzusehen. Darüber hinaus führen die wässrigen Mineralsäuren zu Korrosionsproblemen in den Herstellungsanlagen. Deshalb wurden im weiteren Stand der Technik Verfahren entwickelt, bei denen entsprechende heterogene Katalysatoren eingesetzt werden. Dabei werden neben sauren Ionenaustauschern auch saure synthetische oder natürliche Silicium- oder Aluminiumoxide eingesetzt wie Zeolite oder Tonmineralien.

In der US 4294987 wird in einem derartigen Verfahren die Kondensation in Gegenwart einer starken wässrigen Säure durchgeführt, wonach durch Lösungsmittelextraktion die Säure entfernt wird. Die Umlagerung wird wiederum in Gegenwart von starker Säure, die in geringerer Menge eingesetzt wird, durchgeführt. Diatomeenerde, Tone oder Zeolite können als Katalysator in dieser Reaktionsstufe eingesetzt werden.

Die DE-A-12 30 033 beschreibt ein Verfahren zur Herstellung von Diaminodiphenylalkanen. Dabei wird siliciumhaltiger Ton, ein synthetischer Siliciumdioxid-Aluminiumoxid-Katalysator oder ein Magnesiumoxid-Aluminiumoxid-Katalysator eingesetzt.

Ein weiteres Reaktionsverfahren zur Herstellung von Diaminodiphenylalkanen ist in der DE-A-14 93 431 beschrieben. Dort wird Siliciumdioxid, Siliciumdioxid-Aluminiumoxid oder säurebehandeltes Aluminiumoxid als Katalysator eingesetzt. Bevorzugt sind Kieselgel oder betonitartiger Ton, der Siliciumdioxid und Aluminiumoxid enthält und vorzugsweise säureaktiviert ist.

Die US 4,071,558 beschreibt ein Herstellungsverfahren zur Herstellung von Diaminodiphenylalkanen, bei dem ein mit Säure aktivierter Tonkatalysator, ein Siliciumdioxid-Aluminiumoxid haltiger Crack-Katalysator oder ein Siliciumdioxid-Magnesiumoxid-Katalysator eingesetzt wird.

Die US 4,039,580 beschreibt ein Herstellungsverfahren, bei dem die Kondensation von Anilin und Formaldehyd in Abwesenheit eines Katalysators ausgeführt wird und das Kondensationsprodukt dann in Gegenwart von Diatomeenerde, Tone oder Zeoliten weiter umgesetzt wird zum Diaminodiphenylmethan. Ähnliche Umsetzungen beschreibt auch die US 4,039,581.

Die Katalysatoren aus der Gruppe Magnesium- oder Aluminiumoxide, Tonkatalysatoren oder Siliciumdioxid-Katalysatoren haben sich aufgrund ihrer hohen Preise, der geringen Aktivitäten, der nicht gleichbleibenden Qualität und mangelhafter Katalysatorstandzeiten nicht bewährt.

Im Stand der Technik wird daher zur Herstellung von Diaminodiphenylalkanen als Katalysator ein Ionenaustauscher vorgeschlagen, der saure Gruppen besitzt. So beschreibt die EP 0 043 933 A1 ein Verfahren zur Herstellung von Polyamingemischen mit einem hohen Anteil an 4,4'-Diaminodiphenylmethan und einem niedrigen Anteil an 2,4'-Diaminodiphenylmethan, bei dem als Katalysator ein Ionenaustauscher auf der Basis eines Divinylbenzol/Styrol-Copolymerisats eingesetzt wird. Dieser Ionenaustauscher besitzt Sulfonsäuregruppen, eine spezifische Oberfläche von 2 bis 40 $m^2$/g und eine Porenweite von 0,5 bis 40 nm. Dieser breite Bereich wird in den Beispielen nur durch Beispiele mit Porenweiten von 1 nm belegt. Als saure Gruppen für den Katalysator werden Sulfonsäuregruppen verwendet. Die Ausbeuten bei diesem Verfahren liegen im Bereich von 60 bis 78 %. Mit dem sulfonierten Styrol-divinylbenzol-copolymer-Katalysator lassen sich Diaminodiphenylmethane herstellen, die einen großen Gehalt an 4,4'-Diaminodiphenylmethan besitzen. Dieses Isomer wird insbesondere benötigt zur weiteren Verarbeitung, nämlich zur Umsetzung in entsprechende Diisocyanate der Diphenylmethan-Reihe, die die Ausgangsmaterialien bei der Herstellung von Polyurethanen darstellen oder als Lackrohstoffe verwendet werden. Die Druckschrift beschreibt weiterhin, dass der Anteil an 2,2'- und 2,4'-Diisocyanatodiphenylmethan-Verbindungen möglichst niedrig sein muss, weil diese Isomere für viele Anwendungsgebiete im Polyisocyanat-Bereich nicht erwünscht sind. Gemäß dem Stand der Technik der EP 0 043 933 werden die erhaltenen Diaminodiphenylmethan-Verbindungen unmittelbar einer Phosgenierung unterzogen, um entsprechende Diisocyanate herzustellen.

Das in der EP 0 043 933 beschriebene Verfahren zur Herstellung von Diaminodiphenylalkanen hat den Nachteil, dass es geringe Ausbeuten besitzt und trotz hoher Reaktionstemperatur sehr lange Reaktionszeiten notwendig sind, um eine technisch vertretbare Ausbeute zu erzielen. Ein weiterer Nachteil ist es, dass bei dem Verfahren des Standes der Technik ein nur geringer Anteil an 2,4'-Isomer entsteht.

Neben aromatischen Isocyanaten sind in einigen speziellen Bereichen die entsprechenden aliphatischen Isocyanate von besonderer Bedeutung.

Die nächste Stufe im Herstellungsprozess von aliphatischen Isocyanaten, ist die Hydrierung des aromatischen Ringes der Diaminodiphenylalkane.

Bei der Hydrierung des Diaminodiphenylmethans (MDA) entsteht aus dem 4,4'-Isomer das 4,4'-trans/trans-, cis/cis- und cis/trans-Diaminodicyclohexylmethan (PACM). Der Gehalt an trans/trans-4,4'-Diaminodicyclohexylmethan hat einen erheblichen Einfluss auf die Kristallisationsneigung des Diisocyanats. Wenn bei dem, aus PACM durch Phosgenierung oder andere Verfahren hergestelltem Diisocyanat, der trans/trans-4,4'-Anteil des Produktes zu hoch ist, kann das Diisocyanatodicylohexylmethan bereits bei Raumtemperatur Kristalle bilden, was für die Weiterverarbeitung zu Polyurethanen hinderlich ist. Vor der Weiterverarbeitung müssen daher aufwendige Verfahrensschritte vorgenommen werden, um den Gehalt an 4,4'-Isomer auf einen akzeptablen Wert zu reduzieren, so dass eine Kristallitbildung nicht mehr auftritt. Dies erfolgt üblicherweise durch Anreicherung des 2,4'-Isomers.

Eine weitere Anforderung an den Isomerengehalt bei der Herstellung des Diaminodiphenylmethans ist es, dass ein möglichst geringer Anteil an 2,2'-Isomer vorhanden sein muss, da dieses Isomer im späteren Verarbeitungsschritt zu Polyurethanen einen Kettenabbruch bei der Polymerisationsreaktion verursacht.

Um diesen Zusatzaufwand zu vermeiden ist es für diesen Reaktionsweg wichtig, dass bereits bei der Herstellung von Diaminodiphenylmethan ein bestimmtes Isomerenverhältnis erzielt wird.

Nach dem bisherigen Stand der Technik wird dieses Isomerenverhältnis dadurch erhalten, dass das Diaminodiphenylmethan in aufwändiger Weise aufgereinigt und destilliert wird, um die Isomeren in dem für die Weiterverarbeitung notwendigen Verhältnis bereitstellen zu können.

Es ist bekannt, cycloaliphatische Amine mit einer oder mehreren Aminogruppen durch katalytische Hydrierung der entsprechenden ein- oder mehrkernigen aromatischen Amine mit einer oder mehreren Aminogruppen und gegebenenfalls weiterer Substituenten herzustellen.

Für die Hydrierung werden häufig Katalysatoren auf Basis von Ruthenium (EP 1 366 812, DE 15 93 293, DE 19 48 566, EP 0 001 425) Rhodium (EP 0 630 882, EP 66 212) oder deren Gemische (US 5,545,756, EP 0 231 788) eingesetzt.

Bei der Hydrierung von Diaminodiphenylmethan, nachfolgend vereinfacht als MDA bezeichnet, zu Methylendicyclohexyldiamin, nachfolgend vereinfacht als $H_{12}$MDA bezeichnet, führen insbesondere Formamide (EP 1 604 972) und MDA-Polymere (EP 1 149 629, EP 0 335 336) zu einer Desaktivierung der Katalysatoren. Wenn Chlorid im MDA enthalten ist, wird dieses vom Katalysator absorbiert und führt nachfolgend zu einer verstärkten Bildung unerwünschter mehrkerniger Verbindungen (EP 0 324 190). Das Chlorid muss in regelmäßigen Abständen durch Waschen des Katalysators mit Wasser entfernt werden. Dieser Waschvorgang mit Wasser führt zum Einen zu Produktionsausfällen. Zum Anderen muss anschließend das Reaktionssystem weitestgehend von Wasser befreit werden, um die Bildung unerwünschter Hydroxyverbindungen zu minimieren.

Der synthetische Zugang zu Isocyanaten kann über eine Reihe unterschiedlicher Routen erfolgen. Älteste und auch heute noch vorherrschende Variante zur großtechnischen Herstellung von Isocyanaten ist die so genannte Phosgenroute. Grundlage dieses Verfahrens ist Umsetzung von Aminen mit Phosgen. Nachteil des Phosgenverfahrens ist der Einsatz von Phosgen, das aufgrund seiner Toxizität, seiner Korrosivität und des hohen Chlorgehalts besonders hohe Anforderungen an seine Handhabung im industriellen Maßstab stellt.

Verfahrenstechnische Ansätze, die es erlauben, die Verwendung von Phosgen zur Herstellung von Isocyanaten in technischen Größenordnungen zu umgehen, sind bekannt. Dabei wird der Begriff phosgenfreies Verfahren wird im Zusammenhang mit der Überführung von Aminen in Isocyanate unter Einsatz alternativer Carbonylierungsmittel, z. B. Harnstoff oder Dialkylcarbonat, benutzt (EP 0 018 586, EP 0 355 443, US 4 268 683, EP 0 990 644).

Grundlage der so genannten Harnstoffroute ist die Harnstoff-vermittelte Überführung von Diaminen in Diisocyanate über einen zweistufigen Prozess. Im ersten Schritt wird ein Diamin mit Alkohol in Gegenwart von Harnstoff oder Harnstoff-Äquivalenten (z. B. Alkylcarbonate, Alkylcarbamate) zu einem Diurethan umgesetzt, welches üblicherweise eine Zwischenreinigungstufe durchläuft und dann im zweiten Schritt thermisch in Diisocyanat und Alkohol gespalten wird (EP 0 355 443, US 4,713,476, US 5,386,053). Alternativ kann der eigentlichen Urethanbildung auch die separate Herstellung eines Diharnstoffs durch gezielte Umsetzung des Diamins mit Harnstoff vorgeschaltet sein (EP 0 568 782). Denkbar ist auch eine zweistufige Sequenz aus partieller Umsetzung von Harnstoff mit Alkohol im ersten und anschließender Zudosierung und Urethansierung des Diamins im zweiten Schritt (EP 0 657 420).

Die thermische Spaltung von Urethanen in die entsprechenden Isocyanate und Alkohole ist seit langem bekannt und kann sowohl in der Gasphase bei hohen Temperaturen als auch bei relativ niedrigen Temperaturen in der Flüssigphase durchgeführt werden. Problematisch ist jedoch bei beiden Verfahrensweisen, dass durch die thermische Belastung grundsätzlich auch unerwünschte Nebenreaktionen stattfinden, die zum einen die Ausbeute mindern und zum anderen zur Bildung verharzender Nebenprodukte führen, die den Ablauf eines technischen Prozesses durch Belegungen und Verstopfungen in Reaktoren und Aufarbeitungsvorrichtungen erheblich stören.

Es hat daher nicht an Vorschlägen gefehlt, durch chemische und verfahrenstechnische Maßnahmen Ausbeuteverbesserungen zu erzielen und die unerwünschte Nebenproduktbildung einzuschränken. So wird in einer Reihe von Dokumenten der Einsatz von Katalysatoren beschrieben, die die Spaltreaktion der Urethane beschleunigen (DE 10 22 222, US 3,919,279, DE 26 35 490). Tatsächlich gelingt es in Gegenwart geeigneter Katalysatoren - hierbei handelt es sich um eine Vielzahl basischer, saurer sowie metallorgansicher Verbindungen - durchaus, die Isocyanatausbeute im Vergleich zur unkatalysierten Variante zu steigern. Die Bildung unerwünschter Nebenprodukte kann jedoch auch durch die Anwesenheit eines Katalysators nicht vermieden werden. Dasselbe gilt für die zusätzliche Verwendung von inerten Lösemitteln, wie sie ebenfalls in der US 3,919,279 und DE 26 35 490 empfohlen werden, um eine gleichmäßige Verteilung der zugeführten Wärme und des Katalysators im Reaktionsmedium zu gewährleisten. Grundsätzlich hat die Verwendung von unter Rückfluss siedenden Lösemitteln jedoch eine Reduzierung der Raum/Zeit-Ausbeute an Isocyanaten zur Folge und ist darüber hinaus mit dem Nachteil eines zusätzlichen hohen Energieaufwands behaftet.

In der EP 0 054 817 angeführte Beispiele zur thermisch geführten katalysierten Spaltung von Monourethanen beschreiben die Teilausschleusung des Reaktionsgemisches zur Abtrennung der im Zuge der Urethanspaltung entstehenden verharzenden Nebenprodukte. Diese Prozedur dient der Vermeidung von Belegungen und Verstopfungen in Reaktoren und Aufarbeitungseinrichtungen. Hinweise, die auf eine Ausbeute steigernde Verwertung der Teilausschleusung hindeuten, gibt es nicht. Die EP 0 061 013 beschreibt einen ähnlichen Lösungsansatz, wobei die Thermolyse in diesem Fall in Gegenwart von Lösemitteln durchgeführt wird, deren Aufgabe offenbar in einer besseren Aufnahme der schwerflüchtigen Nebenprodukten besteht. Auch hier wird die Teilausschleusung nicht im Sinne einer Ausbeuteoptimierung verwertet.

Aus der EP 0 355 443 ist nun bekannt, dass eine Ausbeutesteigerung erzielt werden kann, wenn die während der Spaltung von Diurethanen im Spaltreaktor entstehenden höhermolekularen, verwertbaren und nicht verwertbaren Nebenprodukte zur Gewährleistung einer störungsfreien und selektiven Reaktion möglichst kontinuierlich aus dem Reaktor ausgeschleust werden und anschließend in Gegenwart von Alkohol zum großen Teil umgesetzt und dann in die Diurethanherstellung zurückgeführt werden. Die beschriebene Verfahrensweise ist mit einem hohen Energieaufwand verbunden, da die Abtrennung nicht verwertbarer Nebenprodukte aus dem Austrag der Diurethanherstellung destillativ erfolgt, wobei das gesamte Diurethan verdampft werden muss. Im Unterschied zur EP 0 355 443 wird der Urethanisierungsaustrag beim Verfahren der EP 0 566 925 in zwei Teilströme aufgeteilt, von denen nur einer destillativ von seinen hochsiedenden, nicht verwertbaren Nebenprodukten befreit wird, bevor die vereinigten Diurethanströme der Deblockierungsreaktion im Spaltreaktor zugeführt werden. Zudem wird die kontinuierliche Spaltreaktorausschleusung bei der EP 0 566 925 direkt, d. h. ohne Reurethanisierungsschritt, in die Diurethansynthese zurückgeführt.

Die Vorgehensweise der EP 0 566 925 hat zur Folge, das ein Teil der Hochsiederkomponenten aus der Diurethansynthese über die Deblockierungsstufe wieder zurück in die Diurethan-herstellung und weiter in die Diurethanreinigungsprozedur gelangt.

Mögliche Verfahren, die nicht die Nachteile des dargelegten Stand der Technik aufweisen, auch langfristig eine gute Anlagenverfügbarkeit und eine gute Verfahrensausbeute gewährleisten und zudem die Einsparung von Investment und Energiekosten erlauben, sind in der EP 1 512 680, EP 1 512 681, EP 1 512 682, EP 1 593 669, EP 1 602 643, EP 1 634 868 beschrieben.

Die heute vorherrschende Handelsform großtechnisch hergestellten Harnstoffs sind Prills, d. h. kleine Kügelchen mit einem Durchmesser von 1 - 3 mm. Kristalliner Harnstoff neigt auch bei sehr niedrigen Wassergehalten von < 0,1 % so stark zum Verbacken, dass er für eine lose Lagerung in großen Mengen nicht in Frage kommt. Eine Verbesserung der Lagereigenschaften von Harnstoff-Prills, die beispielsweise bei Silolagerung von großen Mengen notwendig erscheint, wird durch eine nachträgliche Oberflächenbehandlung der Prills mit Puderstoffen wie beispielsweise Talkum, Bentonite, Kieselgur, Diatomeenerde oder andere silikatische Stoffe oder durch Schwefel und auch durch Aufdüsen von geringen Mengen Öl erzielt. Man spricht in diesem Zusammenhang auch von konditioniertem Harnstoff.

Bevorzugt setzt die Harnstoffindustrie heute (Ullmann's Encyclopedia of Industrial Chemistry, Release 2006, 7th Edition) der Harnstoffschmelze vor dem Verprillen bis zu 0,6 Gew.-% Formaldehyd zu, um die Stabilität der Prills zu erhöhen. Diese Maßnahme dient zur Vorbeugung gegen Zerfall und Verbackung beim Transport und zur Verbesserung der Lagerstabilität.

Die Verfahren der EP 1 512 680, EP 1 512 681, EP 1 512 682, EP 1 593 669, EP 1 602 643, EP 1 634 868 erlauben prinzipiell auch den Einsatz von konditioniertem Harnstoff, oder auch einer beliebigen Mischung von konditioniertem und unkonditioniertem Harnstoff. Bevorzugt wird jedoch unkonditionierter Harnstoff, der also keine der Lagerstabilität dienende nachträgliche Oberflächenbehandlung erfahren hat, eingesetzt. Der Harnstoff kann in verschiedenen Darreichungsformen (Prills, Granulat, Kristalle, Schmelze, Lösung) zum Einsatz gelangen.

Allgemeine Aufgabe der vorliegenden Erfindung war es, ein Verfahren zur Herstellung von cycloaliphatischen Diisocyanaten, insbesondere von Methylendicyclohexyldiisocyanat ($H_{12}MDI$) zu finden, dass ausgehend aus den Ausgangsstoffen Aldehyd und aromatisches Amin über die Zwischenstufen eines aromatischen Diamins und eines cycloaliphatischen Diamins ökologisch und ökonomisch optimiert arbeitet. Dabei sollte das Verfahren möglichst ohne Chlor und ohne Chlor haltige Chemikalien als Einsatzstoffe sowie in den Verfahren entstehende Stoffe, wie etwa Salzsäure oder Chloride wie Natriumchlorid, durchzuführen sein. Auf den Einsatz von Phosgen sollte verzichtet werden. Außerdem sollten die Umsatzraten (Ausbeute) der jeweiligen Einsatzstoffe zu den jeweiligen Endprodukten, unter Berücksichtigung der jeweiligen vorteilhaften Isomerenverteilung, hoch sein.

Die technische Aufgabe der Erfindung war es auch, in einer ersten Stufe ein Verfahren zur Herstellung von Diaminodiphenylalkanen zur Verfügung zu stellen, das direkt zu dem spezifischen Isomerenverhältnis führt, das man für die Weiterverarbeitung zum cycloaliphatischen Diamin und der entsprechenden Diisocyanate benötigt. Eine weitere technische Aufgabe der Erfindung ist es, ein Verfahren zu entwickeln das wirtschaftlicher arbeitet, weil es geringere Reaktionsdauer benötigt und zu den gewünschten Ausbeuten der Isomeren führt. Gleichzeitig soll auch das Entstehen von Abwasser mit Salzfracht insbesondere von chlorhaltigen Salzen möglichst gering werden.

Die Aufgabe wurde durch das vorliegende Chlor arme mehrstufige kontinuierliche Verfahren wie unten näher ausgeführt gelöst.

Gegenstand der Erfindung ist ein Chlor armes mehrstufiges und kontinuierliches Verfahren zur Herstellung von cycloaliphatischen Diisocyanaten durch

1. Chlor freie Herstellung von aromatischen Diaminen (Diaminodiphenylalkanen), wobei ein aromatisches Amin, das substituiert oder unsubstituiert sein kann, mit einem $C_1$-$C_3$ Aldehyd in Gegenwart eines heterogenen Katalysators umgesetzt wird, wobei der Katalysator ein mesoporöser saurer Ionenaustauscher auf Basis eines Divinylbenzol/Styrol-Copolymerisates ist und der Katalysator acide Zentren in einer Konzentration von 2 bis 6 eq/kg nach DIN 54403 aufweist und der durchschnittliche Porendurchmesser der Katalysatorteilchen gemessen nach ASTM D 4222 1 bis 50 nm beträgt;
2. Chlor freie Hydrierung der aromatischen Diamine zu cycloaliphatischen Diaminen durch Umsetzung der aromatischen Diamine mit Wasserstoff in Gegenwart eines Katalysators;
3. Chlor freie oder Chlor arme und kontinuierliche Herstellung von cycloaliphatischen Diisocyanaten, durch Umsetzung von cycloalipahtischen Diaminen mit Kohlensäurederivaten wie Harnstoff und/oder Harnstoffäquivalenten und Alkoholen zu cycloaliphatischen Diurethanen und anschließenden thermischen Spaltung der Diurethane zu cycloaliphatischen Diisocyanten.

Vorteile des Verfahrens:

Das Verfahren wird in der 1. und 2. Stufe völlig ohne Chlor und Chlor haltige Chemikalien durchgeführt und arbeitet somit Chlor frei. Dabei entstehen während dieser Verfahrensstufen kein Chlor und keine Chlor haltigen Chemikalien. Dadurch wird zum einen erreicht, dass keine Chlor haltigen Verbindungen oder Chlor selbst in die Umwelt, insbesondere in die Luft und ins Abwasser gelangt. Des Weiteren werden die Anlagen zur Durchführung des Verfahrens vor schädlicher Korrosion geschützt. In der 3. Stufe wird auf Phosgen gänzlich verzichtet. Durch diese Maßnahmen werden die Anlagen ebenfalls vor schädlicher Korrosion geschützt und die Belastung für die Umwelt wird minimiert.

Besondere Vorteile der einzelnen Stufen:

1. Stufe

Durch den eingesetzten sauren Ionenaustauscher kann in der 1. Stufe auf die normalerweise eingesetzte Salzsäure verzichtet werden. Es war außerdem überraschend, dass durch den sauren Ionenaustauscher das gewünschte Isomerenverhältnis aus 2,2'-, 2,4', und 4,4-Diaminoaliphenylalkane erhalten werden konnte. Ebenso war es überraschend, dass die Entstehung von N-Methylverbindungen weitgehend zurückgedrängt werden konnte.

2. Stufe

Vorteile des Verfahrens bei der Hydrierung des aromatischen Diamins zum cycloaliphatischen Diamins sind erstens eine erhöhte Aktivität des Katalysators und eine erhöhte Standzeit des Katalysators bedingt durch den Verzicht in der 1. und 2. Stufe des Verfahrens auf Chlor und Chlor haltige Chemikalien. Außerdem wurde das angestrebte Ziel eines weiter unten näher beschriebenen trans-trans-Gehaltes der hydrierten 4,4'-Diamine erreicht.

3. Stufe

In dieser 3. Stufe ist es sehr bedeutsam, dass die Reaktion des cycloaliphatischen Diamins zum Diisocyanat ohne Phosgen durchgeführt werden kann. Der angestrebte spezifische trans-trans-Gehalt des 4,4'-Diisocyanates bleibt während der Umsetzung erhalten. Je nach Art des Katalysators, der in der 3. Stufe eingesetzt wird, ist auch diese 3. Stufe Chlor frei, wenn ein Katalysator ohne Chlor verwendet wird. Wird ein Katalysator mit Chlor eingesetzt, arbeitet diese 3. Stufe Chlor arm, was bedeutet, dass der Gehalt an Chlor (berechnet als Chlor-Ion, M = 35,45 g/mol) kleiner als 100 ppm, bevorzugt kleiner 50 ppm, besonders bevorzugt kleiner 20 ppm, beträgt, bezogen auf alle sich in der 3. Stufe anwesenden Mengen an Stoffen.

Nachfolgend wird das Verfahren mit seinen einzelnen Stufen näher beschrieben.

1.Stufe

Diese technische Aufgabe in der ersten Stufe des erfindungsgemäßen Verfahrens wird gelöst durch ein Verfahren zur Herstellung von Diaminodiphenylalkanen wobei ein aromatisches Amin, das substituiert oder unsubstituiert sein kann, mit einem C1-C3 Aldehyd in Gegenwart eines heterogenen Katalysators umgesetzt wird, wobei der Katalysator ein mesoporöser saurer Ionenaustauscher auf Basis eines Divinylbenzol/Styrol-Copolymerisates ist und der Katalysator acide Zentren in einer Konzentration von 2 bis 6 eq/kg nach DIN 54403 aufweist und der durchschnittliche Porendurchmesser der Katalysatorteilchen gemessen nach ASTM D4222 1 bis 50 nm beträgt.

Unter mesoporösen Ionenaustauschern werden solche verstanden, die einen mittleren Porendurchmesser gemessen nach ASTM D 4222 von 1 bis 50 nm besitzen. Bevorzugt werden jedoch mesoporöse Ionenaustauscher mit einem durchschnittlichen Porendurchmesser der Katalysatorteilchen gemessen nach ASTM D 4222 von 10 bis 32 nm eingesetzt.

In einer bevorzugten Ausführungsform beträgt die Konzentration der aciden Zentren des Ionenaustauschers 4,4 bis 5,7 eq/kg (eq/kg bedeutet Equivalente pro Kilogramm Ionenaustauscher) und ganz besonders bevorzugt 4,7 bis 5,6 eq/kg, gemessen nach DIN 54 403.

Die aciden Zentren sind bevorzugt Säuregruppen und besonders bevorzugt Sulfonsäuregruppen sind.

Unter mesoporösen Ionenaustauschern werden solche verstanden, die einen mittleren Porendurchmesser gemessen nach ASTM D 4222 von 2 bis 50 nm besitzen. Erfindungsgemäß werden jedoch mesoporöse Ionenaustauscher mit einem durchschnittlichen Porendurchmesser der Katalysatorteilchen gemessen nach ASTM D 4222 von 10 bis 32 nm eingesetzt.

Der Porendurchmesser der Ionenaustauscher gemessen nach ASTM D 4222 beträgt vorzugsweise 15 bis einschließlich 30 nm und ganz besonders bevorzugt 22 bis einschließlich 30 nm.

Die Eigenschaften dieser Harze, insbesondere spezifische Oberfläche, Porosität, Stabilität und Austauschkapazität, können durch den Herstellungsprozess variiert werden. So werden z. B. die Größe der Poren und ihre Verteilung durch Zusatz von Porogenen beeinflusst. Porogene sind inerte, organische Substanzen, z. B. Lösungsmittel oder Fällungsmittel, die während der radikalischen Suspensionspolymerisation in den Polymerisationsprozess nicht einbezogen werden. Sie werden nach der Polymerisation wieder aus dem Polymeren entfernt und sind zusammen mit dem Vernetzeranteil für den Grad der Porosität verantwortlich. Porogene dienen als Lösungsmittel für die Monomere und als Fällungsmittel für die entstandenen Polymere. Als Porogene werden in der Suspensionpolymerisation z. B. Isopropanol, Toluol, Heptan, Paraffinwachs, Benzin, Amylalkohol oder Nitromethan verwendet.

Zum Einsatz können saure Harze des Divinylbenzol/Styrol-Copolymersats kommen, die unter folgenden Handelsnamen verkauft werden: Duolite[R], Amberlyst[R], Amberlite[R], Dowex[R], Lewatit[R].

Als Aldehyd wird vorzugsweise Formaldehyd eingesetzt. Formaldehyd kann als wässrige Formalinlösung oder auch gasförmig als Formaldehyd eingesetzt werden. Weiterhin können auch Stoffe verwendet werden, die unter den Reaktionsbedingungen Formaldehyd abspalten, wie beispielsweise Trioxymethylen oder Paraformaldehyd. In bevorzugter Weise wird für die Reaktion eine wässrige Formalinlösung eingesetzt.

Als aromatische Amine können substituierte oder unsubstituierte Amine eingesetzt werden. In bevorzugter Weise sollten die Amine, falls sie substituiert sind, keinen Substituenten in Parastellung besitzen. Geeignete aromatische Amine sind beispielsweise N-Methylanilin, N-Ethylanilin, o-Toluidin, o-Chloranilin, m-Chloranilin, o-Anisidin, 2,3-Xylidin, 3,5-Xylidin, o-Cyclohexylanilin, o-Benzylanilin, alpha-Naphtylanilin, Methylmercaptoanilin oder Anilin. Besonders bevorzugt als aromatisches Amin ist die Verwendung von Anilin.

Bei dem erfindungsgemäßen Herstellungsverfahren werden folgende Zusammensetzung und Isomerenverhältnisse erhalten, die bevorzugt wie folgt verteilt sind:

64 bis.85 Gew.-% 4,4'-Diaminodiphenylalkan,
3 bis 20 Gew.-%, vorzugsweise 7 bis 17 Gew.-% 2,4'-Diaminodiphenylalkan
und

≤ 2 Gew.-%, 2,2'-Diaminodiphenylalkan.

Mit dem erfindungsgemäßen Verfahren wird vorzugsweise Diaminodiphenylmethan der nachfolgenden Isomerenzusammensetzung hergestellt:

64 bis 85 Gew.-% 4,4'-Diaminodiphenylmethan,
3 bis 20 Gew.-%, vorzugsweise 7 bis 17 Gew.-%, 2,4'-Diaminodiphenylmethan und
≤ 2 Gew.-%, 2,2'-Diaminodiphenylmethan.

Der Anteil an Mehrkernverbindungen im Isomerengemisch des Diaminodiphenylalkans, insbesondere des Diaminodiphenylmethans, beträgt ≤ 15 Gew.-%, in bevorzugter Weise < 15 Gew.-%. Unter Mehrkernverbindungen versteht man im Rahmen der Erfindung solche Moleküle mit mehr als zwei aromatischen Kernen, insbesondere Phenylringe.
Bei dem erfindungsgemäßen Herstellungsverfahren werden folgende Zusammensetzung und Isomerenverhältnisse erhalten, die besonders bevorzugt wie folgt verteilt sind:

74 bis 85 Gew.-% 4,4'-Diaminodiphenylalkan,
3 bis 20 Gew.-%, vorzugsweise 7 bis 15 Gew.-%, 2,4'-Diaminodiphenylalkan und
≤ 1 Gew.-%, vorzugsweise ≤ 0,8 Gew.-%, 2,2'-Diaminodiphenylalkan.

Mit dem erfindungsgemäßen Verfahren wird vorzugsweise Diaminodiphenylmethan (MDA) der nachfolgenden Isomerenzusammensetzung hergestellt:

74 bis 85 Gew.-% 4,4'-Diaminodiphenylmethan,
3 bis 20 Gew.-%, vorzugsweise 7 bis 15 Gew.-%, 2,4'-Diaminodiphenylmethan und
≤ 1 Gew.-%, vorzugsweise ≤ 0,8 Gew.-%, 2,2'-Diaminodiphenylmethan.

Der Anteil an Mehrkernverbindungen in dieser besonders bevorzugten Variante im Isomerengemisch des Diaminodiphenylalkans, insbesondere des Diaminodiphenylmethans, beträgt ≤ 10 Gew.-%, in bevorzugter Weise < 10 Gew.-%.
Unter Mehrkernverbindungen versteht man im Rahmen der Erfindung Moleküle mit mehr als zwei aromatischen Kernen, insbesondere Phenylringe.
Die Verunreinigungen durch N-Methylverbindungen im Isomerengemisch des Diaminodiphenylalkans, insbesondere Diaminodiphenylmethan, betragen ≤ 1,0 Gew.-%, vorzugsweise ≤ 0,5 Gew.-% und besonders bevorzugt ≤ 0,3 Gew.-%. Diese Isomerenverteilung ist besonders geeignet, um über Diaminodicyclohexylmethan zu der entsprechenden Diisocyanatodicyclohexylmethan-Verbindung weiterverarbeitet zu werden. Das Isomerenverhältnis, das beim erfindungsgemäßen Verfahren erhalten wird, bestimmt auch das anschließende Isomerenverhältnis in der Diisocyanat-Verbindung.
Mit dem erfindungsgemäßen Verfahren wird bereits bei der Diaminodiphenylalkan-Herstellung das geforderte Isomerenverhältnis erreicht, ohne dass durch zusätzliche Trennungsverfahren, die aufwändig und teuer sind, dieses Isomerenverhältnis erst eingestellt werden muss. Auch sind diese Aufreinigungsverfahren deshalb nachteilig weil es sich hier um sehr reaktive Substanzen handelt, sodass auch bei Aufreinigung oder Destillation mögliche Nebenreaktionen auftreten können.
Ein weiterer Vorteil des erfindungsgemäßen Verfahrens ist es, dass der Anteil an unerwünschten Nebenprodukten, insbesondere N-Methylverbindungen, sehr gering ist. N-Methylverbindungen führen bei der Weiterverarbeitung zum Diisocyanat zu unerwünschten Monoisocyanaten und damit zur Qualitätverschlechterung des Produktes.
Die Verfahren des Standes der Technik, die mit sauren Ionenaustauschern als Katalysatoren arbeiten, führen zu deutlich höheren Isomerenanteilen von 4,4'-Diaminodiphenylalkan. So liegen die entsprechenden Gehalte in den Beispielen der Druckschrift EP 0 043 933 bei 94 Gew.-%, 92 Gew.-% und 91 Gew.-%. Diese Isomerengehalte sind jedoch zu hoch, da bei der Weiterverarbeitung zur aliphatischen Diisocyanat-Verbindung eine Kristallisation eintritt, die unerwünscht ist.
Die gemäß EP 0 043 933 erhaltenen Diaminodiphenylalkane müssen daher durch zusätzliche Verfahrensschritte, wie Destillation, auf das gewünschte Isomerenverhältnis, wie oben bereits näher beschrieben, eingestellt werden.
Ein weiterer Vorteil des erfindungsgemäßen Verfahrens liegt in seiner ökologischen und ökonomischen Ausführung. In bevorzugter Weise wird die katalytische Reaktion bei einer Reaktionstemperatur im Bereich von 80 bis 140 °C, besonders bevorzugt 80 bis 130 °C und ganz besonders bevorzugt 80 bis 120 °C ausgeführt. Die benötigte Reaktionszeit für die katalytische Reaktion beträgt vorzugsweise 30 Minuten bis 5 Stunden, besonders bevorzugt 0,75 Stunden bis 4,5 Stunden und ganz besonders bevorzugt 0,75 bis 3,0 Stunden.

Diese Reaktionszeiten sind im Vergleich zur EP 0 043 933 erheblich niedriger. Aus den Beispielen der Druckschrift geht hervor, dass die Reaktionszeiten hier bei bis zu 20 Stunden liegen und daher etwa zwanzig Mal höher sind als bei dem erfindungsgemäßen Verfahren. Die Reaktionstemperatur ist ähnlich hoch und dies bedeutet einen etwa zwanzigfach höheren Energieaufwand, um die Reaktion durchzuführen.

Das erfindungsgemäße Verfahren besitzt daher den Vorteil, dass es kostengünstiger und wirtschaftlicher durchgeführt werden kann und auch aufgrund der kürzeren Reaktionsdauer einen größeren Umsatz pro Zeiteinheit bietet.

Auch die Ausbeuten des erfindungsgemäßen Verfahrens sind höher als im Stand der Technik. So liegen die Ausbeuten gemäß EP 0 043 933 in den Beispielen im Bereich von 60 bis 78 %. Im Vergleich dazu werden bei dem erfindungsgemäßen Verfahren Gesamtausbeuten von 80 bis 95 % erhalten.

In bevorzugter Weise werden die Ausgangssubstanzen aromatisches Amin und Aldehyd in einem Verhältnis von 5 : 1 bis 15 : 1, vorzugsweise 7 : 1 bis 12 : 1 und ganz besonders bevorzugt 10 : 1 eingesetzt. Es ist bevorzugt, dass das Amin im Überschuss eingesetzt wird, da hierdurch die Selektivität erhöht wird. Überschüssiges Amin kann nach Abschluss der Reaktion abdestilliert werden.

Als Katalysatoren werden mesoporöse, saure Ionenaustauscher basierend auf Divinylbenzol/Styrol-Copolymersaten verwendet wobei der Katalysator acide Zentren in einer Konzentration von 2 bis 6 eq/kg nach DIN 54403 aufweist. Der Katalysator kann in trockenem oder feuchtem Zustand eingesetzt werden. Als acide Gruppen werden bevorzugt Sulfonsäuregruppen verwendet. Der Katalysator wird z. B. hergestellt durch Mischpolymerisation von Styrol mit Divinylbenzol und Sulfonierung mit Schwefelsäure/Oleum.

Das erfindungsgemäße Verfahren kann in bevorzugter Weise kontinuierlich, diskontinuierlich oder semikontinuierlich durchgeführt werden.

In bevorzugter Ausführung wird die Reaktionsdurchführung in einem Rührkessel, einer Rührkesselkaskade, einem Strömungsrohr, einem oder mehreren Festbettreaktoren oder einer Kolonne durchgeführt. Die katalytische Reaktion wird an einem heterogenen Katalysator durchgeführt.

Für die Durchführung des erfindungsgemäßen Verfahrens werden die Ausgangssubstanzen aromatisches Amin und Aldehyd kontinuierlich oder diskontinuierlich gemischt. Es erfolgt dann die katalytische Reaktion bei Temperaturen im Bereich von 80 bis 140 °C. Anschließend wird das Isomerengemisch der Diaminodiphenylalkane nach üblichen Trennmethoden isoliert.

2.Stufe

In der 2. Stufe ist der Gegenstand der Erfindung ein Verfahren zur Hydrierung der aromatischen Diamine zu cycloaliphatischen Diaminen durch Umsetzung der aromatischen Diamine mit Wasserstoff in Gegenwart eines Katalysators.

Prinzipiell sind alle aromatischen Diamine aus der 1. Stufe zur Hydrierung zu cycloaliphatischen Diaminen geeignet. Ganz besonders bevorzugt ist die Hydrierung von MDA zu Methylendicyclohexyldiamin ($H_{12}$MDA).

Als Katalysatoren können prinzipiell alle Verbindungen eingesetzt werden, die die Hydrierung von Phenylgruppen katalysieren. Dies können homogene oder heterogene Katalysatoren sein, bevorzugt sind heterogene Katalysatoren.

Insbesondere haben sich Katalysatoren auf Basis von Nickel, Cobalt, Palladium, Platin, Ruthenium und Rhodium allein oder in Mischung als geeignet erwiesen.

Zur Erhöhung der Aktivität, Selektivität und/oder Standzeit können die Katalysatoren zusätzlich Dotiermetalle oder andere Modifizierungsmittel enthalten. Typische Dotiermetalle sind z. B. Mo, Fe, Ag, Cr, Ni, V, Ga, In, Bi, Ti, Zr und Mn sowie die seltenen Erden. Typische Modifizierungsmittel sind z. B. solche, mit denen die Säure-Base-Eigenschaften der Katalysatoren beeinflusst werden können, wie z. B. Alkali- und Erdalkalimetalle bzw. deren Verbindungen sowie Phosphorsäure oder Schwefelsäure sowie deren Verbindungen. Die Katalysatoren können in Form von Pulvern oder Formkörpern, wie z. B. Extrudaten oder gepressten Pulvern, eingesetzt werden. Es können Vollkontakte, Raney-Typ-Katalysatoren oder Trägerkatalysatoren zur Anwendung kommen. Geeignete Trägermaterialien sind z. B. Aktivkohle, anorganische Oxide, insbesondere Al2O3, SiO2, TiO2, ZrO2, ZnO und MgO, ferner Bentonite, Alumosilikate, Kaoline, Tone und Kieselguren sowie die in den Schriften EP 1 604 972 und EP 1 767 520 beschriebenen Lithiumaluminate. Das Aktivmetall kann in dem Fachmann bekannter Weise auf das Trägermaterial aufgebracht werden, wie z. B. durch Imprägnierung, Aufsprühen oder Fällung. Je nach Art der Katalysatorherstellung sind weitere, dem Fachmann bekannte Präparationsschritte notwendig, wie z. B. Trocknung, Calcinierung, Formgebung und Aktivierung. Zur Formgebung können optional weitere Hilfsstoffe wie z. B. Graphit oder Magnesiumstearat zugesetzt werden.

Bevorzugt eingesetzt werden Trägerkatalysatoren mit Ruthenium, Rhodium oder Rh/Ru-Kombinationen als wesentlichen Aktivmetallen. Bevorzugte Trägermaterialien sind solche auf Basis von Al2O3 und SiO2.

Bevorzugt werden solche Katalysatoren eingesetzt, von denen bekannt ist, dass mit ihnen ein $H_{12}$MDA mit einem trans-trans-Anteil des 4,4'-Isomeren zwischen 10 und 30 %, insbesondere zwischen 15 und 25 % hergestellt werden kann. Solche Katalysatoren sind beispielsweise in den Dokumenten EP 1 366 812, EP 0 066 211, DE 100 54 347, EP 0 392 435, EP 0 630 882, EP 0 639 403 und US 5,545,756 beschrieben.

Besonders bevorzugt wird in Gegenwart eines geträgerten Katalysators, der als Aktivmetall Ruthenium alleine oder zusammen mit mindestens einem Metall der I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, hydriert.

Es ist bekannt, dass der Anteil des trans-trans-4,4'-Isomeren nicht nur vom Katalysator alleine abhängig ist, sondern auch von der Reaktionstemperatur, dem Umsatz und der Verweilzeit im Reaktor. Die Zusammenhänge kann der Fachmann den Lehren der bereits zitierten Dokumente sowie einem Artikel von G.F. Allen (Chem. Ind. 1988, 33, S. 323 - 338) entnehmen. Um gezielt und reproduzierbar ein $H_{12}$MDA mit einem bestimmten trans-trans-Anteil des 4,4'-Isomeren herstellen zu können ist es deshalb von Bedeutung, die Temperatur, den Umsatz und die Verweilzeit im Reaktor genau zu kontrollieren. Zwar ist dies prinzipiell auch in einem absatzweise betriebenen Reaktor möglich, aber bevorzugt wird die Hydrierung in kontinuierlich betriebenen Reaktoren durchgeführt. Geeignete Reaktoren für die kontinuierliche Hydrierung sind dem Fachmann geläufig und z. B. in dem Übersichtsartikel "Reactor types and their industrial application" in Ullmann's Encyclopedia of Industrial Chemistry, 7th online-edition 2007, beschrieben.

In einer bevorzugten Ausführungsform der Erfindung wird die kontinuierliche Hydrierung der aromatischen Diamine, insbesondere MDA in Festbettreaktoren durchgeführt. Als vorteilhaft hat es sich erwiesen, die Reaktion in zwei oder mehr in Reihe geschalteten Reaktionsräumen durchzuführen. Der Vorteil dieser Reaktionsführung besteht vor allem darin, dass die Reaktionsräume unabhängig voneinander geheizt oder gekühlt werden können und sich dadurch bessere Steuerungsmöglichkeiten des trans-trans-4,4'-$H_{12}$MDA-Anteils ergeben. Ein weiterer Vorteil besteht darin, dass ein Nachlassen der Katalysatoraktivität gezielter durch eine Temperaturanpassung ausgeglichen werden kann und bei Bedarf partielle Katalysatorwechsel möglich sind. Die getrennten Reaktionsräume können z. B. durch zwei oder mehr in Reihe geschaltete Festbettreaktoren, wie z. B. Rohrbündelreaktoren und/oder Schachtöfen, realisiert werden. Eine andere Möglichkeit besteht darin, in einem Reaktor räumlich voneinander getrennte Katalysatorschüttungen unterzubringen, die durch dem Fachmann geläufige Verfahren geheizt oder gekühlt werden können. Die Festbettreaktoren können in Sumpffahrweise betrieben werden, bevorzugt wird aber eine Rieselbettfahrweise. Der LHSV-Wert liegt im Bereich von 0,01 bis 1 $h^{-1}$ (=l des zu hydrierenden aromatischen Amins pro l Festbettkatalysator und Stunde). Die Hydrierung erfolgt bei Temperaturen im Bereich von 50 bis 200 °C, vorzugsweise zwischen 80 und 170 °C. Der Wasserstoffdruck liegt zwischen 1 und 30 MPa, bevorzugt zwischen 5 und 15 MPa.

Bevorzugt wird eine Ausführungsform, in der das aromatische Diamin, insbesondere das MDA, in einem Lösungsmittel hydriert wird. Der Anteil des Lösungsmittels liegt zwischen 10 und 90 %, bevorzugt zwischen 50 und 90 % bezogen auf die Masse der Lösung. Geeignete Lösungsmittel sind beispielsweise primäre, sekundäre und tertiäre ein- oder mehrwertige Alkohole, wie Methanol, Ethanol, n- und i-Propanol und 1-, 2-, i- und tert.-Butanol, Ethylenglykol, Ethylenglykolmono(C1-C3)alkylether; lineare Ether, wie Ethylenglykoldi($C_1$-$C_3$)alkylether, cyclische Ether wie Tetrahydrofuran und Dioxan; Alkane, wie n- und iso-Alkane mit 4-12 C-Atomen, z. B. n-Pentan, n-Hexan und Isooctan, und zyklische Alkane, wie Cyclohexan und Dekalin. Während Alkohole zu einer Alkylierung der Aminogruppen führen können, weisen Ether diesen Nachteil nicht auf. Bevorzugtes Lösungsmittel ist Tetrahydrofuran. Lösungsmittel kann auch das Hydrierprodukt selbst sein.

Die Hydrierung kann auch in Gegenwart von Ammoniak oder eines primären, sekundären oder tertiären Amins oder eines polyzyklischen Amins mit einem verbrückendem N-Atom durchgeführt werden.

Es ist besonders bevorzugt, dass man als zu hydrierendes Stoffgemisch ein Roh-MDA, enthaltend mindestens 70 Gew.-% 4,4'-Diaminodiphenylmethan und 0,01 bis 2 Gew.-% N-Methylverbindugen einsetzt. Ebenfalls besonders bevorzugt ist, dass das zu hydrierende Stoffgemisch 74 -85 Gew.-% 4,4'-MDA, 3 -20 Gew.-% 2,4'-MDA, weniger als 1 Gew.-% 2,2'-MDA und bis zu 1 Gew.-% N-Methylverbindungen enthält. Es ist ebenfalls besonders bevorzugt, dass ein H12MDA Stoffgemisch mit einem trans-trans-Anteil des 4,4'-Isomeren zwischen 10 und 30 %, insbesondere zwischen 15 und 25 % in der 2. Stufe erhalten wird, wobei, wie oben beschrieben, die Reaktionsbedingungen und der Katalysator entsprechend ausgewählt werden.

3. Stufe

Allgemein

In der 3. Stufe des Verfahrens ist Gegenstand der Erfindung ein mehrstufiges Verfahren zur Chlor freien oder Chlor armen und kontinuierlichen Herstellung von cycloaliphatischen Diisocyanaten, durch Umsetzung von cycloaliphatischen Diaminen mit Kohlensäurederivaten (Harnstoff oder Harnstoffäquivalenten wie z. B. Alkylcarbonate, Alkylcarbamate)) und Alkoholen zu cycloaliphatischen Diurethanen und anschließenden thermischen Spaltung der Diurethane zu cycloaliphatischen Diisocyanten, insbesondere dadurch, dass die cycloaliphatischen Diurethane nah einstufigen, zweistufigen oder alternativ auch mehrstufigen Verfahren hergestellt werden, nach ihrer Synthese durch Umsetzung von cycloaliphatischen Diaminen mit Alkohol und Harnstoff und/oder Harnstoff-Derivate von Leicht-, Mittel- und gegebenenfalls Hochsiedern befreit, die so gereinigten cycloaliphatischen Diurethane unter Freisetzung des gewünschten Diisocyanats thermisch gespalten, einen Teil des Spaltsumpfes aus der Spaltapparatur kontinuierlich ausgeschleust und nach Aufarbeitung oder alternativ ohne zusätzliche Reinigung mit Alkohol reurethanisiert und in den Prozess recycliert wird.

Variante I

Gegenstand der Erfindung in der 3. Stufe ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Harnstoff und/oder Harnstoffderivaten und Alkoholen in cycloaliphatische Diurethane, und deren thermische Spaltung, wobei

a) cycloaliphatische Diamine der Formel (II)

$H_2N-R-NH_2$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

$R^1$-OH

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamid-säurealkylestern oder Mischungen aus Dialkylcarbonaten und Carbamidsäureestern und in Abwesenheit oder Gegenwart von Katalysatoren zu cycloaliphatischen Diurethanen, umgesetzt werden und das entstehende Ammoniak gleichzeitig abtrennt wird;

b) aus der erhaltenen Reaktionsmischung der Alkohol, die Dialkylcarbonate und/oder Carbamidsäurealkylester abtrennt werden und der Alkohol sowie optional die Dialkylcarbonate und/oder Carbamidsäurealkylester in die Reaktionsstufe a) zurückführt werden;

c) der im Wesentlichen Diurethane enthaltende Stoffstrom aus Stufe b) destillativ in einen Wertstoffstrom und einen Nebenproduktstrom, der ausgeschleust wird, getrennt wird;

d) die über die Schritte b) und c) aufgereinigten Diurethane enthaltende Reaktionsmischung in Gegenwart eines Katalysators lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so gespalten wird, dass ein Teil des Reaktionsgemisches von 10 - 60 Gew.-% bezogen auf den Feed, vorzugsweise 15 - 45 Gew.-% bezogen auf den Feed, ständig ausgeschleust wird;

e) die Spaltprodukte durch Rektifikation in ein rohes cycloaliphatisches Diisocyanat und Alkohol getrennt werden;

f) das rohe cycloaliphatische Diisocyanat, durch Destillation gereinigt und die Reinproduktfraktion isoliert wird;

g) die Sumpfausschleusung aus d) mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 -170 °C und bei einem Druck von 0,5 - 20 bar, vorzugsweise 1-15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;

h) ein Teil der Sumpffraktion der Reindestillation f) kontinuierlich ausschleust und in die Spaltreaktion d), vorzugsweise aber die Urethanisierungsstufe g) geführt wird;

i) die bei der Reindestillation des cycloaliphatischen Diisocyanats anfallende Kopffraktion ebenfalls in die Urethanisierungsstufe g) zurückgeführt oder verworfen wird;

j) der Reurethanisatstrom aus g) in Stufe b) zurückgeführt wird,
oder

k) der Reurethanisatstrom aus g) in die Reaktionsstufe a) zurückgeführt wird, unter der Voraussetzung, das g) in Gegenwart von Katalysatoren bevorzugt ausgewählt aus den Halogeniden von Fe(III) und/oder Cu(I), durchgeführt wird.

Nach dem erfindungsgemäßen Verfahren können cycloaliphatische Diisocyanate im kontinuierlichen Betrieb problemlos mit sehr guten Selektivitäten hergestellt werden. Vorteilhaft bei dem erfindungsgemäßen Mehrstufenverfahren ist insbesondere die Tatsache, dass auch bei Einsatz von cycloaliphatischen Diaminen der Formel (II) als Ausgangsmaterial für die kontinuierliche Diisocyanatsynthese über einen langen Zeitraum eine mit hoher Selektivität störungsfrei ablaufende Reaktion gewährleistet wird.

Variante II

Gegenstand der Erfindung in der 3. Stufe ist auch ein mehrstufiges Verfahren zur kontinuierlichen Herstellung von cycloaliphatischen Diisocyanaten der Formel (I)

$$OCN-R-NCO$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, durch Umsetzung von cycloaliphatischen Diaminen mit Harnstoff und/oder Harnstoffderivaten und Alkoholen in cycloaliphatische Diurethane und deren thermische Spaltung, wobei

a) cycloaliphatische Diamine der Formel (II)

$$H_2N-R-NH_2$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

$$R^1-OH$$

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt,

in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren, zu cycloaliphatischen Diurethanen umgesetzt werden und man das entstehende Ammoniak gleichzeitig abtrennt;

b) aus der erhaltenen Reaktionsmischung der Alkohol, die Dialkylcarbonate und/oder Carbamidsäurealkylester abgetrennt werden und man den Alkohol sowie optional auch die Dialkylcarbonate und/oder Carbamidsäurealkylester, in die Reaktionsstufe a) zurückführt;

c) auf eine Abtrennung der in der erhaltenen Reaktionsmischung gegebenenfalls enthaltenen hochsiedenden Rückstände vollständig oder partiell verzichtet wird;

d) die über die Schritte b) und optional c) aufgereinigten Diurethane enthaltende Reaktionsmischung in Gegenwart eines Katalysators kontinuierlich und lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so spaltet, dass ein Teil des Reaktionsgemisches von 10 - 60 Gew.-%, bezogen auf den Feed, vorzugsweise 15 - 45 Gew.-%,

bezogen auf den Feed, ständig ausgeschleust wird;

e) die Spaltprodukte durch Rektifikation in ein rohes cycloaliphatisches Diisocyanat und Alkohol getrennt werden;

f) das rohe cycloaliphatische Diisocyanat, durch Destillation gereinigt und die Reinproduktfraktion isoliert wird;

g) die Sumpfausschleusung aus d) partiell oder vollständig mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1 - 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;

h) der Reurethanisatstrom aus g) in einen Wertstoff- und einen Abfallstrom aufgetrennt und der an Hochsiederkomponenten reiche Abfallstrom aus dem Prozess ausgeschleust und verworfen wird;

i) ein Teil der Sumpffraktion der Reindestillation f) kontinuierlich ausschleust und in die Spaltreaktion d) oder in die Urethanisierungsstufe g) geführt wird;

j) optional die bei der Reindestillation f) des rohen cycloaliphatischen Diisocyanats anfallende Kopffraktion ebenfalls in die Urethanisierungsstufe g) zurückgeführt wird;

k) der Wertstoffstrom aus h) in Stufe a), b) oder d) zurückgeführt wird.

Nach dem erfindungsgemäßen Verfahren können cycloaliphatische Diisocyanate im kontinuierlichen Betrieb problemlos mit sehr guten Ausbeuten hergestellt werden. Vorteilhaft bei dem erfindungsgemäßen Mehrstufenverfahren ist insbesondere die Tatsache, dass auch bei Einsatz von cycloaliphatischen Diaminen der Formel (II) als Ausgangsmaterial für die kontinuierliche Diisocyanatsynthese Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Weiterhin ist es ein Vorteil des erfindungsgemäßen Mehrstufenverfahrens, dass es erlaubt, die Menge des in die Dampfphase zu überführenden Diurethans auf ein Minimum zu verringern und auf diese Weise den notwendigen Energieaufwand beschränkt.

Variante III

Gegenstand der Erfindung in der 3. Stufe ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, durch Umsetzung von cycloaliphatischen Diaminen mit Harnstoff und/oder Harnstoffderivaten und Alkoholen in cycloaliphatische Diurethane und deren thermische Spaltung, wobei

a) cycloaliphatische Diamine der Formel (II)

$H_2N$-R-$NH_2$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

$R^1$-OH

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamid-säurealkylestern oder Mischungen aus Dialkylcarbonaten und Carbamidsäureestern und in Abwesenheit oder Gegenwart von Katalysatoren zu cycloaliphatischen Diurethanen umgesetzt werden und man das entstehende Ammoniak gleichzeitig abtrennt;

b) aus der erhaltenen Reaktionsmischung der Alkohol, die Dialkylcarbonate und/oder Carbamidsäurealkylester abgetrennt werden und man den Alkohol sowie optional, auch die Dialkylcarbonate und/oder Carbamidsäurealkylester, in die Reaktionsstufe a) zurückführt;

c) auf eine Abtrennung der in der erhaltenen Reaktionsmischung gegebenenfalls enthaltenen hochsiedenden Rückstände vollständig oder partiell verzichtet wird;

d) die über die Schritte b) und optional c) aufgereinigten Diurethane enthaltende Reaktionsmischung in Gegenwart eines Katalysators kontinuierlich und lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so spaltet, dass ein Teil des Reaktionsgemisches von 10 - 60 Gew.-% bezogen auf den Feed, vorzugsweise 15 - 45 Gew.-% bezogen auf den Feed, ständig ausgeschleust wird;

e) die Spaltprodukte durch Rektifikation in ein rohes cycloaliphatisches Diisocyanat und Alkohol getrennt wird;

f) das rohe cycloaliphatische Diisocyanat, durch Destillation gereinigt und die Reinproduktfraktion isoliert wird;

g) die Sumpfausschleusung aus d) in einen Wertstoff- und einen Abfallstrom aufgetrennt und der an Hochsiederkomponenten reiche Abfallstrom aus dem Prozess ausgeschleust und verworfen wird;

h) der Wertstoffstrom aus g) mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1 - 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;

i) ein Teil der Sumpffraktion der Reindestillation f) kontinuierlich ausschleust und in die Spaltreaktion d) oder in die Urethanisierungsstufe h) geführt wird;

j) optional die bei der Reindestillation f) des rohen cycloaliphatischen Diisocyanats anfallende Kopffraktion ebenfalls in die Urethanisierungsstufe h) zurückgeführt wird;

k) der Reurethanisatstrom aus h) in Stufe b) zurückführt wird;
oder

l) der Reurethanisatstrom aus h) in die Reaktionsstufe a) zurückgeführt wird, unter der Voraussetzung, das Stufe h) in Gegenwart von Katalysatoren, ausgewählt aus den Halogeniden von Fe(III) und/oder Cu(I), durchgeführt wird.

Nach dem erfindungsgemäßen Verfahren können cycloaliphatische Diisocyanaten, im kontinuierlichen Betrieb problemlos mit sehr guten Ausbeuten hergestellt werden. Vorteilhaft bei dem erfindungsgemäßen Mehrstufenverfahren ist insbesondere die Tatsache, dass auch bei Einsatz von cycloaliphatischen Diaminen der Formel (II) als Ausgangsmaterial für die kontinuierliche Diisocyanatsynthese Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Weiterhin ist es ein Vorteil des erfindungsgemäßen Mehrstufenverfahrens, dass es erlaubt, die Menge des in die Dampfphase zu überführenden Diurethans auf ein Minimum zu verringern und auf diese Weise den notwendigen Energieaufwand beschränkt.

Variante IV

Gegenstand der Erfindung in der 3. Stufe ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Kohlensäurederivaten und Alkoholen zu Diurethanen, und deren thermische Spaltung, wobei

a) cycloaliphatische Diamine der Formel (II)

$$H_2N-R-NH_2$$

wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

$$R^1-OH$$

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Katalysatoren zu Cycloalkylenbisharn-stoffen der Formel (IV)

$$H_2N-OC-HN-R-NH-CO-NH_2$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;

b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

$$R^1O-OC-HN-R-NH-CO-OR^1$$

überführt wird;

c) aus der erhaltenen Reaktionsmischung der Alkohol, die Dialkylcarbonate und/oder Carbamidsäurealkylester abgetrennt und der Alkohol in die Reaktionsstufe a) zurückführt wird;

d) auf eine Abtrennung der in der erhaltenen Reaktionsmischung gegebenenfalls enthaltenen hochsiedenden Rückstände vollständig oder partiell verzichtet wird;

e) die über die Schritte c) und d) aufgereinigte Diurethane enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig ausgeschleust wird;

f) die Spaltprodukte durch Rektifikation in ein rohes Diisocyanat und Alkohol getrennt werden;

g) das rohe cycloaliphatische Diisocyanat durch Destillation gereinigt und die Reinproduktfraktion isoliert wird;

h) die Sumpfausschleusung aus e) in einen Wertstoff- und einen Abfallstrom aufgetrennt und der an Hochsiederkomponenten reiche Abfallstrom aus dem Prozess ausgeschleust und verworfen wird;

i) der Wertstoffstrom aus h) mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;

j) ein Teil der Sumpffraktion der Reindestillation g) kontinuierlich ausgeschleust und in die Spaltreaktion e), und/oder in die Urethanisierungsstufe i) geführt wird;

k) optional die bei der Reindestillation des rohen cycloaliphatischen Diisocyanats anfallende Kopffraktion ebenfalls in die Urethanisierungsstufe i) zurückgeführt wird;

l) der Reurethanisatstrom aus i) in Stufe b) und/oder c) zurückgeführt wird.

Nach dem erfindungsgemäßen Verfahren können cycloaliphatische Diisocyanate im kontinuierlichen Betrieb problemlos mit sehr guter Ausbeute hergestellt werden. Vorteilhaft bei dem erfindungsgemäßen Mehrstufenverfahren ist insbesondere die Tatsache, dass auch bei Einsatz von cycloaliphatischen Diaminen der Formel (II) als Ausgangsmaterial für die kontinuierliche Diisocyanatsynthese Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Weiterhin ist es ein Vorteil des erfindungsgemäßen Mehrstufenverfahrens, dass es erlaubt, die Menge des in die Dampfphase zu überführenden Diurethans auf ein Minimum zu verringern und auf diese Weise den notwendigen Energieaufwand beschränkt.

Variante V

Gegenstand der Erfindung in der 3. Stufe ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Kohlensäurederivaten und Alkoholen zu Diurethanen, und deren thermische Spaltung, wobei

a) cycloaliphatische Diamine der Formel (II)

$$H_2N-R-NH_2$$

wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

$$R^1-OH$$

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Katalysatoren zu Cycloalkylenbisharn-stoffen der Formel (IV)

$$H_2N-OC-HN-R-NH-CO-NH_2$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;

b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

$$R^1O-OC-HN-R-NH-CO-OR^1$$

überführt wird;

c) aus der erhaltenen Reaktionsmischung der Alkohol, die Dialkylcarbonate und/oder Carbamidsäurealkylester abgetrennt und der Alkohol in die Reaktionsstufe a) zurückgeführt wird;

d) der Stoffstrom aus Stufe c) destillativ in einen Wertstoffstrom und einen Nebenproduktstrom, der ausgeschleust wird, getrennt wird,

e) die über die Schritte c) und d) aufgereinigte Diurethane enthaltende Reaktionsmischung in Gegenwart eines Katalysators lösemittelfrei bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig ausgeschleust wird;

f) die Spaltprodukte durch Rektifikation in ein rohes Diisocyanat und Alkohol getrennt werden;

g) das rohe cycloaliphatische Diisocyanat durch Destillation gereinigt und die Reinproduktfraktion isoliert wird;

h) die Sumpfausschleusung aus e) mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;

i) optional die Reurethanisierungsreaktion h) in Gegenwart spezieller Katalysatoren, ausgewählt aus den Halogeniden von Fe(III) und/oder Cu(I), durchgeführt wird;

j) ein Teil der Sumpffraktion der Reindestillation g) kontinuierlich ausgeschleust und in die Spaltreaktion e), und/oder in die Urethanisierungsstufe h) geführt wird;

k) optional die bei der Reindestillation des rohen cycloaliphatischen Diisocyanats anfallende Kopffraktion ebenfalls in die Urethanisierungsstufe h) zurückgeführt wird;

l) der Reurethanisatstrom aus h) in Stufe c) zurückgeführt wird.

Nach dem erfindungsgemäßen Verfahren können cycloaliphatische Diisocyanate im kontinuierlichen Betrieb problemlos mit sehr guter Ausbeute hergestellt werden. Durch den Verzicht der Rückführung des in seiner Zusammensetzung variablen Reurethanisatstroms in die Diurethanherstellung resultieren zwei Vorteile für das erfindungsgemäße Mehrstufenverfahren: Zum einen wird der Diurethanreaktor mit einem im Vergleich zum Stand der Technik geringern Massenstrom belastet, so dass durch eine kleinere Auslegung des Reaktors Kosteneinsparungspotentiale gehoben werden können. Zum anderem ist gewährleistet, dass die Diurethansynthese jederzeit unter definierten und im Sinne der Ausbeute optimierten stöchiometrischen Verhältnissen durchgeführt werden kann.

Variante VI

Gegenstand der Erfindung in der 3. Stufe ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Kohlensäurederivaten und Alkoholen zu Diurethanen, und deren thermische Spaltung, wobei

a) cycloaliphatische Diamine der Formel (II)

$H_2N-R-NH_2$

wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

$$R^1\text{-OH}$$

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt,
in Abwesenheit oder Gegenwart von Katalysatoren zu Cycloalkylenbisharnstoffen der Formel (IV)

$$H_2N\text{-OC-HN-R-NH-CO-NH}_2$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;

b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

$$R^1O\text{-OC-HN-R-NH-CO-OR}^1$$

überführt wird;

c) aus der erhaltenen Reaktionsmischung der Alkohol, die Dialkylcarbonate und/oder Carbamidsäurealkylester abgetrennt und der Alkohol in die Reaktionsstufe a) zurückführt wird;

d) auf eine Abtrennung der in der erhaltenen Reaktionsmischung gegebenenfalls enthaltenen hochsiedenden Rückstände vollständig oder partiell verzichtet wird;

e) die über die Schritte c) und d) aufgereinigte Diurethane enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig aus dem Sumpf ausgeschleust wird;

f) die Spaltprodukte durch Rektifikation in ein rohes Diisocyanate und Alkohol getrennt werden;

g) das rohe cycloaliphatische Diisocyanat durch Destillation gereinigt und die Reinproduktfraktion isoliert wird;

h) die Sumpfausschleusung aus e) partiell oder vollständig mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;

i) der Wertstoffstrom aus h) in einen Wertstoff- und einen Abfallstrom aufgetrennt und der an Hochsiederkomponenten reiche Abfallstrom aus dem Prozess ausgeschleust und verworfen wird;

j) ein Teil der Sumpffraktion der Reindestillation g) kontinuierlich ausgeschleust und in die Spaltreaktion e) und/oder in die Urethanisierungsstufe h) geführt wird;

k) optional die bei der Reindestillation des rohen cycloaliphatischen Diisocyanats anfallende Kopffraktion ebenfalls in die Urethanisierungsstufe h) zurückgeführt wird;

l) der aufgereinigte Reurethanisatstrom aus i) in Stufe b) und/oder c) oder e) zurückgeführt wird.

Nach dem erfindungsgemäßen Verfahren, insbesondere nach den Varianten, können cycloaliphatische Diisocyanate im kontinuierlichen Betrieb problemlos mit sehr guter Ausbeute hergestellt werden. Vorteilhaft bei dem erfindungsgemäßen Mehrstufenverfahren ist insbesondere die Tatsache, dass auch bei Einsatz von cycloaliphatischen Diaminen der Formel (II) als Ausgangsmaterial für die kontinuierliche Diisocyanatsynthese Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Weiterhin ist es ein Vorteil des erfindungsgemäßen Mehrstufenverfahrens, dass es erlaubt, die Menge des in die Dampfphase zu überführenden Diurethans auf ein Minimum zu verringern und auf diese Weise den notwendigen Energieaufwand beschränkt.

Ein weiterer Vorteil ist der Verzicht auf Phosgen. Je nach Art des Katalysators, der in der 3. Stufe eingesetzt wird, ist auch diese 3. Stufe Chlor frei, wenn ein Katalysator ohne Chlor verwendet wird. Wird ein Katalysator mit Chlor eingesetzt, arbeitet diese 3. Stufe Chlor arm, was bedeutet, dass der Gehalt an Chlor (berechnet als Chlor-Ion, M = 35,45 g/mol) kleiner als 100 ppm, bevorzugt kleiner 50 ppm, besonders bevorzugt kleiner 20 ppm, beträgt, bezogen auf alle sich in der 3. Stufe anwesenden Mengen an Stoffen.

Zur Herstellung der monomeren cycloaliphatischen Diurethane werden die cycloaliphatischen Diamine der Formel (II) mit Harnstoff und/oder Harnstoffderivaten und einem Alkohol der Formel (III), gegebenenfalls auch Mischungen solcher Alkohole, gegebenenfalls aber nicht vorzugsweise in Gegenwart von Dialkycarbonaten, Carbamidsäurealkylestern oder Mischungen aus Dialkylcarbonaten und Carbamidsäureestern, in Abwesenheit oder Gegenwart von Katalysatoren bei Reaktionstemperaturen von 100 - 270 °C und unter einem Druck von 0,5 - 80 bar, innerhalb von 2 bis 20 Stunden zur Reaktion gebracht. Die Umsetzung kann ein-, zwei- oder auch mehrstufig in einer kontinuierlich betriebenen Rührkesselkaskade oder im Druckdestillationsreaktor erfolgen. Die über Destillationsreaktoren verlaufende Variante ist bevorzugt.

Das freigesetzte Ammoniak wird durch Alkoholbrüden kontinuierlich ausgetrieben, wobei die eingebrachte Alkoholmenge zusammen mit dem gebildeten Ammoniak vorzugsweise am Kopf abgezogen und nach partieller Kondensation in einer Alkoholrückgewinnungskolonne vom restlichen Ammoniak befreit und in den Sumpf zurückgeführt wird.

Zur Erhöhung der Reaktionsgeschwindigkeit können die cycloaliphatischen Diurethane, in Gegenwart von Katalysatoren hergestellt werden. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th Edition, publiziert von Chemical Rubber Publishing Co. 2310 Superior Ave. N.E. Cleveland, Ohio, enthalten, beispielsweise Halogenide wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocaramate. Beispielhaft genannt seinen die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Kobalt und Nickel. Als typische Katalysatoren seinen beispielhaft folgende Verbindungen genannt: Lithiumethanolat, Lithiumbutanolat, Natriummethanolat, Kalium-tert.-butanolat, Magnesiumethanolat, Calciummethanolat, Zinn-(II)-chlorid, Zinn-(IV)-chlorid, Bleiacetat, Aluminiumtrichlorid, Bismuttrichlorid, Kupfer-(II)-acetat, Kupfer-(II)-chlorid, Zinkchlorid, Zinkoctoat, Titantetrabutanolat, Vanadiumtrichlorid, Vanadiumacetylacetonat, Mangan-(II)-acetat, Eisen-(II)-acetat, Eisen-(III)-acetat, Eisenoxalat, Cobaltchlorid, Cobaltnaphthenat, Nickelchlorid, Nickelnaphthenat sowie deren Mischungen. Die Katalysatoren können gegebenenfalls auch in Form ihrer Hydrate oder Ammoniakate zu Einsatz kommen. Es ist bevorzugt, an dieser Stelle auf den Einsatz von Katalysatoren zu verzichten. Falls Chlor haltige Katalysatoren eingesetzt werden, dürfen die oben genannten allgemeinen und bevorzugten Bereiche für den Chlorgehalt nicht überschritten werden.

Ausgangsverbindungen für das erfindungsgemäße Verfahren sind cycloaliphatische Diamine der bereits obengenannten Formel (II), Alkohole der bereits obengenannten Formel (III) sowie Harnstoff und/oder als Carboxylierungsmittel geeignete Harnstoffderivate (Kohlensäurederivate) in Abwesenheit oder Gegenwart von Dialkylcarbonaten, Carbamidsäurealkylestern oder Mischungen aus Dialkylcarbonaten und Carbamidsäurealkylestern. Der Harnstoff kann in konditionierter oder unkonditionierter Form zum Einsatz gelangen. Bevorzugt wird unkonditionierter Harnstoff eingesetzt, wobei verschiedene Darreichungsformen möglich sind, wie z. B. Prills, Granulat, Kristalle, Schmelze, Lösung, Suspension. Bevorzugt wird der Harnstoff als Schmelze eingesetzt.

Prinzipiell sind alle cycloaliphatischen Diamine der Formel (II) erfindungsgemäß geeignet.

Bevorzugte geeignete Diamine der Formel (II) sind solche, wie die in der 2. Stufe (Hydrierung) hergestellten Diamine, bevorzugt 1,4-Diaminocyclohexan, 4,4'-Methylendicyclohexyldiamin, 2,4-Methylendicyclohexyldiamin, 2,2'-Methylendicyclohexyldiamin ($H_{12}$MDA) also perhydriertes Diaminodiphenylmethan (MDA). MDA fällt, wie unter der 1. Stufe beschrieben, als Isomerenmischung aus 4,4'-, 2,4- und 2,2'-MDA an. $H_{12}$MDA wird durch vollständige Hydrierung von MDA gemäß der 2. Stufe erhalten und ist demzufolge eine Mischung aus isomeren Methylendicyclohexyldiaminen ($H_{12}$MDA), nämlich 4,4'-, 2,4- und 2,2'-$H_{12}$MDA. Bevorzugt werden als Diamine der Formel (II) 4,4'-Methylendicyclohexyldiamin, 2,4-Methylendicyclohexyldiamin und 2,2'-Methylendicyclohexyldiamin sowie auch beliebige Mischungen mindestens zweier dieser Isomere eingesetzt.

Es ist besonders bevorzugt, dass man als Diamin der Formel (II) ein $H_{12}$MDA Stoffgemisch mit mindestens 70 Gew.-% an 4,4'- H$_{12}$MDA einsetzt. Ebenfalls besonders bevorzugt ist, dass das eingesetzt H$_{12}$MDA Stoffgemisch 74 - 85 Gew.-% 4,4'- H$_{12}$MDA, 3 - 20 Gew.-% 2,4'- H$_{12}$MDA und weniger als 1 Gew.-% 2,2'- H$_{12}$MDA enthält. Des Weiteren ist besonders bevorzugt, dass ein H$_{12}$MDA Stoffgemisch mit einem trans-trans-Anteil des 4,4'-Isomeren zwischen 10 und 30 %, insbesondere zwischen 15 und 25 %, eingesetzt wird.

Als Alkohole der Formel (III) eignen sich beliebige aliphatische oder cycloaliphatische Alkohole. Beispielhaft genannt seien C1-C6-Alkanole wie z. B. Methanol, Ethanol, 1-Propanol, 1-Butanol, 2-Butanol, 1-Hexanol oder Cyclohexanol. Bevorzugt wird 1-Butanol als Alkohol verwendet.

Im Zuge der Umsetzung des Reaktionsgemisches wird Ammoniak freigesetzt, dessen Entfernung aus dem Reaktionsgleichgewicht sich als vorteilhaft erwiesen hat. Beim Austrag des Ammoniaks aus dem Reaktor ist darauf zu achten, dass die Wandtemperaturen des Reaktors und des Austragsrohres oberhalb von 60 °C liegen, damit eine Belegung durch Ammoniumcarbaminat, welches in minimalen Mengen aus Ammoniak und Kohlendioxid durch Zersetzung von Harnstoff gebildet wird, vermieden werden kann. Es hat sich beispielsweise bewährt, die Umsetzung in einem Druckdestillationsreaktor durchzuführen. Das abgezogene, dampfförmige Gemisch aus Alkohol und Ammoniak kann, vorzugsweise unter dem Druck des Druckdestillationsreaktors und ohne es vorher zu kondensieren, in eine Destillationskolonne geführt werden, um vom Ammoniak freien Alkohol zu gewinnen, der in den Prozess zurückgeführt wird. Um eine Belegung des Rückflusskondensators mit Ammoniumcarbaminat zu verhindern, lässt man in diesem zur Einstellung der Temperatur am Kopf auf mindestens 60 °C einen entsprechenden Anteil an Alkohol zu.

Der überschüssige Alkohol, die Dialkylcarbonate, sofern solche gebildet wurden oder in der Reaktionsmischung vorliegen, oder Carbamidsäurealkylester oder Mischungen aus mindestens zwei dieser Komponenten werden vorteilhafterweise zweistufig abgetrennt. Auf der ersten Stufe wird die Reaktionsmischung auf einen Druck von 1 - 500 mbar, vorzugsweise 2 - 150 mbar, entspannt und auf diese Weise in gasförmige Brüden, die die überwiegende Alkoholmenge sowie gegebenenfalls Dialkylcarbonate und/oder Carbamidsäurealkylester enthalten, und in einen flüssigen Austrag aufgetrennt. Im zweiten Schritt wird der flüssige Austrag durch Dünnschichtverdampfung bei 180 - 250 °C, bevorzugt 200 - 230 °C, und einem Druck von 0,1 - 20 mbar, vorzugsweise 1 - 10 mbar, von gegebenenfalls vorhandenem restlichen Alkohol sowie Mittelsiedern wie Dialkylcarbonaten und/oder Carbamidsäurealkylestern befreit, so dass der Rückstand im Wesentlichen aus dem monomeren Polyurethan, vorzugsweise Diurethan, und gegebenenfalls hochsiedenden Oligomeren besteht.

Anschließend wird wie in den einzelnen Verfahren angegeben weiter aufgearbeitet.

Auf eine Abtrennung der in der Reaktionsmischung gegebenenfalls enthaltenen Hochsieder kann gegebenenfalls gänzlich oder teilweise verzichtet werden.

Optional kann der nach Abtrennung von Leicht- und Mittelsiedern erhaltene flüssige, die monomeren Diurethane und gegebenenfalls hochsiedende Oligomere enthaltende Stoffstrom aus der Diurethanherstellung vorzugsweise mit Hilfe eines Dünnschicht- oder Kurzwegverdampfers, bei einer Temperatur von 180 - 260 °C, vorzugsweise 200 - 240 °C und unter einem Druck von 0,01 - 10 mbar, vorzugsweise 0,02 - 5 mbar destillativ in einen Wertstoffstrom, der die monomeren Diurethane und die leichter siedenden Nebenprodukte enthält, und einen nicht destillierbaren Nebenproduktstrom getrennt werden. Der die hochsiedenden Komponenten enthaltende, nicht destillierbare Nebenproduktstrom wird aus dem Herstellverfahren ausgeschleust und üblicherweise als stofflich nicht verwertbarer Rückstand verworfen.

Optional kann der gegebenenfalls hochsiedende Oligomere enthaltende Stoffstrom vor seiner oben beschriebenen destillativen Aufreinigung auch in zwei Teilströme aufgeteilt werden, von denen einer direkt der Deblockierungsreaktion zugeführt wird und der andere zunächst die oben beschriebene Hochsiederabtrennung durchläuft.

Der die monomeren Diurethane und die leichter siedenden Nebenprodukte enthaltende Wertstoffstrom wird in einer geeigneten Vorrichtung teilweise, lösemittelfrei in flüssiger Phase in Gegenwart von Katalysatoren bei Temperaturen von 180 - 280 °C, vorzugsweise 200 - 260 °C, und unter einem Druck von 0,1 - 200 mbar, vorzugweise 0,2 - 100 mbar kontinuierlich thermisch gespalten. Der Umsatz von Diurethan zu Diisocyanat in der Vorrichtung zur thermischen Spaltung kann in Abhängigkeit vom verwendeten Diurethan weitgehend frei gewählt werden und liegt üblicherweise in einem Bereich von 10 - 95 Gew.-%, vorzugsweise 35 - 85 % der zugeführten Diurethanmenge (Feed). Der ungespaltende Anteil der Reaktionsmischung, der nicht umgesetzte Diurethane, hochsiedende Nebenprodukte und andere wieder verwertbare und unverwertbare Nebenprodukte enthält, wird kontinuierlich ausgeschleust. Die Menge der Ausschleusung richtet sich u. a. nach dem gewünschten Umsatz und der gewünschten Kapazität der Spaltreaktion und kann leicht experimentell ermittelt werden. Sie beträgt üblicherweise 10 - 60 Gew.-%, vorzugsweise 15 - 45 Gew.-%, bezogen auf den Feed.

Als Katalysatoren zur chemischen Spaltung der Diurethane finden z. B. die vorgenannten, die Urethanbildung katalysierenden anorganischen und organischen Verbindungen Verwendung. Vorzugsweise werden Chloride des Zinks oder Zinns sowie Zink-, Mangan-, Eisen-, oder Cobaltoxide eingesetzt, wobei der Katalysator als 0,01 -25 Gew.-%ige, vorzugsweise 0,05 - 10 Gew.-%ige Lösung oder Suspension vorzugsweise in dem Alkohol, der auch zur Urethanherstellung verwendet wird, in einer Menge von 5 - 200 ppm, vorzugsweise 10 - 100 ppm, zudosiert wird. Dabei beträgt die Menge an Chlor (berechnet als Chlor-Ion, M = 35,45 g/mol) weniger als 100 ppm, bevorzugt weniger als 50 ppm, bezogen auf alle sich in der Spaltung anwesenden Mengen an Stoffen. Besonders bevorzugt wird die Spaltung mit einem Chlorgehalt, resultierend aus dem eingesetzten Katalysator, von kleiner als 20 ppm (wie definiert) durchgeführt.

Als Spaltvorrichtungen eigenen sich beispielsweise zylinderförmige Spaltreaktoren, wie z. B. Röhrenöfen oder vorzugsweise Verdampfer, beispielsweise Fallfilm-, Dünnschicht- oder Bulkverdampfer, wie z. B. Robertverdampfer, Herbertverdampfer, caddle-typ-Verdampfer, Oskarverdampfer und Heizkerzenverdampfer.

Prinzipiell geht es darum, die mittlere Verweilzeit der Isocyanatgruppen, die bei Deblockierung des Alkohols zwangsläufig freigesetzt werden, in der Spaltzone möglichst gering zu halten und so unerwünschte Nebenreaktionen auf ein Minimum zu beschränken.

Bevorzugt wird die Spaltung in einer kombinierten Spalt- und Rektifizierkolonne durchgeführt, die für die Energiezufuhr im Sumpf mit einem Fallfilmverdampfer, im unteren Drittel mit einer Einrichtung zum zusätzlichen Energieeintrag bzw. zur Energierückgewinnung, im oberen Drittel mit einer Einrichtung zum Abzug von Roh-Diisocyanat und am Kopf mit einem Kondensator für den Rückfluss und den Abzug von reinem Alkohol ausgestattet ist.

Die bei der thermischen Spaltung gebildeten Spaltprodukte, die sich vor allem aus Alkohol, Diisocyanat und partiell gespaltenen Diurethanen zusammensetzen, werden durch Rektifikation bei Temperaturen von 95 - 260 °C und einem Druck von 0,5 - 250 mbar in Alkohol und in eine rohe Diisocyanatmischung - bevorzugt bestehend aus cycloaliphatischem Diisocyanat, partiell gespaltenem cycloaliphatischem Diisocyanat und gegebenenfalls geringen Anteilen an cycloaliphatischen Diurethan - getrennt. Diese Trennung kann beispielsweise in der Spaltkolonne der obengenannten kombinierten Spalt- und Rektifizierkolonne durchgeführt werden.

Die vorzugsweise durch Rektifikation erhaltene rohe Mischung, bestehend aus cycloaliphatischem Diisocyanat, partiell gespaltenem cycloaliphatischem Diurethan und gegebenenfalls geringen Anteilen an cycloaliphatischen Diurethan, wird durch Destillation bei einer Temperatur von 95 - 260 °C und unter einem Druck von 0,5 - 150 mbar gereinigt.

Die während der Spaltung oder nach der Spaltung ausgeschleusten oder durch Aufarbeitung erhaltenen Produktströme können wie oben beschrieben (zum Beispiel Variante I g) und k); Variante III h) und l); Variante IV i) und l)) reurethanisiert werden.

Dabei wird die entsprechende Reaktionsmischung mit Alkohol in Gegenwart oder Abwesenheit von Katalysatoren innerhalb von 1 -150 min, vorzugsweise 3 - 60 min, bei Temperaturen von 20 - 200 °C, vorzugsweise 50 -170 °C und bei einem Druck von 0,5 - 20 bar, vorzugsweise 1 - 15 bar, umgesetzt. Die Umsetzung kann in einer kontinuierlichen Kesselkaskade oder in einem Rohrreaktor durchgeführt werden. Als Katalysatoren kommen grundsätzlich alle Kontakte in Frage, die die NCO/OH-Reaktion fördern. Beispielhaft seien Zinnoctoat, Dibutylzinnlaurat, Zinndichlorid, Zinkdichlorid und Triethylamin aufgeführt. Die Reurethanisierung kann auch in Gegenwart von Fe(III)- oder Cu(I)-Halogeniden oder Mischungen davon durchgeführt werden. Beispielhaft seinen Fe(III)-chlorid, Fe(III)-bromid, Cu(I)-chlorid und Cu(I)-bromid aufgeführt. Der Einsatz dieser Katalysatoren schließt die gleichzeitige Verwendung anderer Katalysatoren, die der Beschleunigung der Urethanisierung dienen, nicht grundsätzlich aus. Bevorzugt werden die Halogenide von Fe(III) oder Cu(I) oder Mischungen davon ohne zusätzliche Verwendung weiterer Kontakte eingesetzt. Dabei beträgt die Menge an Chlor (berechnet als Chlor-Ion, M = 35,45 g/mol) weniger als 100 ppm, bevorzugt weniger als 50 ppm, bezogen auf alle in der Reaktionsmischung (Reurethanisierung) anwesenden Mengen an Stoffen.

Anschließend wird wie in den einzelnen Verfahren beschrieben, weiter verfahren. Weitere technische Angaben zu den einzelnen Verfahren kann der Fachmann aus EP 1 512 680, EP 1 512 681, EP 1 512 682, EP 1 593 669, EP 1 602 643, EP 1 634 868 entnehmen.

Mit dem erfindungsgemäßen mehrstufigen Verfahren zur kontinuierlichen Herstellung von cycloaliphatischen Diisocyanaten unter Rückführung und Ausschleusung der Nebenprodukte kann für destillierbare cycloaliphatische Diisocyanate über einen langen Zeitraum eine mit hoher Selektivität störungsfrei ablaufende Reaktion gewährleistet werden. Das erfindungsgemäße Verfahren eignet sich insbesondere zur Herstellung von cycloaliphatischen Diisocyanaten mit 4 bis 18, vorzugsweise 5 bis 15 Kohlenstoffatomen wie 1,4-Diisocyantocyclohexan, 4,4'-Methylendicyclohexyldiisocyanat (4,4'-$H_{12}$MDI), 2,2'-Methylendicyclohexyldiisocyanat (2,2'-$H_{12}$MDI), 2,4-Methylendicyclohexyldiisocyanat (2,4-$H_{12}$MDI) oder auch Mischungen der vorgenannten isomeren Methylendicyclohexyldiiso-cyanate, wie sie zum Beispiel naturgemäß bei der Umwandlung von $H_{12}$MDA (perhydriertem MDA) in $H_{12}$MDI anfallen.

Besonders bevorzugt wird, ausgehend von dem in der ersten Stufe bevorzugt erhaltenen Isomerengemisch aus 4,4'-MDA, 2,4'-MDA und 2,2'-MDA und anschließender Hydrierung zu den entsprechenden Isomerengemischen des $H_{12}$MDA, das daraus resultierende Isomerengemisch an 4,4'-$H_{12}$MDI mit entsprechendem trans,trans-Gehalt, 2,4'-$H_{12}$MDI und 2,2'-$H_{12}$MDI hergestellt.

Die hergestellten cycloaliphatischen Diisocyanate, eigenen sich bestens zur Herstellung von Urethan-, Isocyanurat-, Amid- und/oder Harnstoffgruppen enthaltenden Kunststoffen nach dem Polyisocyanat-Polyadditionsverfahren. Sie finden darüber hinaus Verwendung zur Herstellung von mit Urethan-, Biuret-, und/oder Isocyanuratgruppen modifizierten Polyisocyanatmischungen. Derartige Polyisocyanatmischungen aus cycloaliphatischen Diisocyanaten werden insbesondere zur Herstellung von hochwertigen, lichtbeständigen Polyurethanbeschichtungen eingesetzt.

Das erfindungsgemäße Verfahren wird unten exemplarisch beschrieben.

Beispiele

1. Stufe: Herstellung von Diaminodiphenylmethan (MDA)

**Beispiele 1 und 2**

232,5 g Anilin und 60 g des feuchten, sulfongruppenhaltigen Ionenaustauscherharzes Amberlyst 36Wet wurden in einem Rührbehälter unter N2 Atmosphäre zusammengegeben und unter Rühren auf 80 bzw. 100 °C, erwärmt. Bei diesen Temperaturen wurden 40 g Formaldehydlösung (37 Gew.-% Formaldehyd in Wasser), entsprechend einem Molverhältnis von Anilin / Formalin von 5 : 1, innerhalb von 60 Minuten zudosiert.
Das eingesetzte Ionenaustauscherharz weist laut Produktdatenblatt des Herstellers eine Konzentration der aciden Zentren von mindestens 5,4 eq/kg und einen durchschnittlichen Porendurchmesser von 240 Å (entspricht 24 nm) auf.
Die Zusammensetzung des Reaktionsproduktes MDA (Gaschromatographie-Analyse nach Abzug des im Überschuss vorliegenden Anilins) nach 60 Minuten stellt sich wie folgt dar:

| Nr. | Temp. °C | 2,2'-Isomer Gew.-% | 2,4'-Isomer Gew.-% | 4,4'-Isomer Gew.-% | N-Methyl- Verbindung Gew.-% | Mehrkernverbindung Gew.-% |
|---|---|---|---|---|---|---|
| 1 | 80 | 0,159 | 9,378 | 79,917 | 0,102 | 10,443 |
| 2 | 100 | 0,198 | 10,986 | 76,829 | 0,150 | 11,837 |

Die Ausbeute ist hoch und beträgt 89,5 % im Beispiel 1 und 88 % im Beispiel 2.

**Beispiele 3 bis 6**

232,5 g Anilin und 60 g des feuchten, sulfongruppenhaltigen Ionenaustauscherharzes Amberlyst 36Wet wurden in einem Rührbehälter unter N2 Atmosphäre zusammengegeben und unter Rühren auf 80 bzw.120 °C erwärmt. Bei dieser Temperatur wurden 20 g Formaldehydlösung (37 Gew.-% Formaldehyd in Wasser), entsprechend einem Molverhältnis von Anilin / Formalin von 10 : 1, innerhalb von 60 Minuten zudosiert.
Das eingesetzte Ionenaustauscherharz weist laut Produktdatenblatt des Herstellers eine Konzentration der aciden Zentren von mindestens 5,4 eq/kg und einen durchschnittlichen Porendurchmesser von 240 Å (entspricht 24 nm) auf.
Die Zusammensetzung des Reaktionsproduktes MDA (Gaschromatographie-Analyse nach Abzug des im Überschuss vorliegenden Anilins) stellt sich wie folgt dar:

| Nr. | Temp. °C | 2,2'-Isomer Gew.-% | 2,4'-Isomer Gew.-% | 4,4'-Isomer Gew.-% | N-Methyl- Verbindung Gew.-% | Mehrkernverbindung Gew.-% |
|---|---|---|---|---|---|---|
| 3 | 80 | 0,362 | 10,905 | 79,996 | 0,025 | 8,711 |
| 4 | 100 | 0,380 | 12,593 | 77,572 | 0,128 | 9,362 |
| 5 | 110 | 0,525 | 13,835 | 75,933 | 0,137 | 9,569 |
| 6 | 120 | 0,748 | 15,224 | 74,224 | 0,153 | 9,846 |

Auch diese Beispiele zeigen, dass das geforderte Isomerenverhältnis ohne weitere Aufreinigungs- oder Destillationsschritte erzeugt werden kann. Die für den vollständigen Formaldehyd Umsatz benötigten Reaktionszeiten betrugen 240 Minuten für Beispiel 3, 140 Minuten für Beispiel 4 und 60 Minuten für die Beispiele 5 und 6. Die Selektivitäten (Ausbeuten) sind hoch und betragen 91,3 % im Beispiel 3, 90,5 % im Beispiel 4, 90,3 % im Beispiel 5 und 90,2 % im Beispiel 6.

**Beispiel 7**

232,5 g Anilin und 60 g des feuchten, sulfongruppenhaltigen Ionenaustauscherharzes Amberlyst 15Wet wurden in einem Rührbehälter unter $N_2$ Atmosphäre zusammengegeben und unter Rühren auf 100°C erwärmt. Das eingesetzte Ionenaustauscherharz weist laut Produktdatenblatt des Herstellers eine Konzentration der aciden Zentren von mindestens 4,7 eq/kg und einen durchschnittlichen Porendurchmesser von 300 Å (entspricht 30 nm) auf. Bei dieser Temperatur wurden 20 g Formaldehydlösung (37 Gew.-% Formaldehyd in Wasser), entsprechend einem Molverhältnis von Anilin / Formalin von 10 : 1, innerhalb von 60 Minuten zudosiert.

Die Zusammensetzung des Reaktionsproduktes (Gaschromatographie-Analyse nach Abzug des im Überschuss vorliegenden Anilins) stellt sich wie folgt dar:

| Nr. | Temp. °C | 2,2'-Isomer Gew.-% | 2,4'-Isomere Gew.-% | 4,4'-Isomer Gew.-% | N-Methyl-Verbindung Gew.-% | Mehrkernverbindung Gew.-% |
|---|---|---|---|---|---|---|
| 7 | 100 | 0,14 | 12,82 | 79,32 | 0,37 | 7,28 |

Das Beispiel zeigt, dass das geforderte Isomerenverhältnis ohne weitere Aufreinigungs- oder Destillationsschritte erzeugt werden kann. Die für den vollständigen Formaldehyd Umsatz benötigten Reaktionszeiten betrug 60 Minuten für Beispiel. Die Selektivität (Ausbeuten) ist hoch und beträgt 90,3 %.

**Beispiel 8**

93,1 g Anilin und 10 g des feuchten, sulfongruppenhaltigen Ionenaustauscherharzes Amberlyst 35Wet, wurden in einem Rührbehälter unter $N_2$ Atmosphäre zusammengegeben und unter Rühren auf 120 °C erwärmt. Bei dieser Temperatur wurden 8,1 g Formaldehydlösung (37 Gew.-% Formaldehyd in Wasser), entsprechend einem Molverhältnis von Anilin / Formalin von 10:1, innerhalb von 60 Minuten zudosiert. Das eingesetzte Ionenaustauscherharz weist laut Produktdatenblatt des Herstellers eine Konzentration der aciden Zentren von mindestens 5,2 eq/kg und einen durchschnittlichen Porendurchmesser von 300 Å (entspricht 30 nm) auf.

Die Zusammensetzung des Reaktionsproduktes (Gaschromatographie-Analyse nach Abzug des im Überschuss vorliegenden Anilins) stellt sich wie folgt dar: 0,9 % 2,2 MDA; 16,8 % 2,4 MDA; 70,6 % 4,4 MDA; 0,06 % N-Methyl- MDA und 11,7 % Mehrkernverbindungen. Die Ausbeute betrug 88,2 %.

**Beispiel 9**

93,1 g Anilin und 10 g des feuchten, sulfongruppenhaltigen Ionenaustauscherharzes Amberlyst 39Wet, wurden in einem Rührbehälter unter N2 Atmosphäre zusammengegeben und unter Rühren auf 120 °C erwärmt. Bei dieser Temperatur wurden 8,1 g Formaldehydlösung (37 Gew.-% Formaldehyd in Wasser), entsprechend einem Molverhältnis von Anilin / Formalin von 10 : 1, innerhalb von 60 Minuten zudosiert. Das eingesetzte Ionenaustauscherharz weist laut Produktdatenblatt des Herstellers eine Konzentration der aciden Zentren von mindestens 5,0 eq/kg und einen durchschnittlichen Porendurchmesser von 230 Å (entspricht 23 nm) auf.

Die Zusammensetzung des Reaktionsproduktes (Gaschromatographie-Analyse nach Abzug des im Überschuss vorliegenden Anilins) stellt sich wie folgt dar: 1,5 % 2,2 MDA; 17,9 % 2,4 MDA; 66,8 % 4,4 MDA; 0,06 % N-Methyl- MDA und 13,8 % Mehrkernverbindungen. Die Ausbeute bertrug 86,1 %.

**Beispiel 10**

93,1 g Anilin und 10 g des feuchten, sulfongruppenhaltigen Ionenaustauscherharzes Amberlyst 70Wet wurden in einem Rührbehälter unter N2 Atmosphäre zusammengegeben und unter Rühren auf 120 °C erwärmt. Bei dieser Temperatur wurden 8,1 g Formaldehydlösung (37 Gew.-% Formaldehyd in Wasser), entsprechend einem Molverhältnis von Anilin / Formalin von 10 : 1, innerhalb von 60 Minuten zudosiert. Das eingesetzte Ionenaustauscherharz weist laut Produktdatenblatt des Herstellers eine Konzentration der aciden Zentren von mindestens 2,55 eq/kg und einen durchschnittlichen Porendurchmesser von 220 Å (entspricht 22 nm) auf.

Die Zusammensetzung des Reaktionsproduktes (Gaschromatographie-Analyse nach Abzug des im Überschuss vorliegenden Anilins) stellt sich wie folgt dar: 1,1 % 2,2 MDA; 15,9 % 2,4 MDA; 69,4 % 4,4 MDA; 0,03 % N-Methyl- MDA und 13,6% Mehrkernverbindungen. Die Ausbeute betrug 86,3 %.

Die Beispiele zeigen, dass das geforderte Isomerenverhältnis ohne weitere Aufreinigungs- oder Destillationsschritte erzeugt werden kann. Die Selektivität (S) der Reaktion errechnet sich aus dem Verhältnis zwischen der gebildeten Stoffmenge des gewünschten Produktes (P) (hier die Summe der 2,2', 2,4'und 4,4'-Isomere des MDA) und der umgesetzten Stoffmenge der Schlüsselkomponente (K) (hier Formaldehyd) unter Berücksichtigung der stöchiometrischen Zahlen (v). Für den Satzbetrieb gilt daher:

$$S_P = \frac{n^0}{n_K^0 - n_K} \cdot \frac{|v_K|}{v_P} \cdot 100$$

Nach den angegebenen Minuten wurde der vollständige Umsatz des eingesetzten Formaldehyds erreicht. Daher ist die Selektivität der Reaktion mit der Ausbeute identisch.

2. Stufe: Hydrierung von MDA

Beispiel 1

Die Hydrierung von MDA erfolgte in einem 2,5-L-Autoklaven, der mit einem Rührwerk sowie einem Korb zur Testung von Festbettkatalysatoren ausgestattet war. Der Korb wurde mit 100 ml eines Rutheniumkatalysators gefüllt, der entsprechend Beispiel 3 der EP 1366812 erhalten wurde. Mit diesem Katalysator wurden 1000 g einer Lösung von 12,5 Gew-% MDA in THF bei 120 °C und 80 bar für 6 h hydriert. Das Endprodukt enthielt 90,5 % $H_{12}MDA$ mit einem Anteil des trans-trans-4,4'-$H_{12}MDA$ von 19,5 %, 2 % niedriger und 7,5 % höher als $H_{12}MDA$ siedende Komponenten. Das MDA enthielt 78,6 % 4,4'-MDA, 11,5 % 2,4'-MDA und 0,7 % 2,2'-MDA.

3. Stufe: Herstellung der Diisocyanate

Beispiel 1: Erfindungsgemässe Herstellung von Methylendicyclohexyldiisocyanat ($H_{12}MDI$) aus perhydriertem Methylendiphenyldiamin ($H_{12}MDA$) und Harnstoff in Gegenwart von n-Butanol - Rückführung des Diurethanisats in die Flash-Stufe.

Der oberste Boden eines Druckdestillationsreaktors wurden stündlich mit 255,3 g $H_{12}MDA$, 149,3 g Harnstoff und 545 g n-Butanol beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 605,9 g/h Bis-(4-butoxycarbonylamino-cyclohexyl)-methan ($H_{12}MDU$) wurden als Schmelze (140 °C) in die Wälzung des Fallfilmverdampfers der Spalt- und Rektifizierkolonne gefahren, wobei die Deblockierungsreaktion bei einer Temperatur von 236 °C und einem Sumpfdruck von 10 mbar in Gegenwart einer stationären Konzentration an Zinndichlorid von 16 ppm durchgeführt wurde. Die Spaltgase $H_{12}MDI$ und Butanol wurden in zwei hintereinandergeschalteten Kondensatoren bei 85 °C und -25 °C auskondensiert. Das gewonnene, etwa 97%ige Roh- $H_{12}MDI$ wurde einer Reindestillation zugeführt, wobei 270,33 g/h $H_{12}MDI$ mit einer Reinheit von > 99,5 % erhalten wurden, was einer Selektivität von 85 % entspricht. 177,2 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 147,6 g/h aus dem Wälzkreislauf ausgeschleust und mit 24,0 g/h Sumpfauskreisung aus der $H_{12}MDI$-Reindestillation sowie dem Kopfprodukt aus der Spalt- und Rektifizierkolonne vereinigt und reurethanisiert. Das Reurethanisat wurde zusammen mit dem Reaktoraustrag der Diurethanherstellung dem Flash-Behälter zugeführt.

Beispiel 2: Erfindungsgemäße Herstellung von Methylendicyclohexyldiisocyanat ($H_{12}MDI$) aus perhydriertem Methylendiphenyldiamin ($H_{12}MDA$) und Harnstoff in Gegenwart von n-Butanol - Reurethanisierung in Gegenwart von CuCl und Rückführung des Reurethanisats in die Diurethansynthese.

Der oberste Boden eines Druckdestillationsreaktors wurden stündlich mit 255,3 g $H_{12}MDA$, 149,3 g Harnstoff und 545 g n-Butanol sowie dem Stoffstrom aus der katalytischen Reurethanisierung beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 601,1 g/h Bis-(4-butoxycarbonylamino-cyclohexyl)-methan ($H_{12}MDU$) wurden als Schmelze (140 °C) in die Wälzung des Fallfilmverdampfers der Spalt- und Rektifizierkolonne gefahren, wobei die Deblockierungsreaktion bei einer Temperatur von 236 °C und einem Sumpfdruck von 10 mbar in Gegenwart einer stationären Konzentration an Zinndichlorid von 16 ppm durchgeführt wurde. Die Spaltgase $H_{12}$MDI und Butanol wurden in zwei hintereinandergeschalteten Kondensatoren bei 85 °C und -25 °C auskondensiert. Das gewonnene, etwa 97%ige Roh-$H_{12}$MDI wurde einer Reindestillation zugeführt, wobei 268,2 g/h $H_{12}$MDI mit einer Reinheit von > 99,5 % erhalten wurden, was einer Selektivität von 84 % entspricht. 175,9 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 145,6 g/h aus dem Wälzkreislauf ausgeschleust und mit 23,9 g/h Sumpfauskreisung aus der $H_{12}$MDI-Reindestillation sowie dem Kopfprodukt aus der Spalt- und Rektifizier-kolonne vereinigt und in Gegenwart von 100 ppm CuCl reurethanisiert. Das Reurethanisat wurde der Diurethanherstellung im Druckdestillationsreaktor zugeführt.

Beispiel 3: Erfindungsgemäße Herstellung von Methylendicyclohexyldüsocyanat ($H_{12}$MDI) aus perhydriertem Methylendiphenyldiamin ($H_{12}$MDA) und Harnstoff in Gegenwart von n-Butanol - Rückführung des Diurethanisats in die Flash-Stufe.

Der oberste Boden eines Druckdestillationsreaktors wurden stündlich mit 280,8 g $H_{12}$MDA, 164,0 g Harnstoff und 599,6 g n-Butanol beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und die verbleibenden 779,0 g/h Bis-(4-butoxycarbonylamino-cyclohexyl)-methan ($H_{12}$MDU) als Schmelze (140 °C) in die Wälzung des Fallfilm-verdampfers der Spalt- und Rektifizierkolonne gefahren, wobei die Deblockierungsreaktion bei einer Temperatur von 236 °C und einem Sumpfdruck von 9 mbar in Gegenwart einer stationären Konzentration an Zinndichlorid von 15 ppm durchgeführt wurde. Die Spaltgase $H_{12}$MDI und Butanol wurden in zwei hintereinandergeschalteten Kondensatoren bei 85 °C und -25 °C auskondensiert. Das gewonnene, etwa 97%ige Roh- $H_{12}$MDI wurde einer Reindestillation zugeführt, wobei 319,52 g/h $H_{12}$MDI mit einer Reinheit von > 99,5 % erhalten wurden, was einer Ausbeute von 91 % entspricht. 227,5 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurde kontinuierlich ein Teilstrom aus dem Wälzkreislauf ausgeschleust und mittels eines Kurzwegverdampfers bei 235 °C und einem Druck von 0,04 mbar in einen Hochsieder reichen Abfallstrom und einen Wertstoffstrom aufgetrennt. Die 181,3 g/h Wertstoffstrom wurde zusammen mit 24,3 g/h Sumpfauskreisung aus der $H_{12}$MDI-Reindestillation sowie dem Kopfprodukt aus der Spalt- und Rektifizierkolonne vereinigt und reurethanisiert. Das Reurethanisat wurde zusammen mit dem Reaktoraustrag der Diurethanherstellung dem Flash-Behälter zugeführt.

Beispiel 4: Erfindungsgemäße Herstellung von Methylendicyclohexyldüsocyanat ($H_{12}$MDI) aus perhydriertem Methylendiphenyldiamin ($H_{12}$MDA) und Harnstoff in Gegenwart von n-Butanol - Reurethanisierung in Gegenwart von CuCl und Rückführung des Reurethanisats in die Diurethansynthese.

Der oberste Boden eines Druckdestillationsreaktors wurden stündlich mit 282,1 g $H_{12}$MDA, 164,5 g Harnstoff und 600,8 g n-Butanol sowie dem Stoffstrom aus der katalytischen Reurethanisierung beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit. Die verbleibenden 778,1 g/h Bis-(4-butoxycarbonylaminocyclohexyl)-methan ($H_{12}$MDU) wurden als Schmelze (140 °C) in die Wälzung des Fallfilmverdampfers der Spalt- und Rektifizierkolonne gefahren, wobei die Deblockierungsreaktion bei einer Temperatur von 237 °C und einem Sumpfdruck von 9 mbar in Gegenwart einer stationären Konzentration an Zinndichlorid von 17 ppm durchgeführt wurde. Die Spaltgase $H_{12}$MDI und Butanol wurden in zwei hintereinandergeschalteten Kondensatoren bei 85 °C und -25 °C auskondensiert. Das gewonnene, etwa 97%ige Roh- $H_{12}$MDI wurde einer Reindestillation zugeführt, wobei 318,17 g/h $H_{12}$MDI mit einer Reinheit von > 99,5 % erhalten wurden, was einer Ausbeute von 90 % entspricht. 228,9 g/h Butanol fielen als Kopfprodukt der Spalt- und Rektifizierkolonne an. Zur Aufrechterhaltung der Massenkonstanz innerhalb der Spalt- und Rektifizier-kolonne und Vermeidung von Belegungen und Verstopfungen der Spaltapparatur wurde kontinuierlich ein Teilstrom aus dem Wälzkreislauf ausgeschleust und mittels eines Kurzwegverdampfers bei 235 °C und einem Druck von 0,04 mbar in einen Hochsieder reichen Abfallstrom und einen Wertstoffstrom aufgetrennt. Die 175,6 g/h Wertstoffstrom wurde zusammen mit 24,7 g/h Sumpfauskreisung aus der $H_{12}$MDI-Reindestillation sowie dem Kopfprodukt aus der Spalt- und Rektifizierkolonne vereinigt und in Gegenwart von 100 ppm CuCl reurethanisiert. Das Reurethanisat wurde der Diurethanherstellung im Druckdestillationsreaktor zugeführt.

Beispiel 5: Erfindungsgemäße Herstellung von Methylendicyclohexyldiisocyanat ($H_{12}$MDI) aus perhydriertem Methylendiphenyldiamin und Harnstoff in Gegenwart von n-Butanol - Reurethanisierung in Gegenwart von CuCl und Rückführung des Reurethanisats in die Leicht- und Mittelsiederabtrennung.

Der oberste Boden eines Destillationsreaktors wurden stündlich mit 275,1 g $H_{12}$MDA, 162,9 g Harnstoff und 590,1 g n-Butanol beaufschlagt und die Reaktionsmischung unter kontinuierlicher Entfernung des freigesetzten Ammoniaks bei Normaldruck, 135 °C und einer mittleren Verweilzeit von 8 Stunden gekocht. Die im Sumpf des Destillationsreaktors anfallende Lösung von Bisharnstoff in Butanol wurde über einen Wärmetauscher auf 190 °C vorgeheizt, auf den obersten Boden eines Druckdestillationsreaktors geführt und bei 11 bis 14 bar, 220 °C und mit einer mittleren Verweilzeit von 10,5 h weiter umgesetzt. Im Sumpf des Druckdestillationsreaktors wurden stündlich 536 g n-Butanol eingespeist und die zusammen mit dem freigesetzten Ammoniak am Kopf abgezogene Alkoholmenge so gewählt, dass sie dem Alkoholeintrag im Sumpf entsprach. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und die verbleibenden 763,2 g/h Bis-(4-butoxycarbonyl-amino-cyclohexyl)-methan ($H_{12}$MDU) als Schmelze (140 °C) in die Wälzung des Fallfilmverdampfers der Spalt- und Rektifizierkolonne gefahren, wobei die Deblockierungsreaktion bei einer Temperatur von 235 °C und einem Sumpfdruck von 9 mbar in Gegenwart einer stationären Konzentration an Zinndichlorid von 16 ppm durchgeführt wurde. Die Spaltgase $H_{12}$MDI und Butanol wurden in zwei hintereinandergeschalteten Kondensatoren bei 85 °C und -25 °C auskondensiert. Das gewonnene, etwa 97%ige Roh- $H_{12}$MDI wurde einer Reindestillation zugeführt, wobei 309,1 g/h $H_{12}$MDI mit einer Reinheit von > 99,5 % erhalten wurden, was einer auf das Amin bezogenen Ausbeute von 90 % entspricht. 226,4 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur kontinuierlich ein Teilstrom aus dem Wälzkreislauf ausgeschleust, dieser im Verhältnis 80 : 20 aufgeteilt und die größere Menge mittels eines Kurzwegverdampfers bei 235 °C und einem Druck von 0,05 mbar in einen Hochsieder reichen Abfallstrom und einen Wertstoffstrom aufgetrennt. Die 129,45 g/h Wertstoffstrom wurden mit 22,7 g/h Sumpfauskreisung aus der $H_{12}$MDI-Reindestillation sowie dem Kopfprodukt aus der Spalt- und Rektifizierkolonne und dem nicht aufgereinigten Teilstrom aus der Ausschleusung vereinigt und in Gegenwart von 100 ppm CuCl reurethanisiert. Das Reurethanisat wurde zusammen mit dem Reaktoraustrag der Diurethanherstellung dem Flash-Behälter zugeführt.

Vergleichsbeispiel 1: Herstellung von Methylendicyclohexyldiisocyanat ($H_{12}$MDI) aus perhydriertem Methylendiphenyldiamin ($H_{12}$MDA) und Harnstoff in Gegenwart von n-Butanol - Reurethanisierung und Rückführung des Reurethanisats in die Diurethansynthese.

Der oberste Boden eines Druckdestillationsreaktors wurden stündlich mit 255,3 g $H_{12}$MDA, 149,3 g Harnstoff und 545 g n-Butanol sowie dem Stoffstrom aus der Reurethanisierung beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 575,1 g/h Bis-(4-butoxycarbonylamino-cyclohexyl)-methan ($H_{12}$MDU) wurden als Schmelze (140 °C) in die Wälzung des Fallfilmverdampfers der Spalt- und Rektifizierkolonne gefahren, wobei die Deblockierungsreaktion bei einer Temperatur von 236 °C und einem Sumpfdruck von 10 mbar in Gegenwart einer stationären Konzentration an Zinndichlorid von 16 ppm durchgeführt wurde. Die Spaltgase $H_{12}$MDI und Butanol wurden in zwei hintereinandergeschalteten Kondensatoren bei 85 °C und -25 °C auskondensiert. Das gewonnene, etwa 97%ige Roh- $H_{12}$MDI wurde einer Reindestillation zugeführt, wobei 252,9 g/h $H_{12}$MDI mit einer Reinheit von > 99,5 % erhalten wurden, was einer Selektivität von 79 % entspricht. 163,5 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 139,9 g/h aus dem Wälzkreislauf ausgeschleust und mit 22,6 g/h Sumpfauskreisung aus der $H_{12}$MDI-Reindestillation sowie dem Kopfprodukt aus der Spalt- und Rektifizierkolonne vereinigt und reurethanisiert. Das Reurethanisat wurde der Diurethanherstellung im Druckdestillations-reaktor zugeführt.

Die Anfangsselektivität des Kreislaufversuchs lag bei ca. 84 %. Sie nahm im Verlauf des Experiments (12 h) jedoch kontinuierlich ab und fiel am Ende unter 75 %.

Vergleichsbeispiel 2: Herstellung von Methylendicyclohexyldiisocyanat ($H_{12}$MDI) aus perhydriertem Methylendiphenyldiamin ($H_{12}$MDA) und Harnstoff in Gegenwart von n-Butanol - direkte Rückführung der Spaltausschleusung in die Diurethansynthese.

Der oberste Boden eines Druckdestillationsreaktors wurden stündlich mit 255,3 g $H_{12}$MDA, 149,3 g Harnstoff und 545 g n-Butanol sowie dem Stoffstrom aus der Spaltreaktorausschleusung und dem Kopfprodukt der Spalt- und Rektifizierkolonne (Butanol) beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 571,8 g/h Bis-(4-butoxycarbonylamino-cyclohexyl)-methan (H$_{12}$MDU) wurden als Schmelze (140 °C) in die Wälzung des Fallfilmverdampfers der Spalt- und Rektifizierkolonne gefahren, wobei die Deblockierungsreaktion bei einer Temperatur von 236 °C und einem Sumpfdruck von 10 mbar in Gegenwart einer stationären Konzentration an Zinndichlorid von 16 ppm durchgeführt wurde. Die Spaltgase H$_{12}$MDI und Butanol wurden in zwei hintereinandergeschalteten Kondensatoren bei 85 °C und -25 °C auskondensiert. Das gewonnene, etwa 97%ige Roh-H$_{12}$MDI wurde einer Reindestillation zugeführt, wobei 249,8 g/h H$_{12}$MDI mit einer Reinheit von > 99,5 % erhalten wurden, was einer Selektivität von 78 % entspricht. 160,6 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 137,5 g/h aus dem Wälzkreislauf ausgeschleust und ohne Reurethanisierung der Diurethanherstellung im Druckdestillationsreaktor zugeführt. 22,8 g/h Sumpfauskreisung aus der H$_{12}$MDI-Reindestillation wurden in die Wälzung der Spaltappararrtur zurückgeführt.

Die Anfangsselektivität des Kreislaufversuchs lag bei ca. 83 %. Sie nahm im Verlauf des Experiments (12 h) jedoch kontinuierlich ab und fiel am Ende unter 75 %.

## Patentansprüche

1. Chlor armes mehrstufiges und kontinuierliches Verfahren zur Herstellung von cycloaliphatischen Diisocyanaten durch

   1. Chlor freie Herstellung von aromatischen Diaminen, wobei ein aromatisches Amin, das substituiert oder unsubstituiert sein kann, mit einem C$_1$-C$_3$ Aldehyd in Gegenwart eines heterogenen Katalysators umgesetzt wird, wobei der Katalysator ein mesoporöser saurer Ionenaustauscher auf Basis eines Divinylbenzol/Styrol-Copolymerisates ist und der Katalysator acide Zentren in einer Konzentration von 2 bis 6 eq/kg nach DIN 54403 aufweist und der durchschnittliche Porendurchmesser der Katalysatorteilchen gemessen nach ASTM D 4222 1 bis 50 nm beträgt;
   2. Chlor freie Hydrierung der aromatischen Diamine zu cycloaliphatischen Diaminen durch Umsetzung der aromatischen Diamine mit Wasserstoff in Gegenwart eines Katalysators;
   3. Chlor freie oder Chlor arme und kontinuierliche Herstellung von cycloaliphatischen Diisocyanaten, durch Umsetzung von cycloalipahtischen Diaminen mit Kohlensäurederivaten wie Harnstoff und/oder Harnstoffäquivalenten und Alkoholen zu cycloaliphatischen Diurethanen und anschließenden thermischen Spaltung der Diurethane zu cycloaliphatischen Diisocyanten.

2. Verfahren nach Anspruch 1,
   **dadurch gekennzeichnet,**
   **dass** als Aldehyd Formaldehyd eingesetzt wird.

3. Verfahren nach Anspruch 1,
   **dadurch gekennzeichnet,**
   **dass** als Amin Anilin eingesetzt wird.

4. Verfahren nach Anspruch 1 bis 3,
   **dadurch gekennzeichnet,**
   **dass** Diaminodiphenylmethan hergestellt wird.

5. Verfahren nach mindestens einem der Ansprüche 1 bis 4, **dadurch gekennzeichnet,**
   **dass** das Isomerenverhältnis des erhaltenen Diaminodiphenylalkans wie folgt verteilt ist:

   | | |
   |---|---|
   | 64 bis 85 Gew.-% | 4,4'-Diaminodiphenylalkan |
   | 3 bis 20 Gew.-% | 2,4'-Diaminodiphenylalkan |
   | bis ≤ 2 Gew.-% | 2,2'-Diaminodiphenylalkan |

und bevorzugt
**dass** das Isomerenverhältnis des erhaltenen Diaminodiphenylmethans wie folgt verteilt ist:

| | |
|---|---|
| 64 bis 85 Gew.-% | 4,4'-Diaminodiphenylmethan |
| 3 bis 20 Gew.-% | 2,4'-Diaminodiphenylmethan |
| $\leq$ 2 Gew.-% | 2,2'-Diaminodiphenylmethan |

6. Verfahren nach mindestens einem der Ansprüche 1 bis 4,
   **dadurch gekennzeichnet,**
   **dass** das Isomerenverhältnis des erhaltenen Diaminodiphenylalkans wie folgt verteilt ist:

| | |
|---|---|
| 74 bis 85 Gew.-% | 4,4'-Diaminodiphenylalkan |
| 3 bis 20 Gew.-% | 2,4'-Diaminodiphenylalkan |
| $\leq$ 1,0 Gew.-% | 2,2'-Diaminodiphenylalkan |

und bevorzugt
**dass** das Isomerenverhältnis des erhaltenen Diaminodiphenylmethans wie folgt verteilt ist:

| | |
|---|---|
| 74 bis 85 Gew.-% | 4,4'-Diaminodiphenylmethan |
| 3 bis 20 Gew.-% | 2,4'-Diaminodiphenylmethan |
| $\leq$ 1,0 Gew.-% | 2,2'-Diaminodiphenylmethan |

7. Verfahren nach mindestens einem der vorherigen Ansprüche,
   **dadurch gekennzeichnet,**
   **dass** der Gehalt an N-Methylverbindungen $\leq$ 1 Gew.-% beträgt.

8. Verfahren nach mindestens einem der vorherigen Ansprüche,
   **dadurch gekennzeichnet,**
   **dass** der Gehalt an Mehrkernverbindungen $\leq$ 15 Gew.% und bevorzugt $\leq$ 10 Gew.-% beträgt.

9. Verfahren nach mindestens einem der vorherigen Ansprüche,
   **dadurch gekennzeichnet,**
   **dass** die Reaktionstemperatur im Bereich von 80 bis 140 °C liegt.

10. Verfahren nach mindestens einem der vorherigen Ansprüche,
    **dadurch gekennzeichnet,**
    **dass** die Reaktionszeit 30 min bis 5 Stunden beträgt.

11. Verfahren nach mindestens einem der vorherigen Ansprüche,
    **dadurch gekennzeichnet,**
    **dass** das Verhältnis von Amin zu Aldehyd 5:1 bis 15:1 beträgt.

12. Verfahren nach mindestens einem der vorherigen Ansprüche,
    **dadurch gekennzeichnet,**
    **dass** der Katalysator in trockener oder feuchter Form eingesetzt wird.

13. Verfahren nach mindestens einem der vorherigen Ansprüche,
    **dadurch gekennzeichnet,**
    **dass** das Verfahren kontinuierlich, diskontinuierlich oder semikontinuierlich durchgeführt wird.

14. Verfahren nach mindestens einem der vorherigen Ansprüche,
    **dadurch gekennzeichnet,**
    **dass** die Reaktion in einem Rührkessel, einer Rührkesselkaskade, einem Strömungsrohr, einem Festbettreaktor oder in einer Kolonne durchgeführt wird.

**15.** Verfahren nach mindestens einem der vorherigen Ansprüche,
**dadurch gekennzeichnet,**
**dass** als acide Zentren für den Katalysator Sulfonsäuregruppen verwendet werden.

**16.** Verfahren nach mindestens einem der vorherigen Ansprüche,
**dadurch gekennzeichnet,**
**dass** die Hydrierung in der 2. Stufe mit Katalysatoren ausgewählt aus Vollkontakten, Raney-Typ-Katalysatoren oder Trägerkatalysatoren erfolgt.

**17.** Verfahren nach mindestens einem der vorherigen Ansprüche,
**dadurch gekennzeichnet,**
**dass** Katalysatoren auf Basis von Nickel, Cobalt, Palladium, Platin, Ruthenium und Rhodium, allein oder in Mischungen, in der 2. Stufe eingesetzt werden.

**18.** Verfahren nach mindestens einem der vorherigen Ansprüche,
**dadurch gekennzeichnet,**
**dass** die Katalysatoren in der 2. Stufe zusätzlich Dotiermetalle und/oder andere Modifizierungsmittel enthalten.

**19.** Verfahren nach mindestens einem der vorherigen Ansprüche,
**dadurch gekennzeichnet,**
**dass** Trägerkatalysatoren in der 2. Stufe eingesetzt werden.

**20.** Verfahren nach mindestens einem der vorherigen Ansprüche,
**dadurch gekennzeichnet,**
**dass** Trägermaterialien, ausgewählt aus Aktivkohle, anorganischen Oxiden, Bentoniten, Alumosilikaten, Kaolinen, Tonen und/oder Kieselguren und/oder Lithiumaluminaten, in der 2. Stufe eingesetzt werden.

**21.** Verfahren nach mindestens einem der vorherigen Ansprüche,
**dadurch gekennzeichnet,**
**dass** in der 2. Stufe in Gegenwart eines geträgerten Katalysators, der als Aktivmetall Ruthenium alleine oder zusammen mit mindestens einem Metall der I. VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, hydriert wird.

**22.** Verfahren nach Anspruch 20,
**dadurch gekennzeichnet,**
**dass** in der 2. Stufe das Trägermaterial ausgewählt ist aus der Reihe $Al_2O_3$, $SiO_2$, $TiO_2$, $ZrO_2$, MgO, ZnO.

**23.** Verfahren nach mindestens einem der vorherigen Ansprüche,
**dadurch gekennzeichnet,**
**dass** Ruthenium und/oder Rhodium als Aktivmetall in der 2. Stufe enthalten sind.

**24.** Verfahren nach mindestens einem der vorherigen Ansprüche,
**dadurch gekennzeichnet,**
**dass** in der 2. Stufe die Hydrierung bei einer Temperatur im Bereich von 20 bis 200 °C, insbesondere 50 bis 150 °C, und einem Wasserstoffpartialdruck im Bereich von 3 bis 30 Mpa, insbesondere 3 bis 10 Mpa, durchgeführt wird.

**25.** Verfahren nach mindestens einem der vorherigen Ansprüche,
**dadurch gekennzeichnet,**
**dass** man die Hydrierung in einem Festbettreaktor, insbesondere in einem Rohrbündelreaktor durchführt.

**26.** Verfahren nach mindestens einem der vorherigen Ansprüche,
**dadurch gekennzeichnet,**
**dass** man die Hydrierung in zwei oder mehr in Reihe geschalteten Festbettreaktoren durchführt.

**27.** Verfahren nach mindestens einem der vorherigen Ansprüche,
**dadurch gekennzeichnet,**
**dass** man die Hydrierung in der 2. Stufe kontinuierlich durchführt.

**28.** Verfahren nach mindestens einem der vorherigen Ansprüche,
**dadurch gekennzeichnet,**
**dass** man als zu hydrierendes Stoffgemisch ein Roh-MDA, enthaltend mindestens 74 Gew.-% 4,4'-Diaminodiphenylmethan und 0,01 bis 2 Gew.-% N-Methylverbindungen, in der 2. Stufe einsetzt.

**29.** Verfahren nach mindestens einem der vorherigen Ansprüche,
**dadurch gekennzeichnet,**
**dass** in der 2. Stufe das zu hydrierende Stoffgemisch 74 - 85 Gew.-% 4,4'-MDA, 3 - 20 Gew.-% 2,4'-MDA, weniger als 1 Gew.-% 2,2'-MDA und bis zu 1 Gew.-% N-Methylverbindungen enthält.

**30.** Verfahren nach mindestens einem der vorherigen Ansprüche,
**dadurch gekennzeichnet,**
**dass** in der 2. Stufe die katalytische Hydrierung in Gegenwart eines Lösungsmittels durchgeführt wird.

**31.** Verfahren nach mindestens einem der vorherigen Ansprüche,
**dadurch gekennzeichnet,**
**dass** in der 2. Stufe Methylendicyclohexyldiamin hergestellt wird.

**32.** Verfahren nach mindestens einem der vorherigen Ansprüche,
**dadurch gekennzeichnet,**
**dass** in der 3. Stufe die cycloaliphatischen Diurethane nach einstufigen, zweistufigen oder alternativ auch mehrstufigen Verfahren hergestellt werden, nach ihrer Synthese durch Umsetzung von cycloaliphatischen Diaminen mit Alkohol und Harnstoff und/oder Harnstoff-Derivate von Leicht-, Mittel- und gegebenenfalls Hochsiedern befreit, die so gereinigten cycloaliphatischen Diurethane unter Freisetzung des gewünschten Diisocyanats thermisch gespalten, einen Teil des Spaltsumpfes aus der Spaltapparatur kontinuierlich ausgeschleust und nach Aufarbeitung oder alternativ ohne zusätzliche Reinigung mit Alkohol reurethanisiert und in den Prozess recycliert wird.

**33.** Verfahren nach mindestens einem der vorherigen Ansprüche,
**dadurch gekennzeichnet,**
**dass** in der 3. Stufe $H_{12}$MDI hergestellt wird.

**34.** Verfahren nach mindestens einem der vorherigen Ansprüche 1 bis 33,
**gekennzeichnet durch** ein mehrstufiges Verfahren zur kontinuierlichen Herstellung von cycloaliphatischen Diisocyanaten der 3. Stufe der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, **durch** Umsetzung cycloaliphatischer Diamine mit Harnstoff und/oder Harnstoffderivaten und Alkoholen in cycloaliphatische Diurethane, und deren thermische Spaltung, wobei

a) cycloaliphatische Diamine der Formel (II)

$H_2N$-R-$NH_2$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

$R^1$-OH

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamid-säurealkylestern oder Mischungen aus Dialkylcarbonaten und Carbamidsäureestern und in Abwesenheit oder Gegenwart von Katalysatoren zu cycloaliphatischen Diurethanen, umgesetzt werden und das entstehende Ammoniak gleichzeitig abtrennt wird;

b) aus der erhaltenen Reaktionsmischung der Alkohol, die Dialkylcarbonate und/oder Carbamidsäurealkylester abtrennt werden und der Alkohol sowie optional die Dialkylcarbonate und/oder Carbamidsäurealkylester in die Reaktionsstufe a) zurückführt werden;

c) der im Wesentlichen Diurethane enthaltende Stoffstrom aus Stufe b) destillativ in einen Wertstoffstrom und einen Nebenproduktstrom, der ausgeschleust wird, getrennt wird;

d) die über die Schritte b) und c) aufgereinigten Diurethane enthaltende Reaktionsmischung in Gegenwart eines Katalysators lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so gespalten wird, dass ein Teil des Reaktionsgemisches von 10 - 60 Gew.-% bezogen auf den Feed, vorzugsweise 15 - 45 Gew.-% bezogen auf den Feed, ständig ausgeschleust wird;

e) die Spaltprodukte **durch** Rektifikation in ein rohes cycloaliphatisches Diisocyanat und Alkohol getrennt werden;

f) das rohe cycloaliphatische Diisocyanat, **durch** Destillation gereinigt und die Reinproduktfraktion isoliert wird;

g) die Sumpfausschleusung aus d) mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1-15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;

h) ein Teil der Sumpffraktion der Reindestillation f) kontinuierlich ausschleust und in die Spaltreaktion d), vorzugsweise aber die Urethanisierungsstufe g) geführt wird;

i) die bei der Reindestillation des cycloaliphatischen Diisocyanats anfallende Kopffraktion ebenfalls in die Urethanisierungsstufe g) zurückgeführt oder verworfen wird;

j) der Reurethanisatstrom aus g) in Stufe b) zurückgeführt wird,

oder

k) der Reurethanisatstrom aus g) in die Reaktionsstufe a) zurückgeführt wird, unter der Voraussetzung, das g) in Gegenwart von Katalysatoren bevorzugt ausgewählt aus den Halogeniden von Fe(III) und/oder Cu(I), durchgeführt wird.

**35.** Verfahren nach mindestens einem der vorherigen Ansprüche 1 bis 33,
**gekennzeichnet durch** ein mehrstufiges Verfahren zur kontinuierlichen Herstellung von cycloaliphatischen Diisocyanaten der 3. Stufe der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, **durch** Umsetzung von cycloaliphatischen Diaminen mit Harnstoff und/oder Harnstoffderivaten und Alkoholen in cycloaliphatische Diurethane und deren thermische Spaltung, wobei

a) cycloaliphatische Diamine der Formel (II)

$H_2N$-R-$NH_2$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

$R^1$-OH

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt,
in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren, zu cycloaliphatischen Diurethanen umgesetzt werden und man das entstehende Ammoniak gleichzeitig abtrennt;

b) aus der erhaltenen Reaktionsmischung der Alkohol, die Dialkylcarbonate und/oder Carbamidsäurealkylester abgetrennt werden und man den Alkohol sowie optional auch die Dialkylcarbonate und/oder Carbamidsäurealkylester, in die Reaktionsstufe a) zurückführt;

c) auf eine Abtrennung der in der erhaltenen Reaktionsmischung gegebenenfalls enthaltenen hochsiedenden Rückstände vollständig oder partiell verzichtet wird;

d) die über die Schritte b) und optional c) aufgereinigten Diurethane enthaltende Reaktionsmischung in Gegenwart eines Katalysators kontinuierlich und lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so spaltet, dass ein Teil des Reaktionsgemisches von 10 - 60 Gew.%, bezogen auf den Feed, vorzugsweise 15 - 45 Gew.%, bezogen auf den Feed, ständig ausgeschleust wird;

e) die Spaltprodukte **durch** Rektifikation in ein rohes cycloaliphatisches Diisocyanat und Alkohol getrennt werden;

f) das rohe cycloaliphatische Diisocyanat, **durch** Destillation gereinigt und die Reinproduktfraktion isoliert wird;

g) die Sumpfausschleusung aus d) partiell oder vollständig mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1 - 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;

h) der Reurethanisatstrom aus g) in einen Wertstoff- und einen Abfallstrom aufgetrennt und der an Hochsiederkomponenten reiche Abfallstrom aus dem Prozess ausgeschleust und verworfen wird;

i) ein Teil der Sumpffraktion der Reindestillation f) kontinuierlich ausschleust und in die Spaltreaktion d) oder in die Urethanisierungsstufe g) geführt wird;

j) optional die bei der Reindestillation f) des rohen cycloaliphatischen Diisocyanats anfallende Kopffraktion ebenfalls in die Urethanisierungsstufe g) zurückgeführt wird;

k) der Wertstoffstrom aus h) in Stufe a), b) oder d) zurückgeführt wird.

36. Verfahren nach mindestens einem der vorherigen Ansprüche 1 bis 33,
**gekennzeichnet durch** ein mehrstufiges Verfahren zur kontinuierlichen Herstellung von cycloaliphatischen Diisocyanaten der 3. Stufe der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, **durch** Umsetzung von cycloaliphatischen Diaminen mit Harnstoff und/oder Harnstoffderivaten und Alkoholen in cycloaliphatische Diurethane und deren thermische Spaltung, wobei

a) cycloaliphatische Diamine der Formel (II)

$H_2N$-R-$NH_2$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

$R^1$-OH

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamid-säurealkylestern oder Mischungen aus Dialkylcarbonaten und Carbamidsäureestern und in Abwesenheit oder Gegenwart von Katalysatoren zu cycloaliphatischen Diurethanen umgesetzt werden und man das entstehende Ammoniak gleichzeitig abtrennt;

b) aus der erhaltenen Reaktionsmischung der Alkohol, die Dialkylcarbonate und/oder Carbamidsäurealkylester abgetrennt werden und man den Alkohol sowie optional, auch die Dialkylcarbonate und/oder Carbamidsäurealkylester, in die Reaktionsstufe a) zurückführt;

c) auf eine Abtrennung der in der erhaltenen Reaktionsmischung gegebenenfalls enthaltenen hochsiedenden Rückstände vollständig oder partiell verzichtet wird;

d) die über die Schritte b) und optional c) aufgereinigten Diurethane enthaltende Reaktionsmischung in Gegenwart eines Katalysators kontinuierlich und lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so spaltet, dass ein Teil des Reaktionsgemisches von 10 - 60 Gew.-% bezogen auf den Feed, vorzugsweise 15 - 45 Gew.-% bezogen auf den Feed, ständig ausgeschleust wird;

e) die Spaltprodukte **durch** Rektifikation in ein rohes cycloaliphatisches Diisocyanat und Alkohol getrennt wird;

f) das rohe cycloaliphatische Diisocyanat, **durch** Destillation gereinigt und die Reinproduktfraktion isoliert wird;

g) die Sumpfausschleusung aus d) in einen Wertstoff- und einen Abfallstrom aufgetrennt und der an Hochsiederkomponenten reiche Abfallstrom aus dem Prozess ausgeschleust und verworfen wird;

h) der Wertstoffstrom aus g) mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1 - 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;

i) ein Teil der Sumpffraktion der Reindestillation f) kontinuierlich ausschleust und in die Spaltreaktion d) oder in die Urethanisierungsstufe h) geführt wird;

j) optional die bei der Reindestillation f) des rohen cycloaliphatischen Diisocyanats anfallende Kopffraktion ebenfalls in die Urethanisierungsstufe h) zurückgeführt wird;

k) der Reurethanisatstrom aus h) in Stufe b) zurückführt wird;

oder

l) der Reurethanisatstrom aus h) in die Reaktionsstufe a) zurückgeführt wird, unter der Voraussetzung, das Stufe h) in Gegenwart von Katalysatoren, ausgewählt aus den Halogeniden von Fe(III) und/oder Cu(I), durchgeführt wird.

**37.** Verfahren nach mindestens einem der vorherigen Ansprüche1 bis 33,
**gekennzeichnet durch** ein mehrstufiges Verfahren zur kontinuierlichen Herstellung von cycloaliphatischen Diisocyanaten der 3. Stufe der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, **durch** Umsetzung cycloaliphatischer Diamine mit Kohlensäurederivaten und Alkoholen zu Diurethanen, und deren thermische Spaltung, wobei

a) cycloaliphatische Diamine der Formel (II)

$H_2N$-R-$NH_2$

wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

$R^1$-OH

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Katalysatoren zu Cycloalkylenbisharn-stoffen der Formel (IV)

$H_2N$-OC-HN-R-NH-CO-$NH_2$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;

b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

$$R^1O\text{-}OC\text{-}HN\text{-}R\text{-}NH\text{-}CO\text{-}OR^1$$

überführt wird;

c) aus der erhaltenen Reaktionsmischung der Alkohol, die Dialkylcarbonate und/oder Carbamidsäurealkylester abgetrennt und der Alkohol in die Reaktionsstufe a) zurückführt wird;

d) auf eine Abtrennung der in der erhaltenen Reaktionsmischung gegebenenfalls enthaltenen hochsiedenden Rückstände vollständig oder partiell verzichtet wird;

e) die über die Schritte c) und d) aufgereinigte Diurethane enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.% bezogen auf den Feed, ständig ausgeschleust wird;

f) die Spaltprodukte **durch** Rektifikation in ein rohes Diisocyanat und Alkohol getrennt werden;

g) das rohe cycloaliphatische Diisocyanat **durch** Destillation gereinigt und die Reinproduktfraktion isoliert wird;

h) die Sumpfausschleusung aus e) in einen Wertstoff- und einen Abfallstrom aufgetrennt und der an Hochsiederkomponenten reiche Abfallstrom aus dem Prozess ausgeschleust und verworfen wird;

i) der Wertstoffstrom aus h) mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;

j) ein Teil der Sumpffraktion der Reindestillation g) kontinuierlich ausgeschleust und in die Spaltreaktion e), und/oder in die Urethanisierungsstufe i) geführt wird;

k) optional die bei der Reindestillation des rohen cycloaliphatischen Diisocyanats anfallende Kopffraktion ebenfalls in die Urethanisierungsstufe i) zurückgeführt wird;

l) der Reurethanisatstrom aus i) in Stufe b) und/oder c) zurückgeführt wird.

**38.** Verfahren nach mindestens einem der vorherigen Ansprüche 1 bis 33, **gekennzeichnet durch** ein mehrstufiges Verfahren zur kontinuierlichen Herstellung von cycloaliphatischen Diisocyanaten der 3. Stufe der Formel (I)

$$OCN\text{-}R\text{-}NCO$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, **durch** Umsetzung cycloaliphatischer Diamine mit Kohlensäurederivaten und Alkoholen zu Diurethanen, und deren thermische Spaltung, wobei

a) cycloaliphatische Diamine der Formel (II)

$$H_2N\text{-}R\text{-}NH_2$$

wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

$$R^1\text{-}OH$$

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Katalysatoren zu Cycloalkylenbisharn-stoffen der Formel (IV)

$$H_2N\text{-}OC\text{-}HN\text{-}R\text{-}NH\text{-}CO\text{-}NH_2$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;

b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

$$R^1O\text{-}OC\text{-}HN\text{-}R\text{-}NH\text{-}CO\text{-}OR^1$$

überführt wird;

c) aus der erhaltenen Reaktionsmischung der Alkohol, die Dialkylcarbonate und/oder Carbamidsäurealkylester abgetrennt und der Alkohol in die Reaktionsstufe a) zurückführt wird;

d) der Stoffstrom aus Stufe c) destillativ in einen Wertstoffstrom und einen Nebenproduktstrom, der ausgeschleust wird, getrennt wird,

e) die über die Schritte c) und d) aufgereinigte Diurethane enthaltende Reaktionsmischung in Gegenwart eines Katalysators lösemittelfrei bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig ausgeschleust wird;

f) die Spaltprodukte **durch** Rektifikation in ein rohes Diisocyanat und Alkohol getrennt werden;

g) das rohe cycloaliphatische Diisocyanat **durch** Destillation gereinigt und die Reinproduktfraktion isoliert wird;

h) die Sumpfausschleusung aus e) mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;

i) optional die Reurethanisierungsreaktion h) in Gegenwart spezieller Katalysatoren, ausgewählt aus den Halogeniden von Fe(III) und/oder Cu(I), durchgeführt wird;

j) ein Teil der Sumpffraktion der Reindestillation g) kontinuierlich ausgeschleust und in die Spaltreaktion e), und/oder in die Urethanisierungsstufe h) geführt wird;

k) optional die bei der Reindestillation des rohen cycloaliphatischen Diisocyanats anfallende Kopffraktion ebenfalls in die Urethanisierungsstufe h) zurückgeführt wird;

l) der Reurethanisatstrom aus h) in Stufe c) zurückgeführt wird.

39. Verfahren nach mindestens einem der vorherigen Ansprüche 1 bis 33,
**gekennzeichnet durch** ein mehrstufiges Verfahren zur kontinuierlichen Herstellung von cycloaliphatischen Diisocyanaten der 3. Stufe der Formel (I)

$$OCN\text{-}R\text{-}NCO$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, **durch** Umsetzung cycloaliphatischer Diamine mit Kohlensäurederivaten und Alkoholen zu Diurethanen, und deren thermische Spaltung, wobei

a) cycloaliphatische Diamine der Formel (II)

$$H_2N\text{-}R\text{-}NH_2$$

wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

$$R^1\text{-}OH$$

wobei $R^1$ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Katalysatoren zu Cycloalkylenbisharnstoffen der Formel (IV)

$$H_2N\text{-}OC\text{-}HN\text{-}R\text{-}NH\text{-}CO\text{-}NH_2$$

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;

b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

$$R^1O\text{-}OC\text{-}HN\text{-}R\text{-}NH\text{-}CO\text{-}OR^1$$

überführt wird;

c) aus der erhaltenen Reaktionsmischung der Alkohol, die Dialkylcarbonate und/oder Carbamidsäurealkylester abgetrennt und der Alkohol in die Reaktionsstufe a) zurückführt wird;

d) auf eine Abtrennung der in der erhaltenen Reaktionsmischung gegebenenfalls enthaltenen hochsiedenden Rückstände vollständig oder partiell verzichtet wird;

e) die über die Schritte c) und d) aufgereinigte Diurethane enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.% bezogen auf den Feed, vorzugsweise 15 bis 45 Gew.% bezogen auf den Feed, ständig aus dem Sumpf ausgeschleust wird;

f) die Spaltprodukte **durch** Rektifikation in ein rohes Diisocyanat und Alkohol getrennt werden;

g) das rohe cycloaliphatische Diisocyanat **durch** Destillation gereinigt und die Reinproduktfraktion isoliert wird;

h) die Sumpfausschleusung aus e) partiell oder vollständig mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;

i) der Wertstoffstrom aus h) in einen Wertstoff- und einen Abfallstrom aufgetrennt und der an Hochsiederkomponenten reiche Abfallstrom aus dem Prozess ausgeschleust und verworfen wird;

j) ein Teil der Sumpffraktion der Reindestillation g) kontinuierlich ausgeschleust und in die Spaltreaktion e) und/oder in die Urethanisierungsstufe h) geführt wird;

k) optional die bei der Reindestillation des rohen cycloaliphatischen Diisocyanats anfallende Kopffraktion ebenfalls in die Urethanisierungsstufe h) zurückgeführt wird;

l) der aufgereinigte Reurethanisatstrom aus i) in Stufe b) und/oder c) oder e) zurückgeführt wird.

40. Verfahren nach mindestens einem der vorherigen Ansprüche,
**dadurch gekennzeichnet,**
**dass** der Gehalt an Chlor, berechnet als Chlor-Ion, M = 35,45 g/mol, kleiner als 100 ppm, bevorzugt kleiner 50 ppm, besonders bevorzugt kleiner 20 ppm, beträgt, bezogen auf alle sich in der 3. Stufe anwesenden Mengen an Stoffen.

41. Verfahren nach mindestens einem der vorherigen Ansprüche,
**dadurch gekennzeichnet,**
**dass** der Gehalt an Chlor, berechnet als Chlor-Ion, M = 35,45 g/mol, kleiner als 100 ppm, bevorzugt kleiner 50 ppm, besonders bevorzugt kleiner 20 ppm, beträgt, bezogen auf alle sich in der Spaltung anwesenden Mengen an Stoffen.

42. Verfahren nach mindestens einem der vorherigen Ansprüche,
**dadurch gekennzeichnet,**
**dass** die während der Spaltung oder nach der Spaltung ausgeschleusten oder durch Aufarbeitung erhaltenen Produktströme reurethanisiert werden, wobei die Menge an Chlor, berechnet als Chlor-Ion, M = 35,45 g/mol, weniger als 100 ppm, bevorzugt weniger als 50 ppm, bezogen auf alle in der Reaktionsmischung anwesenden Mengen an Stoffen.

43. Verfahren nach mindestens einem der vorherigen Ansprüche,
**dadurch gekennzeichnet,**
**dass** in der 3. Stufe konditionierter oder bevorzugt unkonditionierter Harnstoff eingesetzt wird.

44. Verfahren nach Anspruch 43,

**dadurch gekennzeichnet,**
**dass** der Harnstoff, bevorzugt der unkonditionierte Harnstoff, als Prills, Granulat, Kristalle, Schmelze, Lösung, Suspension eingesetzt wird.

## Claims

1. A low-chlorine multistage and continuous process for preparing cycloaliphatic diisocyanates by

   1. chlorine-free preparation of aromatic diamines by reacting an aromatic amine which may be substituted or unsubstituted with a $C_1$-$C_3$ aldehyde in the presence of a heterogeneous catalyst, said catalyst being a mesoporous acidic ion exchanger based on a divinylbenzene/styrene copolymer and said catalyst having acidic sites in a concentration of 2 to 6 eq/kg to DIN 54403, and the average pore diameter of the catalyst particles measured to ASTM D 4222 being 1 to 50 nm;
   2. chlorine-free hydrogenation of the aromatic diamines to cycloaliphatic diamines by reacting the aromatic diamines with hydrogen in the presence of a catalyst;
   3. chlorine-free or low-chlorine and continuous production of cycloaliphatic diisocyanates by reacting cycloaliphatic diamines with carbonic acid derivatives such as urea and/or urea equivalents and alcohols to give cycloaliphatic diurethanes and subsequent thermal cleavage of the diurethanes to give cycloaliphatic diisocyanates.

2. A process according to claim 1,
   **characterized in that**
   the aldehyde used is formaldehyde.

3. A process according to claim 1,
   **characterized in that**
   the amine used is aniline.

4. A process according to any of claims 1 to 3,
   **characterized in that**
   diaminodiphenylmethane is prepared.

5. A process according to at least one of claims 1 to 4, **characterized in that** the isomer ratio of the resulting diaminodiphenylalkane has the following distribution:

   | 64 to 85% by weight of | 4,4'-diaminodiphenylalkane |
   |---|---|
   | 3 to 20% by weight of | 2,4'-diaminodiphenylalkane |
   | up to ≤ 2% by weight of | 2,2'-diaminodiphenylalkane, |

   and preferably
   **in that** the isomer ratio of the resulting diaminodiphenylmethane has the following distribution:

   | 64 to 85% by weight of | 4,4'-diaminodiphenylmethane |
   |---|---|
   | 3 to 20% by weight of | 2,4'-diaminodiphenylmethane |
   | ≤ 2% by weight of | 2,2'-diaminodiphenylmethane. |

6. A process according to at least one of claims 1 to 4, **characterized in that**
   the isomer ratio of the resulting diaminodiphenylalkane has the following distribution:

   | 74 to 85% by weight of | 4,4'-diaminodiphenylalkane |
   |---|---|
   | 3 to 20% by weight of | 2,4'-diaminodiphenylalkane |
   | ≤ 1.0% by weight of | 2,2'-diaminodiphenylalkane, |

   and preferably

**in that** the isomer ratio of the resulting diaminodiphenylmethane has the following distribution:

| | |
|---|---|
| 74 to 85% by weight of | 4,4'-diaminodiphenylmethane |
| 3 to 20% by weight of | 2,4'-diaminodiphenylmethane |
| ≤ 1.0% by weight of | 2,2'-diaminodiphenylmethane. |

7. A process according to at least one of the preceding claims,
   **characterized in that**
   the content of N-methyl compounds is ≤ 1% by weight.

8. A process according to at least one of the preceding claims,
   **characterized in that**
   the content of polycyclic compounds is ≤ 15% by weight and preferably ≤ 10% by weight.

9. A process according to at least one of the preceding claims,
   **characterized in that**
   the reaction temperature is in the range of 80 to 140°C.

10. A process according to at least one of the preceding claims,
    **characterized in that**
    the reaction time is 30 min to 5 hours.

11. A process according to at least one of the preceding claims,
    **characterized in that**
    the ratio of amine to aldehyde is 5:1 to 15:1.

12. A process according to at least one of the preceding claims,
    **characterized in that**
    the catalyst is used in dry or moist form.

13. A process according to at least one of the preceding claims,
    **characterized in that**
    it is performed continuously, batchwise or semicontinuously.

14. A process according to at least one of the preceding claims,
    **characterized in that**
    the reaction is performed in a stirred tank, a stirred tank cascade, a flow tube, a fixed bed reactor or in a column.

15. A process according to at least one of the preceding claims,
    **characterized in that**
    the acidic sites used for the catalyst are sulphonic acid groups.

16. A process according to at least one of the preceding claims,
    **characterized in that**
    the hydrogenation in the 2nd stage is effected with catalysts selected from unsupported catalysts, Raney-type catalysts and supported catalysts.

17. A process according to at least one of the preceding claims,
    **characterized in that**
    catalysts based on nickel, cobalt, palladium, platinum, ruthenium and rhodium, alone or in mixtures, are used in the 2nd stage.

18. A process according to at least one of the preceding claims,
    **characterized in that**
    the catalysts in the 2nd stage additionally contain dopant metals and/or other modifiers.

**19.** A process according to at least one of the preceding claims,
**characterized in that**
supported catalysts are used in the 2nd stage.

**20.** A process according to at least one of the preceding claims,
**characterized in that**
support materials selected from activated carbon, inorganic oxides, bentonites, aluminosilicates, kaolins, clays and/or kieselguhrs and/or lithium aluminates are used in the 2nd stage.

**21.** A process according to at least one of the preceding claims,
**characterized in that**
hydrogenation is effected in the 2nd stage in the presence of a supported catalyst which, as the active metal, contains ruthenium alone or together with at least one metal of transition group I, VII or VIII of the Periodic Table in an amount of 0.01 to 20% by weight of active metals, based on the supported catalyst, applied to a support.

**22.** A process according to claim 20,
**characterized in that**
the support material in the 2nd stage is selected from the group of $Al_2O_3$, $SiO_2$, $TiO_2$, $ZrO_2$, MgO, ZnO.

**23.** A process according to at least one of the preceding claims,
**characterized in that**
ruthenium and/or rhodium is/are present as the active metal in the 2nd stage.

**24.** A process according to at least one of the preceding claims,
**characterized in that**
the hydrogenation in the 2nd stage is performed at a temperature in the range of 20 to 200°C, especially 50 to 150°C, and a partial hydrogen pressure in the range of 3 to 30 MPa, especially 3 to 10 MPa.

**25.** A process according to at least one of the preceding claims,
**characterized in that**
the hydrogenation is performed in a fixed bed reactor, especially in a tube bundle reactor.

**26.** A process according to at least one of the preceding claims,
**characterized in that**
the hydrogenation is performed in two or more fixed bed reactors connected in series.

**27.** A process according to at least one of the preceding claims,
**characterized in that**
the hydrogenation is performed continuously in the 2nd stage.

**28.** A process according to at least one of the preceding claims,
**characterized in that**
the substance mixture to be hydrogenated which is used in the 2nd stage is crude MDA containing at least 74% by weight of 4,4'-diaminodiphenylmethane and 0.01 to 2% by weight of N-methyl compounds.

**29.** A process according to at least one of the preceding claims,
**characterized in that**
the substance mixture to be hydrogenated in the 2nd stage contains 74-85% by weight of 4,4'-MDA, 3-20% by weight of 2,4'-MDA, less than 1% by weight of 2,2'-MDA and up to 1% by weight of N-methyl compounds.

**30.** A process according to at least one of the preceding claims,
**characterized in that**
the catalytic hydrogenation in the 2nd stage is performed in the presence of a solvent.

**31.** A process according to at least one of the preceding claims,
**characterized in that**
methylenedicyclohexyldiamine is prepared in the 2nd stage.

32. A process according to at least one of the preceding claims,
**characterized in that**
the cycloaliphatic diurethanes are prepared in the 3rd stage by one-stage, two-stage or else alternatively multistage processes, and are freed of low boilers, medium boilers and any high boilers after they have been synthesized by reaction of cycloaliphatic diamines with alcohol and urea and/or urea derivatives, the cycloaliphatic diurethanes thus purified are cleaved thermally to release the desired diisocyanate, and a portion of the cleavage bottoms is discharged continuously from the cleavage apparatus and, after workup or alternatively without additional purification, is reurethanized with alcohol and recycled into the process.

33. A process according to at least one of the preceding claims,
**characterized in that**
$H_{12}$MDI is prepared in the 3rd stage.

34. A process according to at least one of the preceding claims 1 to 33,
**characterized by** a multistage process for continuously preparing cycloaliphatic diisocyanates of the 3rd stage of the formula (I)

**OCN-R-NCO**

where R is a divalent cycloaliphatic hydrocarbon radical having 4 to 18, preferably 5 to 15, carbon atoms, with the proviso that the two isocyanate groups are bonded directly to a hydrocarbon cycle and at least 3 carbon atoms are arranged between them, by reacting cycloaliphatic diamines with urea and/or urea derivatives and alcohols to give cycloaliphatic diurethanes, and thermally cleaving the latter, wherein

a) cycloaliphatic diamines of the formula (II)

**$H_2$N-R-NH$_2$**

where R is a divalent cycloaliphatic hydrocarbon radical having 4 to 18, preferably 5 to 15, carbon atoms, with the proviso that the two nitrogen atoms are bonded directly to a hydrocarbon cycle and at least 3 carbon atoms are arranged between them, are reacted with urea and/or urea derivatives and alcohols of the formula (III)

**$R^1$-OH**

where $R^1$ is a radical as remains after removal of the hydroxyl group from a primary or secondary (cyclo)aliphatic alcohol having 3 to 8 carbon atoms, in the absence or presence of dialkyl carbonates, alkyl carbamates or mixtures of dialkyl carbonates and carbamic esters, and in the absence or presence of catalysts, to give cycloaliphatic diurethanes and the ammonia formed is removed simultaneously;
b) the alcohol, the dialkyl carbonates and/or alkyl carbamates are removed from the resulting reaction mixture, and the alcohol and optionally the dialkyl carbonates and/or alkyl carbamates are recycled into reaction stage a);
c) the stream from stage b) which contains essentially diurethanes is separated by distillation into a material-of-value stream and a by-product stream which is discharged;
d) the reaction mixture which contains diurethanes and is purified via steps b) and c) is thermally cleaved continuously in the presence of a catalyst and without solvent at temperatures of 180-280°C, preferably 200-260°C, and under a pressure of 0.1-200 mbar, preferably 0.2-100 mbar, such that a portion of the reaction mixture of 10-60% by weight based on the feed, preferably 15-45% by weight based on the feed, is discharged continuously;
e) the cleavage products are separated by rectification into a crude cycloaliphatic diisocyanate and alcohol;
f) the crude cycloaliphatic diisocyanate is purified by distillation and the pure product fraction is isolated;
g) the bottoms discharge from d) is reacted with the alcohol from e) in the presence or absence of catalysts at temperatures of 20-200°C, preferably 50-170°C, and at a pressure of 0.5-20 bar, preferably 1-15 bar, over the course of 1-150 min, preferably 3-60 min, where the molar ratio of NCO groups and OH groups is up to 1:100, preferably 1:20 and more preferably 1:10;
h) a portion of the bottoms fraction from the purifying distillation f) is discharged continuously and conducted into the cleavage reaction d), but preferably the urethanization stage g);
i) the top fraction obtained in the purifying distillation of the cycloaliphatic diisocyanate is likewise recycled into the urethanization stage g) or discarded;
j) the reurethanized stream from g) is recycled into stage b),

or

k) the reurethanized stream from g) is recycled into the reaction stage a), with the prerequisite that g) is carried out in the presence of catalysts preferably selected from the halides of Fe(III) and/or Cu(I).

**35.** A process according to at least one of the preceding claims 1 to 33,
**characterized by** a multistage process for continuously preparing cycloaliphatic diisocyanates of the 3rd stage of the formula (I)

**OCN-R-NCO**

where R is a divalent cycloaliphatic hydrocarbon radical having 4 to 18, preferably 5 to 15, carbon atoms, with the proviso that the two nitrogen atoms are bonded directly to a hydrocarbon cycle and at least 3 carbon atoms are arranged between them, by reacting cycloaliphatic diamines with urea and/or urea derivatives and alcohols to give cycloaliphatic diurethanes, and thermally cleaving the latter, wherein

a) cycloaliphatic diamines of the formula (II)

**$H_2N$-R-$NH_2$**

where R is a divalent cycloaliphatic hydrocarbon radical having 4 to 18, preferably 5 to 15, carbon atoms, with the proviso that the two nitrogen atoms are bonded directly to a hydrocarbon cycle and at least 3 carbon atoms are arranged between them, are reacted with urea and/or urea derivatives and alcohols of the formula (III)

**$R^1$-OH**

where $R^1$ is a radical as remains after removal of the hydroxyl group from a primary or secondary (cyclo)aliphatic alcohol having 3 to 8 carbon atoms, in the absence or presence of dialkyl carbonates, alkyl carbamates or mixtures of dialkyl carbonates and carbamic esters, and in the absence or presence of catalysts, to give cycloaliphatic diurethanes and the ammonia formed is removed simultaneously;
b) the alcohol, the dialkyl carbonates and/or alkyl carbamates are removed from the resulting reaction mixture, and the alcohol and optionally the dialkyl carbonates and/or alkyl carbamates are recycled into reaction stage a);
c) a removal of any high-boiling residues present in the resulting reaction mixture is completely or partially dispensed with;
d) the reaction mixture which contains diurethanes and is purified via steps b) and optionally c) is thermally cleaved continuously in the presence of a catalyst and without solvent at temperatures of 180-280°C, preferably 200-260°C, and under a pressure of 0.1-200 mbar, preferably 0.2-100 mbar, such that a portion of the reaction mixture of 10-60% by weight based on the feed, preferably 15-45% by weight based on the feed, is discharged continuously;
e) the cleavage products are separated by rectification into a crude cycloaliphatic diisocyanate and alcohol;
f) the crude cycloaliphatic diisocyanate is purified by distillation and the pure product fraction is isolated;
g) the bottoms discharge from d) is reacted partially or completely with the alcohol from e) in the presence or absence of catalysts at temperatures of 20-200°C, preferably 50-170°C, and at a pressure of 0.5-20 bar, preferably 1-15 bar, over the course of 1-150 min, preferably 3-60 min, where the molar ratio of NCO groups and OH groups is up to 1:100, preferably 1:20 and more preferably 1:10;
h) the reurethanized stream from g) is separated into a material-of-value stream and a waste stream and the waste stream which is rich in high boiler components is discharged from the process and discarded;
i) a portion of the bottoms fraction of the purifying distillation f) is discharged continuously and conducted into the cleavage reaction d) or into the urethanization stage g);
j) optionally, the top fraction obtained in the purifying distillation f) of the crude cycloaliphatic diisocyanate is likewise recycled into the urethanization stage g);
k) the material-of-value stream from h) is recycled into stage a), b) or d).

**36.** A process according to at least one of the preceding claims 1 to 33,
**characterized by** a multistage process for continuously preparing cycloaliphatic diisocyanates of the 3rd stage of the formula (I)

**OCN-R-NCO**

where R is a divalent cycloaliphatic hydrocarbon radical having 4 to 18, preferably 5 to 15, carbon atoms, with the proviso that the two nitrogen atoms are bonded directly to a hydrocarbon cycle and at least 3 carbon atoms are arranged between them, by reacting cycloaliphatic diamines with urea and/or urea derivatives and alcohols to give cycloaliphatic diurethanes, and thermally cleaving the latter, wherein

a) cycloaliphatic diamines of the formula (II)

**H$_2$N-R-NH$_2$**

where R is a divalent cycloaliphatic hydrocarbon radical having 4 to 18, preferably 5 to 15, carbon atoms, with the proviso that the two nitrogen atoms are bonded directly to a hydrocarbon cycle and at least 3 carbon atoms are arranged between them, are reacted with urea and/or urea derivatives and alcohols of the formula (III)

**R$^1$-OH**

where R$^1$ is a radical as remains after removal of the hydroxyl group from a primary or secondary (cyclo)aliphatic alcohol having 3 to 8 carbon atoms, in the absence or presence of dialkyl carbonates, alkyl carbamates or mixtures of dialkyl carbonates and carbamic esters, and in the absence or presence of catalysts, to give cycloaliphatic diurethanes and the ammonia formed is removed simultaneously;

b) the alcohol, the dialkyl carbonates and/or alkyl carbamates are removed from the resulting reaction mixture, and the alcohol and optionally the dialkyl carbonates and/or alkyl carbamates are recycled into reaction stage a);

c) a removal of any high-boiling residues present in the resulting reaction mixture is completely or partially dispensed with;

d) the reaction mixture which contains diurethanes and is purified via steps b) and optionally c) is thermally cleaved continuously in the presence of a catalyst and without solvent at temperatures of 180-280°C, preferably 200-260°C, and under a pressure of 0.1-200 mbar, preferably 0.2-100 mbar, such that a portion of the reaction mixture of 10-60% by weight based on the feed, preferably 15-45% by weight based on the feed, is discharged continuously;

e) the cleavage products are separated by rectification into a crude cycloaliphatic diisocyanate and alcohol;

f) the crude cycloaliphatic diisocyanate is purified by distillation and the pure product fraction is isolated;

g) the bottoms discharge from d) is separated into a material-of-value stream and a waste stream and the waste stream which is rich in high boiler components is discharged from the process and discarded;

h) the material-of-value stream from g) is reacted with the alcohol from e) in the presence or absence of catalysts at temperatures of 20-200°C, preferably 50-170°C, and at a pressure of 0.5-20 bar, preferably 1-15 bar, over the course of 1-150 min, preferably 3-60 min, where the molar ratio of NCO groups and OH groups is up to 1:100, preferably 1:20 and more preferably 1:10;

i) a portion of the bottoms fraction of the purifying distillation f) is discharged continuously and conducted into the cleavage reaction d) or into the urethanization stage h);

j) optionally, the top fraction obtained in the purifying distillation f) of the crude cycloaliphatic diisocyanate is likewise recycled into the urethanization stage h);

k) the reurethanized stream from h) is recycled into stage b);

or

l) the reurethanized stream from h) is recycled into the reaction stage a), with the prerequisite that stage h) is carried out in the presence of catalysts selected from the halides of Fe(III) and/or Cu(I).

**37.** A process according to at least one of the preceding claims 1 to 33, **characterized by** a multistage process for continuously preparing cycloaliphatic diisocyanates of the 3rd stage of the formula (I)

**OCN-R-NCO**

where R is a divalent cycloaliphatic hydrocarbon radical having 4 to 18, preferably 5 to 15, carbon atoms, with the proviso that the two isocyanate groups are bonded directly to a hydrocarbon cycle and at least 3 carbon atoms are arranged between them, by reacting cycloaliphatic diamines with carbonic acid derivatives and alcohols to give diurethanes, and thermally cleaving the latter, wherein

a) cycloaliphatic diamines of the formula (II)

**H$_2$N-R-NH$_2$**

where R is a divalent cycloaliphatic hydrocarbon radical having 4 to 18, preferably 5 to 15, carbon atoms, where the two nitrogen atoms are bonded directly to at least one hydrocarbon cycle and at least 3 carbon atoms are arranged between them, are reacted with urea and in the presence of alcohols of the formula (III)

**R$^1$-OH**

where R$^1$ is a radical as remains after removal of the hydroxyl group from a primary or secondary (cyclo)aliphatic alcohol having 3 to 8 carbon atoms, in the absence or presence of catalysts, to give cycloalkylenebisureas of the formula (IV)

**H$_2$N-OC-HN-R-NH-CO-NH$_2$**

where R is a divalent cycloaliphatic hydrocarbon radical having 4 to 18, preferably 5 to 15, carbon atoms, with the proviso that the two nitrogen atoms flanking R are bonded directly to a hydrocarbon cycle and at least 3 carbon atoms are arranged between them, and the ammonia formed is simultaneously removed continuously;
b) the crude cycloalkylenebisurea obtained is converted in a second reactor using the alcohol of the formula (III) used as a solvent in a), while continuously driving out the ammonia released, to cycloalkylenediurethane of the formula (V)

**R$^1$O-OC-HN-R-NH-CO-OR$^1$;**

c) the alcohol, the dialkyl carbonates and/or alkyl carbamates are removed from the resulting reaction mixture and the alcohol is recycled into reaction stage a);
d) a removal of any high-boiling residues present in the resulting reaction mixture is completely or partially dispensed with;
e) the reaction mixture which contains diurethanes and is purified via steps c) and d) is thermally cleaved continuously in the presence of a catalyst and without solvent at temperatures of 180 to 280°C, preferably 200 to 260°C, and under a pressure of 0.1 to 200 mbar, preferably 0.2 to 100 mbar, such that a portion of the reaction mixture of 10 to 60% by weight based on the feed, preferably 15 to 45% by weight based on the feed, is discharged continuously;
f) the cleavage products are separated by rectification into a crude diisocyanate and alcohol;
g) the crude cycloaliphatic diisocyanate is purified by distillation and the pure product fraction is isolated;
h) the bottoms discharge from e) is separated into a material-of-value stream and a waste stream and the waste stream which is rich in high boiler components is discharged from the process and discarded;
i) the material-of-value stream from h) is reacted with the alcohol from f) in the presence or absence of catalysts at temperatures of 20 to 200°C, preferably 50 to 170°C, and at a pressure of 0.5 to 20 bar, preferably 1 to 15 bar, over the course of 1 to 150 min, preferably 3 to 60 min, where the molar ratio of NCO groups and OH groups is up to 1:100, preferably 1:20 and more preferably 1:10;
j) a portion of the bottoms fraction of the purifying distillation g) is discharged continuously and conducted into the cleavage reaction e) and/or into the urethanization stage i);
k) optionally, the top fraction obtained in the purifying distillation of the crude cycloaliphatic diisocyanate is likewise recycled into the urethanization stage i);
l) the reurethanized stream from i) is recycled into stage b) and/or c).

**38.** A process according to at least one of the preceding claims 1 to 33,
**characterized by** a multistage process for continuously preparing cycloaliphatic diisocyanates of the 3rd stage of the formula (I)

OCN-R-NCO

where R is a divalent cycloaliphatic hydrocarbon radical having 4 to 18, preferably 5 to 15, carbon atoms, with the proviso that the two isocyanate groups are bonded directly to a hydrocarbon cycle and at least 3 carbon atoms are arranged between them, by reacting cycloaliphatic diamines with carbonic acid derivatives and alcohols to give diurethanes, and thermally cleaving the latter, wherein

a) cycloaliphatic diamines of the formula (II)

$$H_2N-R-NH_2$$

where R is a divalent cycloaliphatic hydrocarbon radical having 4 to 18, preferably 5 to 15, carbon atoms, where the two nitrogen atoms are bonded directly to a hydrocarbon cycle and at least 3 carbon atoms are arranged between them, are reacted with urea and in the presence of alcohols of the formula (III)

$$R^1-OH$$

where R1 is a radical as remains after removal of the hydroxyl group from a primary or secondary (cyclo)aliphatic alcohol having 3 to 8 carbon atoms, in the absence or presence of catalysts, to give cycloalkylenebisureas of the formula (IV)

$$H_2N-OC-HN-R-NH-CO-NH_2$$

where R is a divalent cycloaliphatic hydrocarbon radical having 4 to 18, preferably 5 to 15, carbon atoms, with the proviso that the two nitrogen atoms flanking R are bonded directly to a hydrocarbon cycle and at least 3 carbon atoms are arranged between them, and the ammonia formed is simultaneously removed continuously;
b) the crude cycloalkylenebisurea obtained is converted in a second reactor using the alcohol of the formula (III) used as a solvent in a), while continuously driving out the ammonia released, to cycloalkylenediurethane of the formula (V)

$$R^1O-OC-HN-R-NH-CO-OR^1;$$

c) the alcohol, the dialkyl carbonates and/or alkyl carbamates are removed from the resulting reaction mixture and the alcohol is recycled into reaction stage a);
d) the stream from stage c) is separated by distillation into a material-of-value stream and a by-product stream which is discharged,
e) the reaction mixture which contains diurethanes and is purified via steps c) and d) is thermally cleaved continuously in the presence of a catalyst and without a solvent at temperatures of 180 to 280°C, preferably 200 to 260°C, and under a pressure of 0.1 to 200 mbar, preferably 0.2 to 100 mbar, such that a portion of the reaction mixture of 10 to 60% by weight based on the feed, preferably 15 to 45% by weight based on the feed, is discharged continuously;
f) the cleavage products are separated by rectification into a crude diisocyanate and alcohol;
g) the crude cycloaliphatic diisocyanate is purified by distillation and the pure product fraction is isolated;
h) the bottoms discharge from e) is reacted with the alcohol from f) in the presence or absence of catalysts at temperatures of 20 to 200°C, preferably 50 to 170°C, and at a pressure of 0.5 to 20 bar, preferably 1 to 15 bar, over the course of 1 to 150 min, preferably 3 to 60 min, where the molar ratio of NCO groups and OH groups is up to 1:100, preferably 1:20 and more preferably 1:10;
i) optionally, the reurethanization reaction h) is performed in the presence of specific catalysts selected from the halides of Fe(III) and/or Cu(I);
j) a portion of the bottoms fraction of the purifying distillation g) is discharged continuously and conducted into the cleavage reaction e) and/or into the urethanization stage h);
k) optionally, the top fraction obtained in the purifying distillation of the crude cycloaliphatic diisocyanate is likewise recycled into the urethanization stage h);
l) the reurethanized stream from h) is recycled into stage c).

39. A process according to at least one of the preceding claims 1 to 33,
**characterized by** a multistage process for continuously preparing cycloaliphatic diisocyanates of the 3rd stage of the formula (I)

$$OCN-R-NCO$$

where R is a divalent cycloaliphatic hydrocarbon radical having 4 to 18, preferably 5 to 15, carbon atoms, with the proviso that the two isocyanate groups are bonded directly to a hydrocarbon cycle and at least 3 carbon atoms are arranged between them, by reacting cycloaliphatic diamines with carbonic acid derivatives and alcohols to give diurethanes, and thermally cleaving the latter, wherein

a) cycloaliphatic diamines of the formula (II)

$$H_2N\text{-}R\text{-}NH_2$$

where R is a divalent cycloaliphatic hydrocarbon radical having 4 to 18, preferably 5 to 15, carbon atoms, where the two nitrogen atoms are bonded directly to at least one hydrocarbon cycle and at least 3 carbon atoms are arranged between them, are reacted with urea and in the presence of alcohols of the formula (III)

$$R^1\text{-}OH$$

where $R^1$ is a radical as remains after removal of the hydroxyl group from a primary or secondary (cyclo)aliphatic alcohol having 3 to 8 carbon atoms, in the absence or presence of catalysts, to give cycloalkylenebisureas of the formula (IV)

$$H_2N\text{-}OC\text{-}HN\text{-}R\text{-}NH\text{-}CO\text{-}NH_2$$

where R is a divalent cycloaliphatic hydrocarbon radical having 4 to 18, preferably 5 to 15, carbon atoms, with the proviso that the two nitrogen atoms flanking R are bonded directly to a hydrocarbon cycle and at least 3 carbon atoms are arranged between them, and the ammonia formed is simultaneously removed continuously;
b) the crude cycloalkylenebisurea obtained is converted in a second reactor using the alcohol of the formula (III) used as a solvent in a), while continuously driving out the ammonia released, to cycloalkylenediurethane of the formula (V)

$$R^1O\text{-}OC\text{-}HN\text{-}R\text{-}NH\text{-}CO\text{-}OR^1;$$

c) the alcohol, the dialkyl carbonates and/or alkyl carbamates are removed from the resulting reaction mixture and the alcohol is recycled into reaction stage a);
d) a removal of any high-boiling residues present in the resulting reaction mixture is completely or partially dispensed with;
e) the reaction mixture which contains diurethanes and is purified via steps c) and d) is thermally cleaved in the presence of a catalyst, continuously and without a solvent, at temperatures of 180 to 280°C, preferably 200 to 260°C, and under a pressure of 0.1 to 200 mbar, preferably 0.2 to 100 mbar, such that a portion of the reaction mixture of 10 to 60% by weight based on the feed, preferably 15 to 45% by weight based on the feed, is discharged continuously from the bottom;
f) the cleavage products are separated by rectification into a crude diisocyanate and alcohol;
g) the crude cycloaliphatic diisocyanate is purified by distillation and the pure product fraction is isolated;
h) the bottoms discharge from e) is reacted partially or completely with the alcohol from f) in the presence or absence of catalysts at temperatures of 20 to 200°C, preferably 50 to 170°C, and at a pressure of 0.5 to 20 bar, preferably 1 to 15 bar, over the course of 1 to 150 min, preferably 3 to 60 min, where the molar ratio of NCO groups and OH groups is up to 1:100, preferably 1:20 and more preferably 1:10;
i) the material-of-value stream from h) is separated into a material-of-value stream and a waste stream, and the waste stream which is rich in high boiler components is discharged from the process and discarded;
j) a portion of the bottoms fraction of the purifying distillation g) is discharged continuously and conducted into the cleavage reaction e) and/or into the urethanization stage h);
k) optionally, the top fraction obtained in the purifying distillation of the crude cycloaliphatic diisocyanate is likewise recycled into the urethanization stage h);
l) the purified reurethanized stream from i) is recycled into stage b) and/or c) or e).

**40.** A process according to at least one of the preceding claims,
**characterized in that**
the content of chlorine, calculated as chlorine ions, M = 35.45 g/mol, is less than 100 ppm, preferably less than 50 ppm, more preferably less than 20 ppm, based on all amounts of substances present in the 3rd stage.

**41.** A process according to at least one of the preceding claims,
**characterized in that**
the content of chlorine, calculated as chlorine ions, M = 35.45 g/mol, is less than 100 ppm, preferably less than 50 ppm, more preferably less than 20 ppm, based on all amounts of substances present in the cleavage.

**42.** A process according to at least one of the preceding claims,
**characterized in that**
the product streams discharged during the cleavage or after the cleavage or obtained by workup are reurethanized, where the amount of chlorine, calculated as the chlorine ion, M = 35.45 g/mol, is less than 100 ppm, preferably less than 50 ppm, based on all amounts of substances present in the reaction mixture.

**43.** A process according to at least one of the preceding claims,
**characterized in that**
conditioned or preferably unconditioned urea is used in the 3rd stage.

**44.** A process according to claim 43,
**characterized in that**
the urea, preferably the unconditioned urea, is used in the form of prills, granules, crystals, melt, solution, suspension.

**Revendications**

**1.** Procédé pauvre en chlore, en plusieurs étapes et continu pour la préparation de diisocyanates cycloaliphatiques par

1. une préparation exempte de chlore de diamines aromatiques, une amine aromatique, qui peut être substituée ou non substituée, étant transformée avec un $C_1$-$C_3$-aldéhyde en présence d'un catalyseur hétérogène, le catalyseur étant un échangeur d'ions acide mésoporeux à base d'un copolymère de divinylbenzène/styrène et le catalyseur présentant des centres acides en une concentration de 2 à 6 équiv/kg selon la norme DIN 54403 et le diamètre moyen des pores des particules catalytiques, mesuré selon la norme ASTM D 4222, valant 1 à 50 nm ;
2. une hydrogénation exempte de chlore des diamines aromatiques en diamines cycloaliphatiques par transformation des diamines aromatiques avec de l'hydrogène en présence d'un catalyseur ;
3. une préparation exempte de chlore ou pauvre en chlore et continue de diisocyanates cycloaliphatiques par transformation de diamines cycloaliphatiques avec des dérivés de l'acide carbonique, tels que l'urée et/ou des équivalents d'urée, et des alcools en diuréthanes cycloaliphatiques et une dissociation thermique consécutive des diuréthanes en diisocyanates cycloaliphatiques.

**2.** Procédé selon la revendication 1, **caractérisé en ce que** du formaldéhyde est utilisé comme aldéhyde.

**3.** Procédé selon la revendication 1, **caractérisé en ce qu'**on utilise de l'aniline comme amine.

**4.** Procédé selon la revendication 1 à 3, **caractérisé en ce qu'**on prépare du diaminodiphénylméthane.

**5.** Procédé selon au moins l'une quelconque des revendications 1 à 4, **caractérisé en ce que** le rapport d'isomères du diaminodiphénylalcane obtenu est réparti comme suit :

64 à 85% en poids de 4,4'-diaminodiphénylalcane
3 à 20% en poids de 2,4'-diaminodiphénylalcane
jusqu'à ≤ 2% en poids de 2,2'-diaminodiphénylalcane
et de préférence **en ce que** le rapport d'isomères du diaminodiphénylméthane obtenu est réparti comme suit :

64 à 85% en poids de 4,4'-diaminodiphénylméthane
3 à 20% en poids de 2,4'-diaminodiphénylméthane
≤ 2% en poids de 2,2'-diaminodiphénylméthane.

**6.** Procédé selon au moins l'une quelconque des revendications 1 à 4, **caractérisé en ce que** le rapport d'isomères du diaminodiphénylalcane obtenu est réparti comme suit :

74 à 85% en poids de 4,4'-diaminodiphénylalcane
3 à 20% en poids de 2,4'-diaminodiphénylalcane
≤ 1,0% en poids de 2,2'-diaminodiphénylalcane
et de préférence **en ce que** le rapport d'isomères du diaminodiphénylméthane obtenu est réparti comme suit :

74 à 85% en poids de 4,4'-diaminodiphénylméthane
3 à 20% en poids de 2,4'-diaminodiphénylméthane
≤ 1,0% en poids de 2,2'-diaminodiphénylméthane.

7. Procédé selon au moins l'une quelconque des revendications précédentes, **caractérisé en ce que** la teneur en composés N-méthyle est ≤ 1% en poids.

8. Procédé selon au moins l'une quelconque des revendications précédentes, **caractérisé en ce que** la teneur en composés à plusieurs noyaux est ≤ 15% en poids et de préférence ≤ 10% en poids.

9. Procédé selon au moins l'une quelconque des revendications précédentes, **caractérisé en ce que** la température de réaction se situe dans la plage de 80 à 140°C.

10. Procédé selon au moins l'une quelconque des revendications précédentes, **caractérisé en ce que** le temps de réaction est de 30 minutes à 5 heures.

11. Procédé selon au moins l'une quelconque des revendications précédentes, **caractérisé en ce que** le rapport d'amine à aldéhyde vaut 5:1 à 15:1.

12. Procédé selon au moins l'une quelconque des revendications précédentes, **caractérisé en ce que** le catalyseur est utilisé sous forme sèche ou humide.

13. Procédé selon au moins l'une quelconque des revendications précédentes, **caractérisé en ce que** le procédé est réalisé en continu, de manière discontinue ou de manière semi-continue.

14. Procédé selon au moins l'une quelconque des revendications précédentes, **caractérisé en ce que** la réaction est réalisée dans une cuve agitée, une cascade de cuves agitées, un tube d'écoulement, un réacteur à lit fixe ou dans une colonne.

15. Procédé selon au moins l'une quelconque des revendications précédentes, **caractérisé en ce qu'**on utilise, comme centres acides pour le catalyseur, des groupes acide sulfonique.

16. Procédé selon au moins l'une quelconque des revendications précédentes, **caractérisé en ce que** l'hydrogénation dans la 2ème étape a lieu avec des catalyseurs choisis parmi les contacts pleins, les catalyseurs de type Raney ou les catalyseurs supportés.

17. Procédé selon au moins l'une quelconque des revendications précédentes, **caractérisé en ce qu'**on utilise, dans la 2ème étape, des catalyseurs à base de nickel, de cobalt, de palladium, de platine, de ruthénium et de rhodium, seuls ou en mélange.

18. Procédé selon au moins l'une quelconque des revendications précédentes, **caractérisé en ce que** les catalyseurs dans la 2ème étape contiennent en plus des métaux de dopage et/ou d'autres agents de modification.

19. Procédé selon au moins l'une quelconque des revendications précédentes, **caractérisé en ce qu'**on utilise, dans la 2ème étape, des catalyseurs supportés.

20. Procédé selon au moins l'une quelconque des revendications précédentes, **caractérisé en ce qu'**on utilise, dans la 2ème étape, des matériaux support, choisis parmi le charbon actif, les oxydes inorganiques, les bentonites, les aluminosilicates, les kaolins, les argiles et/ou les kieselgurs et/ou les aluminates de lithium.

21. Procédé selon au moins l'une quelconque des revendications précédentes, **caractérisé en ce qu'**on hydrogène dans la 2ème étape en présence d'un catalyseur supporté, qui contient comme métal actif du ruthénium, seul ou ensemble avec au moins un métal du Ier, VIIème ou VIIIème groupe secondaire du système périodique en une quantité de 0,01 à 20% en poids de métal actif, par rapport au catalyseur supporté, appliqué sur un support.

22. Procédé selon la revendication 20, **caractérisé en ce que**, dans la 2ème étape, le matériau support est choisi dans la série $Al_2O_3$, $SiO_2$, $TiO_2$, $ZrO_2$, MgO, ZnO.

23. Procédé selon au moins l'une quelconque des revendications précédentes, **caractérisé en ce que** du ruthénium et/ou du rhodium est/sont contenu(s) comme métal actif dans la 2ème étape.

24. Procédé selon au moins l'une quelconque des revendications précédentes, **caractérisé en ce qu'**on réalise, dans la 2ème étape, l'hydrogénation à une température dans la plage de 20 à 200°C, en particulier de 50 à 150°C, et à une pression partielle d'hydrogène dans la plage de 3 à 30 MPa, en particulier de 3 à 10 MPa.

25. Procédé selon au moins l'une quelconque des revendications précédentes, **caractérisé en ce qu'**on réalise l'hydrogénation dans un réacteur à lit fixe, en particulier dans un réacteur à faisceau tubulaire.

26. Procédé selon au moins l'une quelconque des revendications précédentes, **caractérisé en ce qu'**on réalise l'hydrogénation dans deux réacteurs à lit fixe ou plus, commutés en série.

27. Procédé selon au moins l'une quelconque des revendications précédentes, **caractérisé en ce qu'**on réalise l'hydrogénation dans la 2ème étape en continu.

28. Procédé selon au moins l'une quelconque des revendications précédentes, **caractérisé en ce qu'**on utilise, comme mélange de substances à hydrogéner dans la 2ème étape, un MDA brut, contenant au moins 74% en poids de 4,4'-diaminodiphénylméthane et 0,01 à 2% en poids de composés N-méthyle.

29. Procédé selon au moins l'une quelconque des revendications précédentes, **caractérisé en ce que** dans la 2ème étape, le mélange de substances à hydrogéner contient 74-85% en poids de 4,4'-MDA, 3-20% en poids de 2,4'-MDA, moins de 1% en poids de 2,2'-MDA et jusqu'à 1% en poids de composés N-méthyle.

30. Procédé selon au moins l'une quelconque des revendications précédentes, **caractérisé en ce que** dans la 2ème étape, l'hydrogénation catalytique est réalisée en présence d'un solvant.

31. Procédé selon au moins l'une quelconque des revendications précédentes, **caractérisé en ce qu'**on prépare, dans la 2ème étape, de la méthylènedicyclohexyldiamine.

32. Procédé selon au moins l'une quelconque des revendications précédentes, **caractérisé en ce que** dans la 3ème étape, les diuréthanes cycloaliphatiques sont préparés selon des procédés à une étape, à deux étapes ou en variante aussi à plusieurs étapes, libérés, après leur synthèse par transformation de diamines cycloaliphatiques avec un alcool et de l'urée et/ou des dérivés d'urée, des fractions à bas point d'ébullition, à point d'ébullition moyen et le cas échéant à point d'ébullition élevé, les diuréthanes cycloaliphatiques ainsi purifiés sont dissociés thermiquement avec libération du diisocyanate souhaité, une partie du fond de la dissociation est soutirée en continu de l'appareillage de dissociation et, après traitement ou en variante sans purification supplémentaire, ré-uréthanisée par de l'alcool et recyclée dans le procédé.

33. Procédé selon au moins l'une quelconque des revendications précédentes, **caractérisé en ce qu'**on prépare du $H_{12}MDI$ dans la 3ème étape.

34. Procédé selon au moins l'une quelconque des revendications précédentes 1 à 33, **caractérisé par** un procédé à plusieurs étapes pour la préparation en continu de diisocyanates cycloaliphatiques de la 3ème étape de formule (I)

OCN-R-NCO

dans laquelle R représente un radical hydrocarboné divalent cycloaliphatique comprenant 4 à 18, de préférence 5 à 15 atomes de carbone, sous réserve que les deux groupes isocyanate soient liés directement à un cycle hydrocarboné et qu'au moins 3 atomes de carbone soient interposés entre eux, par transformation de diamines cycloaliphatiques avec de l'urée et/ou des dérivés d'urée et des alcools en diuréthanes cycloaliphatiques et leur dissociation thermique, où

a) on transforme des diamines cycloaliphatiques de formule (II)

$H_2N$-R-$NH_2$

dans laquelle R représente un radical hydrocarboné divalent cycloaliphatique comprenant 4 à 18, de préférence 5 à 15 atomes de carbone, sous réserve que les deux atomes d'azote soient liés directement à un cycle hydrocarboné et qu'au moins 3 atomes de carbone soient interposés entre eux, avec de l'urée et/ou des dérivés d'urée et des alcools de formule (III)

$$R^1\text{-OH}$$

dans laquelle $R^1$ représente un radical tel qu'il reste après l'élimination du groupe hydroxyle d'un alcool (cyclo)aliphatique primaire ou secondaire comprenant 3 à 8 atomes de carbone,
en l'absence ou en présence de carbonates de dialkyle, d'esters alkyliques de l'acide carbamique ou de mélanges de carbonates de dialkyle et d'esters de l'acide carbamique et en l'absence ou en présence de catalyseurs, en diuréthanes cycloaliphatiques et on sépare simultanément l'ammoniac qui se forme ;
b) on sépare l'alcool, les carbonates de dialkyle et/ou les esters alkyliques de l'acide carbamique du mélange réactionnel obtenu et on recycle l'alcool ainsi qu'éventuellement les carbonates de dialkyle et/ou les esters alkyliques de l'acide carbamique dans l'étape de réaction a) ;
c) on sépare le flux de substances de l'étape b), contenant essentiellement des diuréthanes, par distillation en un flux de substances de valeur et un flux de produits secondaires, qui est soutiré ;
d) on dissocie en continu par voie thermique le mélange réactionnel contenant les diuréthanes, purifié via les étapes b) et c) en présence d'un catalyseur, sans solvant, à des températures de 180 à 280°C, de préférence de 200 à 260°C, et à une pression de 0,1 à 200 mbars, de préférence de 0,2 à 100 mbars, de telle sorte qu'une partie du mélange réactionnel représentant 10 à 60% en poids, par rapport à l'alimentation, de préférence de 15 à 45% en poids, par rapport à l'alimentation, est constamment soutirée ;
e) on sépare les produits de dissociation par rectification en un diisocyanate cycloaliphatique brut et en alcool ;
f) on purifie le diisocyanate cycloaliphatique brut par distillation et on isole la fraction de produit pur ;
g) on transforme le produit soutiré du fond de d) avec l'alcool de e) en présence ou en l'absence de catalyseurs en l'espace de 1 à 150 min, de préférence de 3 à 60 min, à des températures de 20 à 200°C, de préférence de 50 à 170°C et à une pression de 0,5 à 20 bars, de préférence de 1 à 15 bars, le rapport molaire des groupes NCO aux groupes OH valant jusqu'à 1:100, de préférence 1:20 et de manière particulièrement préférée 1:10 ;
h) on soutire en continu une partie de la fraction du fond de la distillation pure f) et on la guide dans la réaction de dissociation d), de préférence cependant dans l'étape d'uréthanisation g) ;
i) on recycle la fraction de tête produite lors de la distillation pure du diisocyanate cycloaliphatique également dans l'étape d'uréthanisation g) ou on la rejette ;
j) on recycle le flux de substance ré-uréthanisée de g) dans l'étape b) ou
k) on recycle le flux de substance ré-uréthanisée de g) dans l'étape de réaction a), à condition que g) soit réalisée en présence de catalyseurs, de préférence choisis parmi les halogénures de Fe(III) et/ou Cu(I).

35. Procédé selon au moins l'une quelconque des revendications précédentes 1 à 33, **caractérisé par** un procédé à plusieurs étapes pour la préparation en continu de diisocyanates cycloaliphatiques de la 3ème étape de formule (I)

$$\text{OCN-R-NCO}$$

dans laquelle R représente un radical hydrocarboné divalent cycloaliphatique comprenant 4 à 18, de préférence 5 à 15 atomes de carbone, sous réserve que les deux atomes d'azote soient liés directement à un cycle hydrocarboné et qu'au moins 3 atomes de carbone soient interposés entre eux, par transformation de diamines cycloaliphatiques avec de l'urée et/ou des dérivés d'urée et des alcools en diuréthanes cycloaliphatiques et leur dissociation thermique, où

a) on transforme des diamines cycloaliphatiques de formule (II)

$$H_2N\text{-R-}NH_2$$

dans laquelle R représente un radical hydrocarboné divalent cycloaliphatique comprenant 4 à 18, de préférence 5 à 15 atomes de carbone, sous réserve que les deux atomes d'azote soient liés directement à un cycle hydrocarboné et qu'au moins 3 atomes de carbone soient interposés entre eux, avec de l'urée et/ou des dérivés d'urée et des alcools de formule (III)

$$R^1\text{-OH}$$

dans laquelle $R^1$ représente un radical tel qu'il reste après l'élimination du groupe hydroxyle d'un alcool (cyclo)aliphatique primaire ou secondaire comprenant 3 à 8 atomes de carbone,
en l'absence ou en présence de carbonates de dialkyle, d'esters alkyliques de l'acide carbamique ou de mélanges de carbonates de dialkyle et d'esters de l'acide carbamique et en l'absence ou en présence de catalyseurs, en diuréthanes cycloaliphatiques et on sépare simultanément l'ammoniac qui se forme ;

b) on sépare l'alcool, les carbonates de dialkyle et/ou les esters alkyliques de l'acide carbamique du mélange réactionnel obtenu et on recycle l'alcool ainsi qu'éventuellement aussi les carbonates de dialkyle et/ou les esters alkyliques de l'acide carbamique dans l'étape de réaction a) ;

c) on renonce totalement ou partiellement à une séparation des résidus à point d'ébullition élevé le cas échéant contenus dans le mélange réactionnel obtenu ;

d) on dissocie en continu par voie thermique le mélange réactionnel contenant les diuréthanes, purifié via les étapes b) et éventuellement c) en présence d'un catalyseur, en continu et sans solvant, à des températures de 180 à 280°C, de préférence de 200 à 260°C, et à une pression de 0,1 à 200 mbars, de préférence de 0,2 à 100 mbars, de telle sorte qu'une partie du mélange réactionnel représentant 10 à 60% en poids, par rapport à l'alimentation, de préférence de 15 à 45% en poids, par rapport à l'alimentation, est constamment soutirée ;

e) on sépare les produits de dissociation par rectification en un diisocyanate cycloaliphatique brut et en alcool ;

f) on purifie le diisocyanate cycloaliphatique brut par distillation et on isole la fraction de produit pur ;

g) on transforme le produit soutiré du fond de d), partiellement ou complètement, avec l'alcool de e) en présence ou en l'absence de catalyseurs en l'espace de 1 à 150 min, de préférence de 3 à 60 min, à des températures de 20 à 200°C, de préférence de 50 à 170°C et à une pression de 0,5 à 20 bars, de préférence de 1 à 15 bars, le rapport molaire des groupes NCO aux groupes OH valant jusqu'à 1:100, de préférence 1:20 et de manière particulièrement préférée 1:10 ;

h) on sépare le flux de substance ré-uréthanisée de g) en un flux de produit de valeur et en un flux de déchet et le flux de déchet riche en composants à point d'ébullition élevé est soutiré du procédé et est rejeté ;

i) on soutire en continu une partie de la fraction du fond de la distillation pure f) et on la guide dans la réaction de dissociation d) ou dans l'étape d'uréthanisation g) ;

j) on recycle éventuellement aussi la fraction de tête se formant lors de la distillation pure f) du diisocyanate cycloaliphatique brut dans l'étape d'uréthanisation g) ;

k) on recycle le flux de produit de valeur de h) dans l'étape a), b) ou d).

36. Procédé selon au moins l'une quelconque des revendications précédentes 1 à 33, **caractérisé par** un procédé à plusieurs étapes pour la préparation en continu de diisocyanates cycloaliphatiques de la 3ème étape de formule (I)

OCN-R-NCO

dans laquelle R représente un radical hydrocarboné divalent cycloaliphatique comprenant 4 à 18, de préférence 5 à 15 atomes de carbone, sous réserve que les deux atomes d'azote soient liés directement à un cycle hydrocarboné et qu'au moins 3 atomes de carbone soient interposés entre eux, par transformation de diamines cycloaliphatiques avec de l'urée et/ou des dérivés d'urée et des alcools en diuréthanes cycloaliphatiques et leur dissociation thermique, où

a) on transforme des diamines cycloaliphatiques de formule (II)

$H_2N$-R-$NH_2$

dans laquelle R représente un radical hydrocarboné divalent cycloaliphatique comprenant 4 à 18, de préférence 5 à 15 atomes de carbone, sous réserve que les deux atomes d'azote soient liés directement à un cycle hydrocarboné et qu'au moins 3 atomes de carbone soient interposés entre eux, avec de l'urée et/ou des dérivés d'urée et des alcools de formule (III)

$R^1$-OH

dans laquelle $R^1$ représente un radical tel qu'il reste après l'élimination du groupe hydroxyle d'un alcool (cyclo)aliphatique primaire ou secondaire comprenant 3 à 8 atomes de carbone,
en l'absence ou en présence de carbonates de dialkyle, d'esters alkyliques de l'acide carbamique ou de mélanges de carbonates de dialkyle et d'esters de l'acide carbamique et en l'absence ou en présence de catalyseurs, en diuréthanes cycloaliphatiques et on sépare simultanément l'ammoniac qui se forme ;

b) on sépare l'alcool, les carbonates de dialkyle et/ou les esters alkyliques de l'acide carbamique du mélange réactionnel obtenu et on recycle l'alcool ainsi qu'éventuellement aussi les carbonates de dialkyle et/ou les esters alkyliques de l'acide carbamique dans l'étape de réaction a) ;

c) on renonce totalement ou partiellement à une séparation des résidus à point d'ébullition élevé le cas échéant contenus dans le mélange réactionnel obtenu ;

d) on dissocie en continu par voie thermique le mélange réactionnel contenant les diuréthanes, purifié via les étapes b) et éventuellement c) en présence d'un catalyseur, en continu et sans solvant, à des températures de 180 à 280°C, de préférence de 200 à 260°C, et à une pression de 0,1 à 200 mbars, de préférence de 0,2 à 100 mbars, de telle sorte qu'une partie du mélange réactionnel représentant 10 à 60% en poids, par rapport à l'alimentation, de préférence de 15 à 45% en poids, par rapport à l'alimentation, est constamment soutirée ;

e) on sépare les produits de dissociation par rectification en un diisocyanate cycloaliphatique brut et en alcool ;

f) on purifie le diisocyanate cycloaliphatique brut par distillation et on isole la fraction de produit pur ;

g) on sépare le produit soutiré du fond de d) en un flux de produit de valeur et en un flux de déchet et le flux de déchet riche en composants à point d'ébullition élevé est soutiré du procédé et rejeté ;

h) on transforme le flux de produit de valeur g) avec l'alcool de e) en présence ou en l'absence de catalyseurs en l'espace de 1 à 150 min, de préférence de 3 à 60 min, à des températures de 20 à 200°C, de préférence de 50 à 170°C et à une pression de 0,5 à 20 bars, de préférence de 1 à 15 bars, le rapport molaire des groupes NCO aux groupes OH valant jusqu'à 1:100, de préférence 1:20 et de manière particulièrement préférée 1:10 ;

i) on soutire en continu une partie de la fraction du fond de la distillation pure f) et on la guide dans la réaction de dissociation d) ou dans l'étape d'uréthanisation h) ;

j) on recycle éventuellement aussi la fraction de tête se formant lors de la distillation pure f) du diisocyanate cycloaliphatique brut dans l'étape d'uréthanisation h) ;

k) on recycle le flux de substance ré-uréthanisée de h) dans l'étape b) ou

l) on recycle le flux de substance ré-uréthanisée de h) dans l'étape de réaction a), à condition que l'étape h) soit réalisée en présence de catalyseurs, choisis parmi les halogénures de Fe(III) et/ou Cu(I).

**37.** Procédé selon au moins l'une quelconque des revendications précédentes 1 à 33, **caractérisé par** un procédé à plusieurs étapes pour la préparation en continu de diisocyanates cycloaliphatiques de la 3ème étape de formule (I)

OCN-R-NCO

dans laquelle R représente un radical hydrocarboné divalent cycloaliphatique comprenant 4 à 18, de préférence 5 à 15 atomes de carbone, sous réserve que les deux groupes isocyanate soient liés directement à un cycle hydrocarboné et qu'au moins 3 atomes de carbone soient interposés entre eux, par transformation de diamines cycloaliphatiques avec des dérivés de l'acide carbonique et des alcools en diuréthanes et leur dissociation thermique, où

a) on transforme des diamines cycloaliphatiques de formule (II)

$H_2N$-R-$NH_2$

dans laquelle R représente un radical hydrocarboné cycloaliphatique divalent comprenant 4 à 18, de préférence 5 à 15 atomes de carbone, les deux atomes d'azote étant liés directement à au moins un cycle hydrocarboné et au moins 3 atomes de carbone étant interposés entre eux, avec de l'urée et en présence d'alcools de formule (III)

$R^1$- OH

dans laquelle $R^1$ représente un radical tel qu'il reste après l'élimination du groupe hydroxyle d'un alcool (cyclo)aliphatique primaire ou secondaire comprenant 3 à 8 atomes de carbone, en l'absence ou en présence de catalyseurs, pour donner des cycloalkylène-bis-urées de formule (IV)

$H_2N$-OC-HN-R-NH-CO-$NH_2$

dans laquelle R représente un radical hydrocarboné divalent cycloaliphatique comprenant 4 à 18, de préférence 5 à 15 atomes de carbone, sous réserve que les deux atomes d'azote flanquant R soient liés directement à un cycle hydrocarboné et qu'au moins 3 atomes de carbone soient interposés entre eux, et on sépare simultanément en continu l'ammoniac qui se forme ;

b) on transforme la cycloalkylène-bis-urée brute qui se forme dans un deuxième réacteur avec l'alcool de formule (III) utilisé comme solvant dans a), en chassant en continu l'ammoniac qui se dégage, en cycloalkylènediuréthane de formule (V)

$$R^1O\text{-}OC\text{-}HN\text{-}R\text{-}NH\text{-}CO\text{-}OR^1$$

c) on sépare l'alcool, les carbonates de dialkyle et/ou les esters alkyliques de l'acide carbamique du mélange réactionnel obtenu et on recycle l'alcool dans l'étape de réaction a) ;

d) on renonce totalement ou partiellement à une séparation des résidus à point d'ébullition élevé le cas échéant contenus dans le mélange réactionnel obtenu ;

e) on dissocie thermiquement le mélange réactionnel contenant les diuréthanes, purifié via les étapes c) et d) en présence d'un catalyseur, en continu et sans solvant à des températures de 180 à 280 °C, de préférence de 200 à 260 °C, et sous une pression de 0,1 à 200 mbars, de préférence de 0,2 à 100 mbars, de telle sorte qu'une partie du mélange réactionnel de 10 à 60% en poids par rapport à l'alimentation, de préférence de 15 à 45% en poids par rapport à l'alimentation, est constamment soutirée ;

f) on sépare les produits de dissociation par rectification en un diisocyanate brut et en alcool ;

g) on purifie par distillation le diisocyanate cycloaliphatique brut et on isole la fraction de produit pur ;

h) on sépare le produit soutiré du fond de e) en un flux de produit de valeur et en un flux de déchet et le flux de déchet riche en composants à point d'ébullition élevé est soutiré du processus et est rejeté ;

i) on fait réagir le flux de produit de valeur de h) avec l'alcool de f) en présence ou en l'absence de catalyseurs en l'espace de 1 à 150 min, de préférence de 3 à 60 min, à des températures de 20 à 200 °C, de préférence de 50 à 170 °C et à une pression de 0,5 à 20 bars, de préférence de 1 à 15 bars, le rapport molaire des groupes NCO aux groupes OH valant jusqu'à 1:100, de préférence 1:20 et de manière particulièrement préférée 1:10 ;

j) on soutire en continu une partie de la fraction de fond de la distillation pure g) et elle est guidée dans la réaction de dissociation e) et/ou dans l'étape d'uréthanisation i) ;

k) on recycle éventuellement aussi la fraction de tête produite lors de la distillation pure du diisocyanate cycloaliphatique brut dans l'étape d'uréthanisation i) ;

l) on recycle le flux de substance ré-uréthanisée de i) dans l'étape b) et/ou c).

38. Procédé selon au moins l'une quelconque des revendications précédentes 1 à 33, **caractérisé par** un procédé à plusieurs étapes pour la préparation en continu de diisocyanates cycloaliphatiques de la 3ème étape de formule (I)

$$OCN\text{-}R\text{-}NCO$$

dans laquelle R représente un radical hydrocarboné divalent cycloaliphatique comprenant 4 à 18, de préférence 5 à 15 atomes de carbone, sous réserve que les deux groupes isocyanate soient liés directement à un cycle hydrocarboné et qu'au moins 3 atomes de carbone soient interposés entre eux, par transformation de diamines cycloaliphatiques avec des dérivés de l'acide carbonique et des alcools en diuréthanes et leur dissociation thermique, où

a) on transforme des diamines cycloaliphatiques de formule (II)

$$H_2N\text{-}R\text{-}NH_2$$

dans laquelle R représente un radical hydrocarboné cycloaliphatique divalent comprenant 4 à 18, de préférence 5 à 15 atomes de carbone, les deux atomes d'azote étant liés directement à au moins un cycle hydrocarboné et au moins 3 atomes de carbone étant interposés entre eux, avec de l'urée et en présence d'alcools de formule (III)

$$R^1\text{-}OH$$

dans laquelle $R^1$ représente un radical tel qu'il reste après l'élimination du groupe hydroxyle d'un alcool (cyclo)aliphatique primaire ou secondaire comprenant 3 à 8 atomes de carbone, en l'absence ou en présence de catalyseurs, pour donner des cycloalkylène-bis-urées de formule (IV)

$$H_2N\text{-}OC\text{-}HN\text{-}R\text{-}NH\text{-}CO\text{-}NH_2$$

dans laquelle R représente un radical hydrocarboné divalent cycloaliphatique comprenant 4 à 18, de préférence 5 à 15 atomes de carbone, sous réserve que les deux atomes d'azote flanquant R soient liés directement à un cycle hydrocarboné et qu'au moins 3 atomes de carbone soient interposés entre eux, et on sépare simultanément en continu l'ammoniac qui se forme ;

b) on transforme la cycloalkylène-bis-urée brute qui se forme dans un deuxième réacteur avec l'alcool de formule (III) utilisé comme solvant dans a), en chassant en continu l'ammoniac qui se dégage, en cycloalkylènediuréthane de formule (V)

$$R^1O\text{-}OC\text{-}HN\text{-}R\text{-}NH\text{-}CO\text{-}OR^1$$

c) on sépare l'alcool, les carbonates de dialkyle et/ou les esters alkyliques de l'acide carbamique du mélange réactionnel obtenu et on recycle l'alcool dans l'étape de réaction a) ;

d) on sépare le flux de substances de l'étape c) par distillation en un flux de substances de valeur et un flux de produits secondaires, qui est soutiré,

e) on dissocie en continu par voie thermique le mélange réactionnel contenant les diuréthanes, purifié via les étapes c) et d) en présence d'un catalyseur, en l'absence de solvants, à des températures de 180 à 280°C, de préférence de 200 à 260°C, et à une pression de 0,1 à 200 mbars, de préférence de 0,2 à 100 mbars, de telle sorte qu'une partie du mélange réactionnel représentant 10 à 60% en poids, par rapport à l'alimentation, de préférence de 15 à 45% en poids, est constamment soutirée ;

f) on sépare les produits de dissociation par rectification en un diisocyanate brut et en alcool ;

g) on purifie par distillation le diisocyanate cycloaliphatique brut et on isole la fraction de produit pur ;

h) on fait réagir le produit évacué du fond de e) avec l'alcool de f) en présence ou en l'absence de catalyseurs en l'espace de 1 à 150 min, de préférence de 3 à 60 min, à des températures de 20 à 200 °C, de préférence de 50 à 170 °C et à une pression de 0,5 à 20 bars, de préférence de 1 à 15 bars, et le rapport molaire des groupes NCO aux groupes OH valant jusqu'à 1:100, de préférence 1:20 et de manière particulièrement préférée 1:10 ;

i) on effectue éventuellement la réaction de ré-uréthanisation h) en présence de catalyseurs particuliers, sélectionnés parmi les halogénures de Fe (III) et/ou de Cu(I) ;

j) on soutire en continu une partie de la fraction du fond de la distillation pure g) et elle est guidée dans la réaction de dissociation e) et/ou dans l'étape d'uréthanisation h) ;

k) on recycle éventuellement aussi la fraction de tête se formant lors de la distillation pure du diisocyanate cycloaliphatique brut dans l'étape d'uréthanisation h) ;

l) on recycle le flux de substance ré-uréthanisée provenant de h) dans l'étape c).

39. Procédé selon au moins l'une quelconque des revendications précédentes 1 à 33, **caractérisé par** un procédé à plusieurs étapes pour la préparation en continu de diisocyanates cycloaliphatiques de la 3ème étape de formule (I)

$$OCN\text{-}R\text{-}NCO$$

dans laquelle R représente un radical hydrocarboné divalent cycloaliphatique comprenant 4 à 18, de préférence 5 à 15 atomes de carbone, sous réserve que les deux groupes isocyanate soient liés directement à un cycle hydrocarboné et qu'au moins 3 atomes de carbone soient interposés entre eux, par transformation de diamines cycloaliphatiques avec des dérivés de l'acide carbonique et des alcools en diuréthanes et leur dissociation thermique, où

a) on transforme des diamines cycloaliphatiques de formule (II)

$$H_2N\text{-}R\text{-}NH_2$$

dans laquelle R représente un radical hydrocarboné cycloaliphatique divalent comprenant 4 à 18, de préférence 5 à 15 atomes de carbone, les deux atomes d'azote étant liés directement à au moins un cycle hydrocarboné et au moins 3 atomes de carbone étant interposés entre eux, avec de l'urée et en présence d'alcools de formule (III)

$$R^1\text{-}OH$$

dans laquelle $R^1$ représente un radical tel qu'il reste après l'élimination du groupe hydroxyle d'un alcool (cyclo)aliphatique primaire ou secondaire comprenant 3 à 8 atomes de carbone, en l'absence ou en présence de catalyseurs, pour donner des cycloalkylène-bis-urées de formule (IV)

$$H_2N\text{-}OC\text{-}HN\text{-}R\text{-}NH\text{-}CO\text{-}NH_2$$

dans laquelle R représente un radical hydrocarboné divalent cycloaliphatique comprenant 4 à 18, de préférence 5 à 15 atomes de carbone, sous réserve que les deux atomes d'azote flanquant R soient liés directement à un cycle hydrocarboné et qu'au moins 3 atomes de carbone soient interposés entre eux, et on sépare simultanément en continu l'ammoniac qui se forme ;

b) on transforme la cycloalkylène-bis-urée brute qui se forme dans un deuxième réacteur avec l'alcool de formule (III) utilisé comme solvant dans a), en chassant en continu l'ammoniac qui se dégage, en cycloalkylènediuréthane de formule (V)

$$R^1O-OC-HN-R-NH-CO-OR^1$$

c) on sépare l'alcool, les carbonates de dialkyle et/ou les esters alkyliques de l'acide carbamique du mélange réactionnel obtenu et on recycle l'alcool dans l'étape de réaction a) ;

d) on renonce totalement ou partiellement à une séparation des résidus à point d'ébullition élevé le cas échéant contenus dans le mélange réactionnel obtenu ;

e) on dissocie thermiquement le mélange réactionnel contenant les diuréthanes, purifié via les étapes c) et d) en présence d'un catalyseur, en continu et sans solvant à des températures de 180 à 280 °C, de préférence de 200 à 260 °C, et sous une pression de 0,1 à 200 mbars, de préférence de 0,2 à 100 mbars, de telle sorte qu'une partie du mélange réactionnel de 10 à 60% en poids par rapport à l'alimentation, de préférence de 15 à 45% en poids par rapport à l'alimentation, est constamment soutirée du fond ;

f) on sépare les produits de dissociation par rectification en un diisocyanate brut et en alcool ;

g) on purifie par distillation le diisocyanate cycloaliphatique brut et on isole la fraction de produit pur ;

h) on transforme le produit soutiré du fond de e), partiellement ou complètement, avec l'alcool de f) en présence ou en l'absence de catalyseurs en l'espace de 1 à 150 min, de préférence de 3 à 60 min, à des températures de 20 à 200°C, de préférence de 50 à 170°C et à une pression de 0,5 à 20 bars, de préférence de 1 à 15 bars, le rapport molaire des groupes NCO aux groupes OH valant jusqu'à 1:100, de préférence 1:20 et de manière particulièrement préférée 1:10 ;

i) on sépare le flux de produit de valeur de h) en un flux de produit de valeur et en un flux de déchet et le flux de déchet riche en composants à point d'ébullition élevé est soutiré du procédé et est rejeté ;

j) on soutire en continu une partie de la fraction du fond de la distillation pure g) et elle est guidée dans la réaction de dissociation e) et/ou dans l'étape d'uréthanisation h) ;

k) on recycle éventuellement également la fraction de tête se formant lors de la distillation pure du diisocyanate cycloaliphatique brut dans l'étape d'uréthanisation h) ;

l) on recycle le flux de substance ré-uréthanisée purifié de i) dans l'étape b) et/ou c) ou e).

**40.** Procédé selon au moins l'une quelconque des revendications précédentes, **caractérisé en ce que** la teneur en chlore, calculée sous forme d'ion chlore, M = 35,45 g/mole, est inférieure à 100 ppm, de préférence inférieure à 50 ppm, de manière particulièrement préférée inférieure à 20 ppm, par rapport à toutes les quantités de substances présentes dans la 3ème étape.

**41.** Procédé selon au moins l'une quelconque des revendications précédentes, **caractérisé en ce que** la teneur en chlore, calculée sous forme d'ion chlore, M = 35,45 g/mole, est inférieure à 100 ppm, de préférence inférieure à 50 ppm, de manière particulièrement préférée inférieure à 20 ppm, par rapport à toutes les quantités de substances présentes dans la dissociation.

**42.** Procédé selon au moins l'une quelconque des revendications précédentes, **caractérisé en ce que** les flux de produits soutirés pendant la dissociation ou après la dissociation ou obtenus par traitement sont ré-uréthanisés, la quantité de chlore, calculée sous forme d'ion chlore, M = 35,45 g/mole, étant inférieure à 100 ppm, de préférence inférieure à 50 ppm, par rapport à toutes les quantités de substances présentes dans le mélange réactionnel.

**43.** Procédé selon au moins l'une quelconque des revendications précédentes, **caractérisé en ce qu'**on utilise, dans la 3ème étape, de l'urée conditionnée ou de préférence non conditionnée.

**44.** Procédé selon la revendication 43, **caractérisé en ce que** l'urée, de préférence l'urée non conditionnée, est utilisée sous forme de billes, de granulat, de cristaux, de masse fondue, de solution, de suspension.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4294987 A
- DE 1230033 A
- DE 1493431 A
- US 4071558 A
- US 4039580 A
- US 4039581 A
- EP 0043933 A1
- EP 0043933 A
- EP 1366812 A
- DE 1593293
- DE 1948566
- EP 0001425 A
- EP 0630882 A
- EP 66212 A
- US 5545756 A
- EP 0231788 A
- EP 1604972 A
- EP 1149629 A
- EP 0335336 A
- EP 0324190 A
- EP 0018586 A
- EP 0355443 A
- US 4268683 A
- EP 0990644 A
- US 4713476 A
- US 5386053 A
- EP 0568782 A
- EP 0657420 A
- DE 1022222
- US 3919279 A
- DE 2635490
- EP 0054817 A
- EP 0061013 A
- EP 0566925 A
- EP 1512680 A
- EP 1512681 A
- EP 1512682 A
- EP 1593669 A
- EP 1602643 A
- EP 1634868 A
- EP 1767520 A
- EP 0066211 A
- DE 10054347
- EP 0392435 A
- EP 0639403 A

**In der Beschreibung aufgeführte Nicht-Patentliteratur**

- Ullmann's Encyclopedia of Industrial Chemistry. 2006
- **G.F. ALLEN.** *Chem. Ind.,* 1988, vol. 33, 323-338
- Reactor types and their industrial application. Ullmann's Encyclopedia of Industrial Chemistry. 2007